# EUROPEAN PATENT APPLICATION

(11) **EP 4 361 266 A1**
(43) Date of publication of application: **01.05.2024**
(21) Application number: 22827547.5
(22) Date of filing: 21.06.2022
(51) Int. Cl.: C12N 15/11, A61K 31/713, C12N 15/113, A61P 3/06

(54) **SIRNA INHIBITING ANGPTL3 GENE EXPRESSION, AND USE THEREOF**

(30) Priority: 21.06.2021 CN 202110688187
(71) Applicant: Shanghai Junshi Biosciences Co., Ltd., Pilot Free Trade Zone Shanghai 201210 (CN)
(72) Inventor: PAN, Jun, Jiangsu 215002 (CN); MA, Xinxin, Jiangsu 215002 (CN); SONG, Peiming, Jiangsu 215002 (CN); WU, Chun, Jiangsu 215002 (CN); FENG, Hui, Jiangsu 215002 (CN); YAO, Sheng, Jiangsu 215002 (CN); LV, Jiasheng, Jiangsu 215002 (CN); SIYANG, Haixiao, Jiangsu 215002 (CN); YIN, Yijie, Jiangsu 215002 (CN); GUO, Wantao, Jiangsu 215002 (CN); LI, Haiming, Jiangsu 215002 (CN); CHEN, Dawei, Jiangsu 215002 (CN); GU, Jiamin, Jiangsu 215002 (CN); KONG, Xianqi, Jiangsu 215002 (CN)
(74) Representative: V.O.
(86) International application number: PCT/CN2022/100023
(87) International publication number: WO 2022/268054

(57) **Abstract**

The present invention relates to an siRNA for inhibiting the expression of an angiopoietin-like protein 3 (ANGPTL3) gene, an siRNA conjugate, and a pharmaceutical composition thereof, and a method for reducing the expression of the ANGPTL3 gene by using the siRNA, the siRNA conjugate, and the pharmaceutical composition thereof. The siRNA, the siRNA conjugate, and the pharmaceutical composition thereof of the present invention can be used for treating and/or preventing an ANGPTL3 gene-mediated disease or disorder.

## Description

### TECHNICAL FIELD

The present invention belongs to the field of biological pharmaceuticals, and particularly relates to an siRNA for inhibiting the expression of a human angiopoietin-like protein 3 (ANGPTL3) gene, a pharmaceutical composition thereof, and use thereof in the preparation of a medicament for treating a related disease mediated by ANGPTL3.

### BACKGROUND

Angiopoietin-like 3 (also known as ANGPTL3, ANGPL3, ANG3, or angiopoietin-like protein 3) is an angiopoietin protein encoded by a human angiopoietin-like 3 gene, with a primary function in the regulation of lipid metabolism. ANGPTL3 is of 460 amino acids in full length, consisting of a signal peptide, a N-terminal coiled-coil domain, and a C-terminal fibrinogen (FBN)-like domain. It is known that it is produced mainly by hepatocytes and then secreted into the blood, and forms active molecules upon cleavage by PCSK3 or PCSK6, which can inhibit lipoprotein lipase (which catalyzes the hydrolysis of triglycerides) and endothelial lipase (which hydrolyzes lipoprotein phospholipids), resulting in increased levels of triglycerides, high-density lipoproteins (HDLs) and phospholipids in the plasma. In addition, loss-of-function mutations in ANGPTL3 result in familial hypobetalipoproteinemia characterized by low levels of triglycerides and low-density lipoproteins (LDL-C) in the plasma. In humans, loss of function of ANGPTL3 is also associated with a reduced risk of atherosclerotic cardiovascular disease.

RNA interference (RNAi) technology was first discovered in 1998 by Fire et al., and then was soon widely used. Double-stranded RNA that causes gene silencing in RNA interference is siRNA, which generally consists of a segment of double-stranded RNA of about 21-23 nucleotides in length, and includes in its sequence a sense strand and an antisense strand that are paired with a target mRNA, thereby inducing a degradation reaction of the host cell against these mRNAs. Although siRNA can specifically inhibit gene expression, siRNA in cells is easily degraded, and stable gene silencing is difficult to achieve. Lentiviral vectors have high intracellular infection efficiency and low immunogenicity, and can interfere with cells in a division stage and infect cells in a non-division stage. RNAi can be initiated by Lentiviral vectors which can stably express siRNAs in various animal cells for a long period of time and inhibit the expression of target genes, so that the lentiviral vectors have characteristics of high efficiency, stability, strong specificity and wide application range. Small interfering RNAs (siRNAs) can inhibit or block the expression of any target gene of interest in a sequence-specific manner based on the mechanism of RNA interference, thereby achieving the purpose of treating diseases.

At present, a number of RNAi drugs are in the development stage or have been approved for marketing, and have shown good therapeutic effects. However, there is still a need for RNAi drugs with a highly effective and efficient ANGPTL3-specific inhibitory effect on the expression of the ANGPTL3 gene.

### SUMMARY

The present invention provides an siRNA for inhibiting the expression of an ANGPTL3 gene, which comprises a sense strand and an antisense strand, wherein each nucleotide in the siRNA is independently a modified or unmodified nucleotide, the antisense strand comprises at least 17 contiguous nucleotides differing from those of any one of antisense strand sequences shown in Table 1, Table 2, or Table 3 by 0, 1, 2, or 3 nucleotides, and the sense strand has at least 15, 16, 17, 18, 19, 20, or 21 nucleotides complementary to those of the antisense strand.

In some embodiments, the sense strand described herein comprises at least 17 contiguous nucleotides differing from those of any one of sense strand sequences shown in Table 1, Table 2, or Table 3 by 0, 1, 2, or 3 nucleotides.

In some embodiments, the antisense strand described herein comprises any one of antisense strand nucleotide sequences shown in Table 1, and the sense strand comprises any one of sense strand nucleotide sequences shown in Table 1.

In some embodiments, the sense strand and the antisense strand described herein comprise or consist of nucleotide sequences (5' → 3') selected from:
R-6
   sense strand: GUCUCAAAAUGGAAGGUUAUA (SEQ ID NO: 6)
   antisense strand: UAUAACCUUCCAUUUUGAGACUU (SEQ ID NO: 40)
R-15
   sense strand: AGAACACCCAGAAGUAACU (SEQ ID NO: 15)
   antisense strand: AGUUACUUCUGGGUGUUCUGG(SEQ ID NO: 49)
R-23
   sense strand: AAAUCACGAAACCAACUAU (SEQ ID NO: 23)
   antisense strand: AUAGUUGGUUUCGUGAUUUCC (SEQ ID NO: 57)
R-24
   sense strand: ACAUCUAGUUGCGAUUACU (SEQ ID NO: 24)
   antisense strand: AGUAAUCGCAACUAGAUGUAG(SEQ ID NO: 58)
   wherein C, G, U, and A indicate cytidine-3'-phosphate, guanosine-3'-phosphate, uridine-3'-phosphate, and adenosine-3'-phosphate, respectively.

In some embodiments, at least one nucleotide in the sense strand and the antisense strand described herein is a modified nucleotide.

In some embodiments, the modified nucleotide described herein is selected from a 2'-O-methyl-modified nucleotide, a 2'-deoxy-2'-fluoro-modified nucleotide, a 2'-deoxynucleotide, a 2'-methoxyethyl-modified nucleotide, a 2'-amino-modified nucleotide, a 2'-alkyl-modified nucleotide, a 2'-alkoxy-modified nucleotide, a 2'-F-arabinonucleotide, a phosphorothioate-modified nucleotide, an abasic nucleotide, a morpholino nucleotide, and a locked nucleotide.

In some embodiments, the modified nucleotide described herein is selected from: a 2'-O-methyl-modified nucleotide, a 2'-deoxy-2'-fluoro-modified nucleotide, a 2'-deoxynucleotide, and a phosphorothioate-modified nucleotide.

In some embodiments, the modified nucleotide described herein is selected from nucleotides as follows:
(1) in the direction from the 5' end to the 3' end, nucleotides at positions 7 and 9 of the sense strand are 2'-deoxy-2'-fluoro-modified nucleotides, and nucleotides at the rest of positions are 2'-O-methyl-modified nucleotides; or nucleotides at positions 7, 9, and 14 of the sense strand are 2'-deoxy-2'-fluoro-modified nucleotides, and nucleotides at the rest of positions are 2'-O-methyl-modified nucleotides; or nucleotides at positions 8 and 14 of the sense strand are 2'-deoxy-2'-fluoro-modified nucleotides, and nucleotides at the rest of positions are 2'-O-methyl-modified nucleotides; and/or
(2) in the direction from the 5' end to the 3' end, nucleotides at positions 6, 8, 9, 10, 12, 14, and 16 of the antisense strand are 2'-deoxy-2'-fluoro-modified nucleotides, and nucleotides at the rest of positions are 2'-O-methyl-modified nucleotides; or nucleotides at positions 2, 8, 10, 12, 14, and 20 of the antisense strand are 2'-deoxy-2'-fluoro-modified nucleotides, and nucleotides at the rest of positions are 2'-O-methyl-modified nucleotides; and/or
(3) in the direction from the 5' end to the 3' end, a nucleotide at position 11 of the sense strand is a 2'-deoxynucleotide; and/or
(4) in the direction from the 5' end to the 3' end, the 5' end of the sense strand comprises 1 or 2 phosphorothioate-modified nucleotides; and/or the 5' end and the 3' end of the antisense strand each independently comprise 1 or 2 phosphorothioate-modified nucleotides.

In some embodiments, the modified nucleotide described herein is selected from nucleotides as follows:
in the direction from the 5' end to the 3' end, nucleotides at positions 7 and 9 of the sense strand are 2'-deoxy-2'-fluoro-modified nucleotides, a nucleotide at position 11 of the sense strand is a 2'-deoxynucleotide, and nucleotides at the rest of positions are 2'-O-methyl-modified nucleotides; and nucleotides at positions 6, 8, 9, 10, 12, 14, and 16 of the antisense strand are 2'-deoxy-2'-fluoro-modified nucleotides, and nucleotides at the rest of positions are 2'-O-methyl-modified nucleotides; or
in the direction from the 5' end to the 3' end, nucleotides at positions 7, 9, and 14 of the sense strand are 2'-deoxy-2'-fluoro-modified nucleotides, a nucleotide at position 11 of the sense strand is a 2'-deoxynucleotide, and nucleotides at the rest of positions are 2'-O-methyl-modified nucleotides; and nucleotides at positions 2, 8, 10, 12, 14, and 20 of the antisense strand are 2'-deoxy-2'-fluoro-modified nucleotides, and nucleotides at the rest of positions are 2'-O-methyl-modified nucleotides; or
in the direction from the 5' end to the 3' end, nucleotides at positions 8 and 14 of the sense strand are 2'-deoxy-2'-fluoro-modified nucleotides, a nucleotide at position 11 of the sense strand is a 2'-deoxynucleotide, and nucleotides at the rest of positions are 2'-O-methyl-modified nucleotides; and nucleotides at positions 2, 8, 10, 12, 14, and 20 of the antisense strand are 2'-
deoxy-2'-fluoro-modified nucleotides, and nucleotides at the rest of positions are 2'-O-methyl-modified nucleotides.

In some embodiments, the 5' end of the sense strand described herein comprises 1 or 2 phosphorothioate-modified nucleotides; and/or the 5' end and the 3' end of the antisense strand each independently comprise 1 or 2 phosphorothioate-modified nucleotides.

In some embodiments, the antisense strand described herein comprises any one of antisense strand nucleotide sequences shown in Table 3,
and the sense strand comprises any one of sense strand nucleotide sequences shown in Table 3; or the antisense strand comprises any one of antisense strand nucleotide sequences shown in Table 5, and the sense strand comprises any one of sense strand nucleotide sequences shown in Table 5.

In some embodiments, the sense strand and the antisense strand described herein comprise or consist of nucleotide sequences (5' → 3') selected from:
XR-1-01
   sense strand: mGmUmCmUmCmAAfmAAfmUGfmGmAmAmGmGmUmUmAmUmA (SEQ ID NO: 178); antisense strand:
   mUmAmUmAmACfmCUfUfCfmCAfmUUfmUUfmGmAmGmAmCmUmU (SEQ ID NO: 212)
XR-1-02
   sense strand: mGmUmCmUmCmAAfmAAfmUdGmGmAmAmGmGmUmUmAmUmA (SEQ ID NO: 179); antisense strand:
   mUmAmUmAmACfmCUfUfCfmCAfmUUfmUUfmGmAmGmAmCmUmU (SEQ ID NO: 213)
XR-1-03
   sense strand: mGmUmCmUmCmAmAAfmAUfdGmGmAmAmGmGmUmUmAmUmA (SEQ ID NO: 180); antisense strand:
   mUmAmUmAmACfmCUfUfCfmCAfmUUfmUUfmGmAmGmAmCmUmU (SEQ ID NO: 214)
XR-1-04
   sense strand: mG*mU*mCmUmCmAAfmAAfmUdGmGmAmAmGmGmUmUmAmUmA (SEQ ID NO: 181); antisense strand:
   mUmAmUmAmACfmCUfUfCfmCAfmUUfmUUfmGmAmGmAmCmUmU (SEQ ID NO: 215)
XR-1-05
   sense strand: mG*mU*mCmUmCmAmAAfmAUfdGmGmAmAmGmGmUmUmAmUmA (SEQ ID NO: 182); antisense strand:
   mUmAmUmAmACfmCUfUfCfmCAfmUUfmUUfmGmAmGmAmCmUmU (SEQ ID NO: 216)
XR-1-07
   sense strand: mGmUmCmUmCmAAfmAAfmUdGmGmAmAmGmGmUmUmAmUmA (SEQ ID NO: 183); antisense strand:
   mU*mA*mUmAmACfmCUfUfCfmCAfmUUfmUUfmGmAmGmAmC*mU*mU (SEQ ID NO: 217)
XR-1-08
   sense strand: mGmUmCmUmCmAAfmAAfmUdGmGmAmAmGmGmUmUmAmUmA (SEQ ID NO: 184); antisense strand:
   mUmAmUmAmACfmCUfUfCfCfAfmUUfmUUfmGmAmGmAmCmUmU (SEQ ID NO: 218)
XR-1-09
   sense strand: mGmUmCmUmCmAAfmAAfmUdGmGmAmAmGmGmUmUmAmUmA (SEQ ID NO: 185); antisense strand:
   mU*mA*mUmAmACfmCUfUfCfmCAfmUUfmUUfmGmAmGmAmC*mU*mU (SEQ ID NO: 219)
XR-2-01
   sense strand: mAmAmAmUmCmAmCGfmAmAdAmCmCAfmAmCmUmAmU (SEQ ID NO: 186); antisense strand:
   mAUfmAmGmUmUmGGfmUUfmUCfmGUfmGmAmUmUmUCfmC (SEQ ID NO: 220)
XR-2-02
   sense strand: mAmAmAmUmCmAmCGfmAmAAfmCmCAfmAmCmUmAmU (SEQ ID NO: 187); antisense strand:
   mAUfmAmGmUmUmGGfmUUfmUCfmGUfmGmAmUmUmUCfmC (SEQ ID NO: 221)
XR-2-03
   sense strand: mAmAmAmUmCmAmCGfmAAfdAmCmCAfmAmCmUmAmU (SEQ ID NO: 188); antisense strand:
   mAUfmAmGmUmUmGGfmUUfmUCfmGUfmGmAmUmUmUCfmC (SEQ ID NO: 222)
XR-2-04
   sense strand: mAmAmAmUmCmACfmGAfmAdAmCmCAfmAmCmUmAmU (SEQ ID NO: 189); antisense strand:
   mAUfmAmGmUmUmGGfmUUfmUCfmGUfmGmAmUmUmUCfmC (SEQ ID NO: 223)
XR-2-05
   sense strand: mAmAmAmUmCmAmCGfmAmAdAmCmCmAAfmCmUmAmU (SEQ ID NO: 190); antisense strand:
   mAUfmAmGmUmUmGGfmUUfmUCfmGUfmGmAmUmUmUCfmC (SEQ ID NO: 224)
XR-2-06
   sense strand: mAmAmAmUmCmAmCGfmAmAdAmCmCmAmACfmUmAmU (SEQ ID NO: 191); antisense strand:
   mAUfmAmGmUmUmGGfmUUfmUCfmGUfmGmAmUmUmUCfmC (SEQ ID NO: 225)
XR-2-07
   sense strand: mA*mA*mAmUmCmAmCGfmAmAdAmCmCAfmAmCmUmAmU(SEQ ID NO: 192); antisense strand:
   mAUfmAmGmUmUmGGfmUUfmUCfmGUfmGmAmUmUmUCfmC (SEQ ID NO: 226)
XR-2-09
   sense strand: mAmAmAmUmCmAmCGfmAmAdAmCmCAfmAmCmUmAmU (SEQ ID NO: 193); antisense strand:
   mAUfmAmGmUmUmGmGUfmUUfCfmGUfmGmAmUmUmUCfmC (SEQ ID NO: 227)
XR-2-10
   sense strand: mAmAmAmUmCmAmCGfmAmAdAmCmCAfmAmCmUmAmU (SEQ ID NO: 194); antisense strand:
   mAUfmAmGmUmUmGmGmUmUmUCfmGUfmGmAmUmUmUCfmC (SEQ ID NO: 228)
XR-2-11
   sense strand: mAmAmAmUmCmAmCGfmAmAdAmCmCAfmAmCmUmAmU (SEQ ID NO: 195); antisense strand:
   mAUfmAmGmUmUmGGfmUUfmUCfmGUfmGmAmUmUmUCfmC (SEQ ID NO: 229)
XR-2-12
   sense strand: mAmAmAmUmCmAmCGfmAmAdAmCmCAfmAmCmUmAmU (SEQ ID NO: 196); antisense strand:
   mA*Uf*mAmGmUmUmGGfmUUfmUCfmGUfmGmAmUmUmU*Cf*mC (SEQ ID NO: 230)
XR-2-13
   sense strand: mAmAmAmUmCmAmCGfmAmAdAmCmCAfmAmCmUmAmU (SEQ ID NO: 197); antisense strand:
   mA*Uf*mAmGmUmUmGGfmUUfmUCfmGUfmGmAmUmUmUCf*mC (SEQ ID NO: 231)
XR-2-14
   sense strand: mAmAmAmUmCmAmCGfmAmAdAmCmCAfmAmCmUmAmU (SEQ ID NO: 198); antisense strand:
   mA*UfmAmGmUmUmGGfmUUfmUCfmGUfmGmAmUmUmU*Cf*mC (SEQ ID NO: 232)
XR-2-15
   sense strand: mAmAmAmUmCmAmCGfmAmAdAmCmCAfmAmCmUmAmU (SEQ ID NO: 199); antisense strand:
   mA*UfmAmGmUmUmGGfmUUfmUCfmGUfmGmAmUmUmUCf*mC (SEQ ID NO: 233)
XR-3-01
   sense strand: mAmAmAmUmCmACfmGAfmAdAmCmCAfmAmCmUmAmU (SEQ ID NO: 200); antisense strand:
   mA*Uf*mAmGmUmUmGGfmUUfmUCfmGUfmGmAmUmUmU*Cf*mC (SEQ ID NO: 234)
XR-3-02
   sense strand: mA*mAmAmUmCmACfmGAfmAdAmCmCAfmAmCmUmAmU (SEQ ID NO: 201); antisense strand:
   mA*Uf*mAmGmUmUmGGfmUUfmUCfmGUfmGmAmUmUmU*Cf*mC (SEQ ID NO: 235)
XR-3-03
   sense strand: mAmA*mAmUmCmACfmGAfmAdAmCmCAfmAmCmUmAmU (SEQ ID NO: 202); antisense strand:
   mA*Uf*mAmGmUmUmGGfmUUfmUCfmGUfmGmAmUmUmU*Cf*mC (SEQ ID NO: 236)
XR-3-04
   sense strand: mA*mA*mAmUmCmACfmGAfmAdAmCmCAfmAmCmUmAmU (SEQ ID NO: 203); antisense strand:
   mA*Uf*mAmGmUmUmGGfmUUfmUCfmGUfmGmAmUmUmU*Cf*mC (SEQ ID NO: 237)
XR-3-05
   sense strand: mAmAmAmUmCmACfmGAfmAdAmCmCAfmAmCmUmAmU (SEQ ID NO: 204); antisense strand:
   mA*Uf*mAmGmUmUmGGfmUUfmUmCmGUfmGmAmUmUmU*Cf*mC (SEQ ID NO: 238)
XR-4-0 1
   sense strand: mGmUmCmUmCmAAfmAAfmUdGmGmAmAmGmGmUmUmAmUmA (SEQ ID NO: 205); antisense strand:
   mU*mA*mUmAmACfmCUfUfCfmCAfmUUfmUUfmGmAmGmAmC*mU*mU (SEQ ID NO: 239)
XR-4-02
   sense strand: mG*mUmCmUmCmAAfmAAfmUdGmGmAmAmGmGmUmUmAmUmA (SEQ ID NO: 206);
   antisense strand:
      mU*mA*mUmAmACfmCUfUfCfmCAfmUUfmUUfmGmAmGmAmC*mU*mU (SEQ ID NO: 240)
XR-4-03
   sense strand: mGmU*mCmUmCmAAfmAAfmUdGmGmAmAmGmGmUmUmAmUmA (SEQ ID NO: 207);
   antisense strand:
      mU*mA*mUmAmACfmCUfUfCfmCAfmUUfmUUfmGmAmGmAmC*mU*mU (SEQ ID NO: 241)
XR-4-04
   sense strand: mG*mU*mCmUmCmAAfmAAfmUdGmGmAmAmGmGmUmUmAmUmA (SEQ ID NO: 208);
   antisense strand:
      mU*mA*mUmAmACfmCUfUfCfmCAfmUUfmUUfmGmAmGmAmC*mU*mU (SEQ ID NO: 242)
XR-4-05
   sense strand: mGmUmCmUmCmAAfmAAfmUdGfGmAmAmGmGmUmUmAmUmA (SEQ ID NO: 209);
   antisense strand:
      mU*mA*mUmAmACfmCUfUfCfmCAfmUUfmUUfmGmAmGmAmC*mU*mU (SEQ ID NO: 243)
XR-4-06
   sense strand: mGmUmCmUmCmAAfmAAfmUdGmGfAmAmGmGmUmUmAmUmA (SEQ ID NO: 210);
   antisense strand:
      mU*mA*mUmAmACfmCUfUfCfmCAfmUUfmUUfmGmAmGmAmC*mU*mU (SEQ ID NO: 244)
XR-4-07
   sense strand: mGmUmCmUmCmAAfmAAfmUdGmGmAmAmGmGmUmUmAmUmA (SEQ ID NO: 211); antisense strand:
   mU*mA*mUmAmACfmCUfUfCfmCAfmUUfmUUfmGmAmGmAmC*mU*mU (SEQ ID NO: 245),
wherein C, G, U, and A indicate cytidine-3'-phosphate, guanosine-3'-phosphate, uridine-3'-phosphate, and adenosine-3'-phosphate, respectively; m indicates that one adjacent nucleotide on the right side of the letter m is a 2'-O-methyl-modified nucleotide; f indicates that one adjacent nucleotide on the left side of the letter f is a 2'-deoxy-2'-fluoro-modified nucleotide; * indicates that one adjacent nucleotide on the left side of * is a phosphorothioate-modified nucleotide; f* indicates that one adjacent nucleotide on the left side of f* is a phosphorothioate- and 2'-fluoro-modified nucleotide; d indicates that one adjacent nucleotide on the right side of the letter d is a 2'-deoxyribonucleotide.

In some embodiments, the sense strand and the antisense strand described herein are each independently of 17-25 nucleotides in length; preferably, the sense strand and the antisense strand are each independently of 19-23 nucleotides in length.

In yet another aspect, the present invention provides an siRNA conjugate comprising the siRNA described herein and a targeting group.

In some embodiments, the targeting group described herein is a ligand with affinity for an asialoglycoprotein receptor.

In some embodiments, the targeting group comprised in the siRNA conjugate disclosed herein comprises a group derived from a lipophil, wherein the lipophil is selected from cholesteryl, cholic acid, amantanoacetic acid, 1-pyrenebutanoic acid, dihydrotestosterone, 1,3-*bis-*O(hexadecyl)glycerol, geranyloxyhexyl, hexadecyl glycerol, borneol, menthol, 1,3-propanediol, heptadecyl, palmitic acid, myristic acid, O-3-(oleoyl)lithocholic acid, O-3-(oleoyl)cholic acid, dimethoxytribenzyl, and phenoxazine. It should be understood that the "group derived from a lipophil" refers to a monovalent group formed after removal of an atom or a group from the lipophil, which retains the original biological activity and function of the lipophil. One skilled in the art can readily identify the atom or the group on the lipophil that can be removed such that the site at which the resulting lipophil group is linked to the rest of the siRNA conjugate does not affect the biological function of the lipophil group as a lipophil. For example, the lipophil group can be covalently linked to the rest of the siRNA conjugate through a group such as a hydroxyl group in its carboxyl group or a hydroxyl group on the compound in the form of an ester group (-COO-), an ether (-O-), an amide group (-CO-NH-), or the like.

In some embodiments, the targeting group of the present invention comprises a group derived from a carbohydrate, wherein the carbohydrate is selected from allose, altrose, arabinose, cladinose, erythrose, erythrulose, fructose, D-fucitol, L-fucitol, fucosamine, fucose, fuculose, galactosamine, D-galactosaminol, A-acetyl-galactosamine (GalNAc), galactose, glucosamine, *N*-acetyl-glucosamine, glucosaminitol, glucose, glucose-6-phosphate, gulonoglyceraldehyde, L-glycero-D-mannose-heptose, glycerol, glycerone, gulose, idose, lyxose, mannosamine, mannose, mannose-6-phosphate, psicose, quinovose, quinovosamine, rhamnitol, rhamnosamine, rhamnose, ribose, ribulose, sedoheptulose, sorbose, tagatose, talose, tartaric acid, threose, xylose, and xylulose. Specifically, the targeting group is a ligand group comprising N acetyl-galactosamine (GalNAc). It should be understood that the "group derived from a carbohydrate" refers to a monovalent group formed after removal of an atom or a group, which retains the original biological activity and function of the carbohydrate. One skilled in the art can readily identify the atom or the group on each carbohydrate that can be removed such that the site at which the resulting carbohydrate group is linked to the rest of the siRNA conjugate does not affect the biological function of carbohydrate group as a carbohydrate. For example, the carbohydrate group can be covalently linked to the rest of the siRNA conjugate through O in the hydroxyl group in the form of an ether. The hydroxyl groups linked to the ring carbon atoms adjacent to the epoxy atom are typically selected for the linkage. An exemplary ligand group derived from *N*-acetyl-galactosamine (GalNAc) is shown as follows: 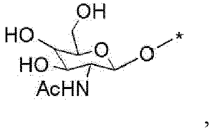 wherein * indicates a position at which the ligand group is linked to the rest of the siRNA conjugate.

In some other embodiments, the siRNA conjugate disclosed herein specifically binds to a specific receptor of a specific tissue, thereby achieving tissue-specific targeting. In some embodiments, the conjugate of the present invention specifically targets a receptor on the surface of hepatocytes and thus specifically targets a liver tissue. In some embodiments, the conjugate of the present invention specifically targets an asialoglycoprotein receptor (ASGPR) on the surface of hepatocytes. In some embodiments, the targeting group is a ligand group comprising a group derived from *N* acetyl-galactosamine (GalNAc).

In some embodiments, the targeting group described herein comprises the following structures: 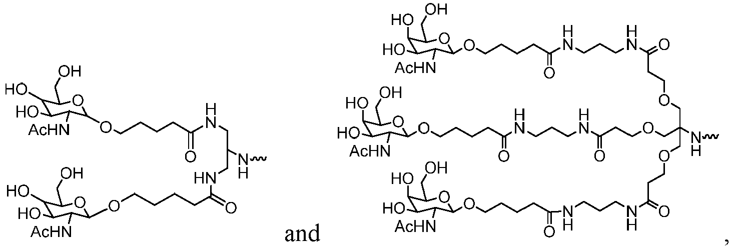 wherein the wavy line indicates a position at which the targeting group is linked to the linker. Generally, the targeting group is linked to the linker in the form of an amide bond.

In some embodiments, the siRNA conjugate described herein further comprises a linker, and the siRNA, the linker, and the targeting group are sequentially covalently or non-covalently linked.

In some embodiments, the linker described herein is selected from: 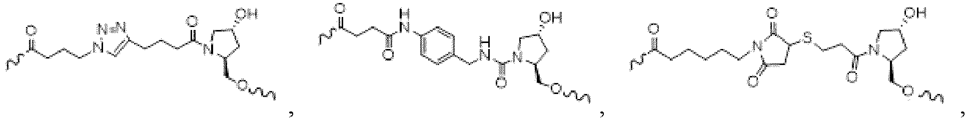 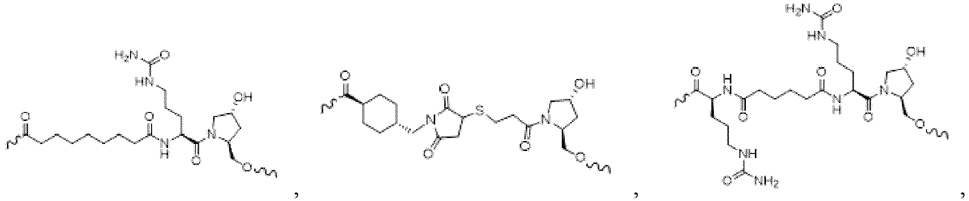 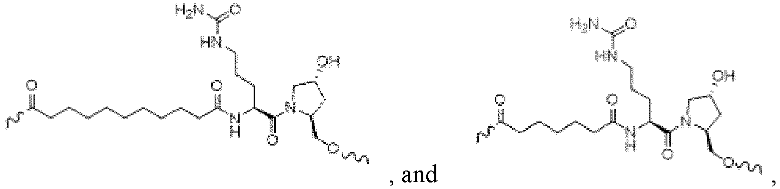 wherein the wavy line indicates a position at which the linker is linked to the rest of the siRNA conjugate, wherein the targeting group is covalently linked to the carbonyl group of the linker, and the siRNA is covalently linked to O at the other end of the linker through a phosphoester bond.

In some embodiments, the siRNA is linked, at the 3' end of a sense strand thereof, to the targeting group or the linker via a phosphonyl group. In some embodiments, the targeting group is linked to the linker in the form of an amide bond.

In some embodiments, the targeting group described herein is independently a ligand with affinity for an asialoglycoprotein receptor.

The present invention discloses an siRNA conjugate for delivering an siRNA or an active molecular group. In some embodiments, the siRNA conjugate disclosed herein facilitates tissue-specific targeting. In some embodiments, the targeting group disclosed herein binds to a cell surface receptor. Thus, any cell surface receptor or biomarker, or a targeting group corresponding to a portion thereof, is considered suitable for use in the present invention.

Specifically, the conjugates for targeted delivery of the siRNA to the liver include, but are not limited to, examples of compound structures in Table A, wherein R² is the siRNA as defined herein.

**Table A: Examples of siRNA conjugates**

| | |
|---|---|
| 1 | 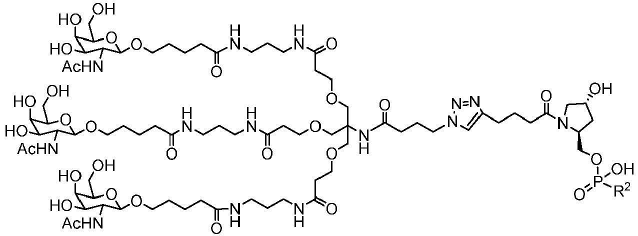 |
| 2 | 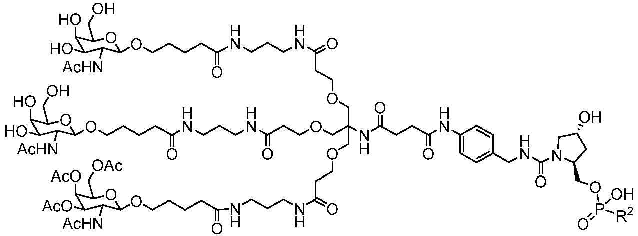 |
| 3 | 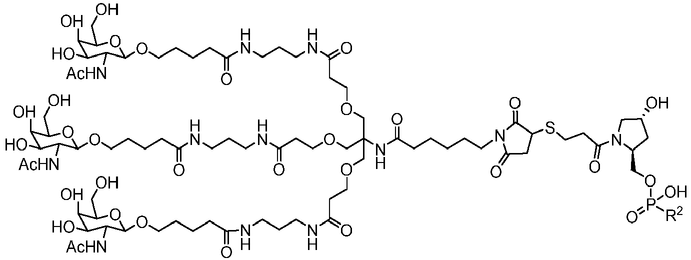 |
| 4 | 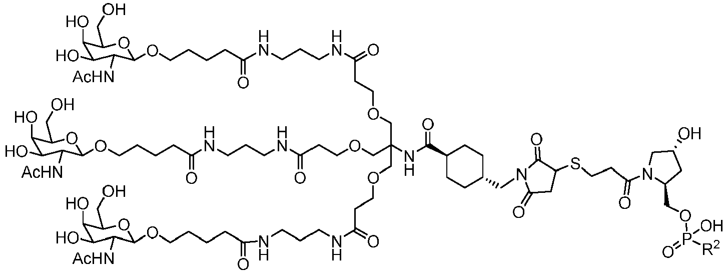 |
| 5 | 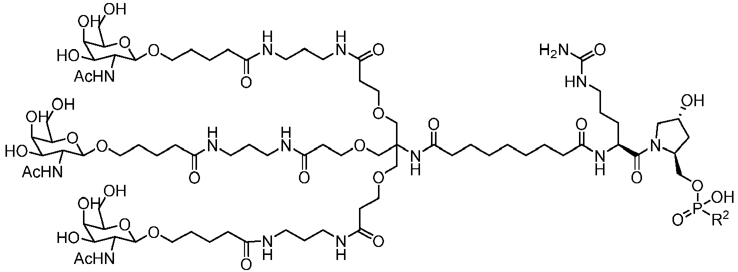 |
| 6 | 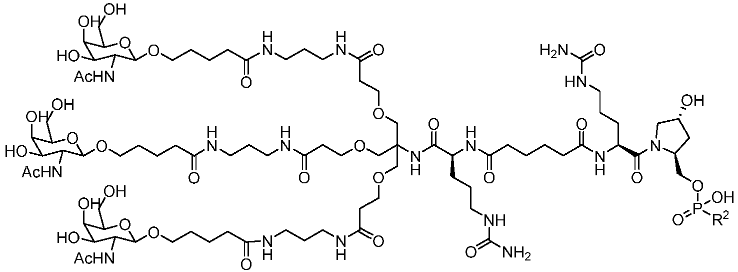 |
| 7 | 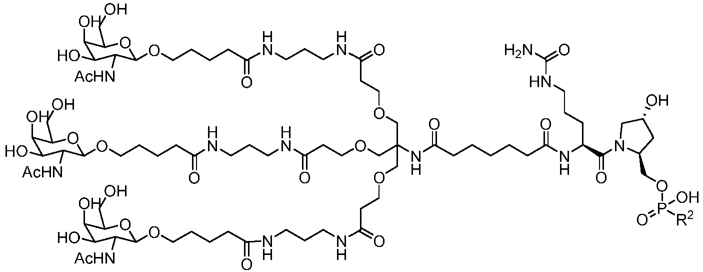 |
| 8 | 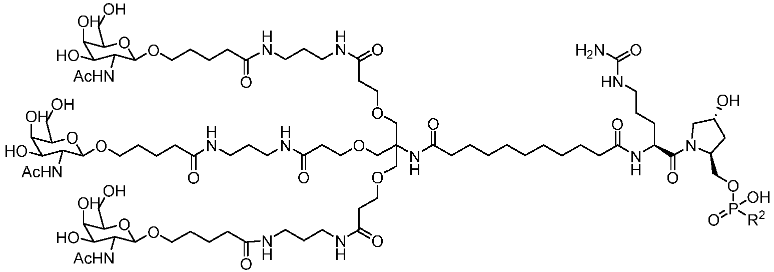 |

The compounds described herein include, but are not limited to, optical isomers, racemic compounds and other mixtures thereof. In these cases, single enantiomers or diastereomers, i.e., optically active configurations, can be obtained by asymmetric synthesis or chiral resolution. Resolution of the racemates can be achieved, for example, by conventional methods such as recrystallization in the presence of a resolving agent, or using column chromatography such as chiral high-pressure liquid chromatography (HPLC). In addition, some compounds containing carbon-carbon double bonds have Z- and E-configurations (or cis- and trans-configurations). When tautomerism exists in the compound described herein, the term "compound" (including conjugates) includes all tautomeric forms of the compound. Such compounds also include crystals and chelates. Similarly, the term "salt" includes all tautomeric forms of the compound and crystal forms of the compound. In the structures shown in Table 1, amino acid residues are derived from L-amino acids, D-amino acids, dl-amino acids, and any combination thereof, and the specific structures disclosed above do not limit the configuration of particular amino acids.

In some embodiments, R² comprises any one of antisense strand nucleotide sequences shown in Table 1, 2 or 3, and any one of sense strand nucleotide sequences shown in Table 1, 2 or 3.

In some embodiments, R² comprises any one of nucleotide sequences of the sense strand and the antisense strand of the siRNAs shown in Table 3. Preferably, the siRNA is selected from an siRNA indicated by any one of siRNA numbers in Table 3.

In some embodiments, R² comprises a sense strand sequence set forth in SEQ ID NO: 188 and an antisense strand sequence set forth in SEQ ID NO: 222.

In some embodiments, R² comprises a sense strand sequence set forth in SEQ ID NO: 200 and an antisense strand sequence set forth in SEQ ID NO: 234.

In some embodiments, R² comprises a sense strand sequence set forth in SEQ ID NO: 201 and an antisense strand sequence set forth in SEQ ID NO: 235.

In some embodiments, R² comprises a sense strand sequence set forth in SEQ ID NO: 205 and an antisense strand sequence set forth in SEQ ID NO: 239.

The present invention also provides a pharmaceutical composition, which comprises the siRNA or siRNA conjugate described herein, and a pharmaceutically acceptable carrier thereof.

In yet another aspect, the present invention provides a pharmaceutical combination for inhibiting the expression of an ANGPTL3 gene, which comprises
the siRNA, the siRNA conjugate, or the pharmaceutical composition thereof described herein, and one or more additional therapeutic agents for inhibiting the expression of an ANGPTL3 gene.

In yet another aspect, the present invention provides use of the siRNA, the siRNA conjugate, the pharmaceutical composition, or the pharmaceutical combination described herein in the preparation of a medicament for treating and/or preventing an ANGPTL3-mediated disease or disorder.

In yet another aspect, the present invention provides a method for treating and/or preventing an ANGPTL3-mediated disease or disorder, which comprises administering to a subject in need thereof the siRNA, the siRNA conjugate, the pharmaceutical composition, or the pharmaceutical combination described herein.

In yet another aspect, the present invention provides the siRNA, the siRNA conjugate, the pharmaceutical composition, or the pharmaceutical combination described herein, for use in treating and/or preventing an ANGPTL3-mediated disease or disorder.

In yet another aspect, the present invention provides a method for inhibiting the expression of an ANGPTL3 gene in a subject, which comprises administering to the subject in need thereof the siRNA, the siRNA conjugate, the pharmaceutical composition, or the pharmaceutical combination described herein.

In some embodiments, the disease or disorder described herein includes atherosclerosis, hypercholesterolemia, hypertriglyceridemia, acute coronary syndrome, dyslipidemia, myocardial infarction, coronary artery lesion, stroke, coronary artery disease, cardiovascular disease, diabetes, hyperlipidemia, type 2 diabetes, and kidney disease.

### BRIEF DESCRIPTION OF THE DRAWINGS

FIG. 1: effect of siRNA conjugates on the level of ANGPTL3 protein in plasma in mice.
FIG. 2: effect of siRNA conjugates on the level of triglyceride in plasma in mice.
FIG. 3: effect of siRNA conjugates on the level of LDL-c in plasma in mice.
FIG. 4: effect of siRNA conjugates on the level of HDL-c in plasma in mice.
FIG. 5: effect of siRNA conjugates on the level of total cholesterol in plasma in mice.

### DETAILED DESCRIPTION

### Definitions

Unless otherwise stated, embodiments of the present invention will employ conventional techniques of molecular biology (including recombinant techniques), microbiology, cytobiology, biochemistry, and immunology, which are all within the skill of the art.

In order to facilitate the understanding of the present invention, some technical and scientific terms are specifically defined as follows. Unless otherwise
specifically defined herein, all technical and scientific terms used herein have the same meanings as commonly understood by those of ordinary skill in the art to which the present invention belongs. The singular forms used herein (including claims) include their plural forms, unless otherwise specified in the context explicitly. In addition, it should be noted that whenever a value or range of values of a parameter is recited, it is intended that the value and the intermediate value of the range of values recited are also part of the present invention.

The term "about" used in combination with a numerical value is intended to encompass the numerical values in a range from a lower limit less than the specified numerical value by 5% to an upper limit greater than the specified numerical value by 5%, including but not limited to ±5%, ±2%, ±1%, and ±0.1%, as these variations are suitable for implementing the disclosed methods.

The term "and/or" should be understood as any one of the options or a combination of any two or more of the options.

The term "or" should be understood as having the same meaning as "and/or" defined above. For example, when items in a list are separated, "or" or "and/or" should be interpreted as being inclusive, that is, including at least one number or one of a list of elements, but also including more than one, and, optionally, additional unlisted items. Only if the contrary term is explicitly indicated, such as "only one" or "exactly one" or "consisting of ..." in the claims, will it refer to only one number listed or one element of a list.

As used herein, the terms "a" and "an" should be interpreted as "at least one", unless the contrary is explicitly indicated.

The term "including" or "comprising" is intended to be used interchangeably with the phrase "including, but not limited to".

The term "at least" preceding a number or series of numbers is understood as including numbers that are adjacent to the term "at least" as well as all subsequent numbers or integers that may be logically included, as is clear from the context. For example, the number of nucleotides in a nucleic acid molecule must be an integer. For example, "at least 18 nucleotides of 21 nucleotides of a nucleic acid molecule" means that 18, 19, 20, or 21 nucleotides have the specified properties. When "at least" appears before a series of numbers or a range, it should be understood that at least each number in the series or range can be modified.

The term "ANGPTL3" refers to an angiopoietin-like protein 3 having an amino acid sequence of any vertebrate or mammalian source, including, but not limited to, humans, cattle, chickens, rodents, mice, rats, pigs, sheep, primates, monkeys and guinea pigs, unless otherwise stated. The term also refers to fragments or variants of natural ANGPTL3 that retain at least one *in vivo* or *in vitro* activity of natural ANGPTL3. The term encompasses the full-length unprocessed precursor form of ANGPTL3 as well as the mature form resulting from post-translational cleavage of the signal peptide and the form resulting from proteolytic processing of the fibrinogen-like domain. A sequence of a human ANGPTL3 mRNA transcript can be found, for example, in GenBank Accession No. GI:452408443 (NM-014495.3; SEQ ID NO: 1). A predicted sequence of rhesus monkey ANGPTL3 mRNA can be found, for example, in GenBank Accession No. GI:297278846 (XM-001086114.2; SEQ ID NO: 2). As used herein, "ANGPTL3" also refers to a specific polypeptide expressed in a cell through naturally occurring DNA sequence variations of an ANGPTL3 gene, such as a single nucleotide polymorphism of an ANGPTL3 gene.

The term "interfering RNA" or "RNAi" or "interfering RNA sequence" includes single-stranded RNA (e.g., mature miRNA, ssRNAi oligonucleotide, or ssDNAi oligonucleotide) or double-stranded RNA (i.e., duplex RNA such as siRNA, dsRNA, shRNA, aiRNA, or precursor miRNA) that is capable of reducing or inhibiting the expression of a target gene or sequence (e.g., by mediating the degradation and inhibition of translation of mRNA complementary to the interfering RNA sequence) when the interfering RNA is in the same cell as the target gene or sequence. Thus, interfering RNA refers to single-stranded RNA complementary to a target mRNA sequence or double-stranded RNA formed from two complementary strands or from a single self-complementary strand.

Interfering RNA includes "small interfering RNA" or "siRNA", each strand of which comprises about 15 to about 60 nucleotides (e.g., being of about 15-60, 15-50, 15-40, 15-30, 15-25, 17-25, 19-25, 17-23, 17-21, 19-23, or 19-21 nucleotides in length, or 15, 16, 17, 18, 19, 20, 21, 22, 23, 24, or 25 nucleotides in length). The range, ranges of lengths, and intermediate lengths recited above are also contemplated as part of the present invention. In one specific embodiment, the siRNA is chemically synthesized. The siRNA of the present invention are capable of silencing the expression of a target sequence *in vitro* and/or *in vivo.* In other embodiments, the siRNA comprises at least one modified nucleotide, e.g., the siRNA comprises one, two, three, four, five, six, seven, eight, nine, ten, or more modified nucleotides in a double-stranded region.

As used herein, the term "dsRNA" or "precursor RNAi molecule" is intended to include any precursor molecule that is processed *in vivo* by an endonuclease to produce an active siRNA.

The term "siRNA" refers to a molecule that contains siRNA as the term as defined herein, which mediates targeted cleavage of RNA transcripts by an RNA-induced silencing complex (RISC) pathway. iRNA directs sequence-specific degradation of mRNA through a process known as RNA interference (RNAi). iRNA regulates (e.g., inhibits) the expression of ANGPTL3 in a cell (e.g., a cell in a subject, such as a mammalian subject).

Generally, the majority of the nucleotides of each strand of the siRNA are ribonucleotides, but as detailed herein, each or both of the two strands may also include one or more non-ribonucleotides, e.g., deoxyribonucleotides and/or modified nucleotides. In addition, as used in this specification, "siRNA" may include ribonucleotides with chemical modifications; the siRNA may include substantial modifications at multiple nucleotides. As used herein, the term "modified nucleotide" refers to a nucleotide that independently has one modified sugar moiety, one modified internucleotide linkage, and/or one modified nucleobase. Thus, the term "modified nucleotide" encompasses an internucleoside linkage, a substitution, addition or removal of, for example, a functional group or atom of a sugar moiety or nucleobase. Modifications suitable for use in the present invention include all types of modifications disclosed herein or known in the art.

The term "antisense strand" refers to a strand of an iRNA (e.g., an siRNA) that includes a region that is substantially complementary to a target sequence (e.g., an ANGPTL3 mRNA). As used herein, the term "complementary region" refers to a region on the antisense strand that is substantially complementary to a sequence. Where the complementary region is not fully complementary to the target sequence, there may be mismatches within or at terminal regions of the molecule. Generally, the most tolerated mismatches are present in the terminal regions, e.g., within 5, 4, 3, or 2 nucleotides of the 5'- and/or 3'-end of the siRNA.

The term "sense strand" refers to an iRNA strand comprising a region substantially complementary to a region of an antisense strand as defined herein.

The antisense and sense strands of the siRNA can be of the same or different lengths, as described herein and as known in the art.

As used herein, and unless otherwise specified, when used to describe a first nucleotide sequence related to a second nucleotide sequence, the term "complementarity" refers to the ability of an oligonucleotide or polynucleotide comprising the first nucleotide sequence to hybridize and form a duplex structure under certain conditions with an oligonucleotide or polynucleotide comprising the second nucleotide sequence. Such conditions may be, for example, stringent conditions, wherein the stringent conditions may comprise: 400 mM NaCl, 40 mM PIPES, pH 6.4, 1 mM EDTA, 50 °C or 70 °C for 12-16 h.

The term "conjugate" as used herein corresponds to "conjugate" or "conjugates" in English. The conjugate refers to a new compound generated after two or more compound molecules are covalently linked (conjugated) through a bivalent or multivalent compound molecule with the function of linkage. The conjugate can also be generated directly from two molecules via conjugation or condensation. The common antibody-drug conjugate (ADC) is a conjugate, also known as an antibody drug conjugate. In the present invention, the product resulting from the conjugation of the siRNA molecule with the targeting group via a linker is also a conjugate or an siRNA conjugate. In some embodiments, the product resulting from the conjugation of the siRNA with the targeting group is an siRNA conjugate.

The term "conjugation" used herein refers to a chemical process in which two or more compound molecules undergo a certain reaction to form a new chemical bond and a new molecule. In certain contexts, "conjugation" may be used interchangeably with "linkage" or in place of one another.

The term "carbohydrate" refers to a monosaccharide, a disaccharide, a trisaccharide, or a polysaccharide.

The term "monosaccharide" includes allose, maltose, arabinose, cladinose, brown sugar, erythrose, fructose, D-fucoitol, L-fucoitol, fucosamine, fucose, galactosamine, D-galactosaminol, *N*-acetyl-galactosamine, galactose, glucosamine, *N*-acetyl-glucosamine, glucosaminitol, glucose, glucose-6-phosphate, glucose glyceraldehyde, L-glycerol-D-mannose-heptose, glycerol, glucose, iodine, lyxose, mannosamine, mannose, mannose-6-phosphate, psicose, quinovose, quinovosamine, rhamnitol, rhamnose, ribose, ribulose, heptose, sorbose, tagatose, talose, tartaric acid, threose, xylose and xylulose. The monosaccharide may be in the D- or L-configuration. The monosaccharide may also be a deoxy sugar (alcoholic hydroxyl substituted with hydrogen), an amino sugar (alcoholic hydroxyl substituted with amino), a thio sugar (alcoholic hydroxyl substituted with thiol, or CO substituted with CS, or a cyclic epoxy substituted with sulfur), a seleno sugar, a tellurose, an aza sugar (ring carbon substituted with nitrogen), an imino sugar (epoxy substituted with nitrogen), a phosphido sugar (ring carbon substituted with phosphorus), a C-substituted monosaccharide (hydrogen on a non-terminal carbon atom substituted with carbon), an unsaturated monosaccharide, a sugar alcohol (carbonyl substituted with CHOH group), an aldonic acid (aldehyde substituted with carboxyl), a ketoaldonic acid, an uronic acid, an aldonic acid, or the like. The amino sugar includes amino monosaccharides, and is preferably galactosamine, glucosamine, mannosamine, fucosamine, quinovosamine, neuraminic acid, muramic acid, lactosamine, acosamine, bacillosamine, daunomamine, desosamine, forosamine, aminocarboxamide, carnosamine, mannosamine, trehalose, mycosamine, peroxidase amine, pneumoconimine, purinamine, or rhodamine. It should be understood that monosaccharides and the like may be further substituted.

The terms "disaccharide", "trisaccharide", and "polysaccharide" include ubiquinose, acarbose, glucosamine, amylopectin, amylose, apiose, ascose, ascorbic acid, flavone sugar, cellobiose, cellotriose, cellulose, chacotriose, thioether, chitin, collagen, cyclodextrin, melamine, dextrin, 2-deoxyribose, 2-deoxyglucose, diglucose, maltose, digital ketose, EVALOSE, fructus evodiae, fructo-oligosaccharide, galacto-oligosaccharide, gentianose, gentiobiose, dextran, glycogen, hamamelose, heparin, inulin, isoevodia sapogenin, isomaltose, isomaltotriose, isopentanose, monascus polysaccharide, lactose, lactosamine, layered arabinose, levoglucosan, levoglucosenone, maltose, mannooligosaccharide, mannotriose, melibiose, muramic acid, trehalose, neuraminic acid, black glucose, nojirimycin, sophorose, stachyose, streptococcal sugar, sucrose, and trehalose. Furthermore, it should be understood that "disaccharide", "trisaccharide", and "polysaccharide" may be further substituted. The disaccharide also includes amino sugars and derivatives thereof, particularly mycosamine sugar derived at the C-4' position or 4-deoxy-3-amino-glucose derived at the C-6' position.

The phrase "inhibiting the expression of an ANGPTL3 gene" as used herein includes inhibiting the expression of any ANGPTL3 gene (such as, a mouse ANGPTL3 gene, a rat ANGPTL3 gene, a monkey ANGPTL3 gene, or a human ANGPTL3 gene) and variants or mutants of an ANGPTL3 gene encoding an ANGPTL3 protein.

"Inhibiting the expression of an ANGPTL3 gene" comprises any level of ANGPTL3 gene inhibition, e.g., at least partial inhibition, such as at least about 20% inhibition, of the expression of an ANGPTL3 gene. In certain embodiments, the inhibition is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

The expression of the ANGPTL3 gene can be evaluated based on the level of any variable associated with the expression of the ANGPTL3 gene, e.g., the level of an ANGPTL3 mRNA or the level of an ANGPTL3 protein. The expression of the ANGPTL3 gene can also be evaluated indirectly based on the level of serum lipid, triglyceride, cholesterol (including LDL-C, HDL-C, VLDL-C, IDL-C, and total cholesterol), or free fatty acid. The inhibition can be evaluated by an absolute or relative reduction in one or more of these variables as compared to a control level. The control level may be any type of control level used in the art, such as a pre-administration baseline level or a level determined from a similar subject, cell, or sample untreated or treated with a control (e.g., buffer-only control or inert agent control).

A "subject" includes any human or non-human animal. The term "non-human animal" includes all vertebrates, e.g., mammals and non-mammals, such as non-human primates, sheep, dog, cat, horse, cattle, chicken, amphibians, and reptiles. As used herein, the term "cyno" refers to a cynomolgus monkey.

"Therapeutically effective amount", "therapeutically effective dose", and "effective amount" refer to an amount of the siRNA of the present invention that is effective in preventing or ameliorating one or more symptoms of a disease or condition or the progression of the disease or condition when administered alone or in combination with other therapeutic agents to a cell, a tissue, or a subject. The therapeutically effective dose also refers to an amount of the antibody or the antigen-binding fragment thereof sufficient to cause amelioration of symptoms, e.g., an amount for treating, curing, preventing or ameliorating a related condition or promoting the treatment, cure, prevention or amelioration of such condition. When an active ingredient is administered to an individual alone, a therapeutically effective dose refers to the amount of the ingredient. In the case of administration in combination, a therapeutically effective dose refers to the combined amount of active ingredients that produces a therapeutic effect, regardless of whether these active ingredients are administered in combination, sequentially or simultaneously. An effective amount of a therapeutic agent will increase a diagnostic index or parameter by at least 10%, generally at least 20%, preferably at least about 30%, more preferably at least 40%, and most preferably at least 50%.

The term "treating" or "treatment" means that an advantageous or desired result includes, such as reducing the level of triglyceride in a subject. The term "treating" or "treatment" also includes, but is not limited to, alleviation or amelioration of one or more symptoms of a lipid metabolism disorder, such as a reduction in the size of eruptive xanthomas. "Treatment" also refers to extended survival as compared to the expected survival without treatment.

The term "preventing" or "prevention", when used in reference to a disease, disorder, or condition thereof that may benefit from a reduction in the expression of an ANGPTL3 gene, refers to reducing the probability that a subject will develop symptoms associated with such a disease, disorder, or condition, e.g., hypertriglyceride levels or eruptive xanthomas. The reduced probability of developing hypertriglyceride levels or eruptive xanthomas is, for example, either the inability to develop hypertriglyceride levels or eruptive xanthomas, or the development of hypertriglyceride levels or eruptive xanthomas of lower severity relative to a population having the same risk factor but not receiving the treatment as described herein, when an individual has one or more of the risk factors for hypertriglyceride levels or eruptive xanthomas. Failure to develop a disease, disorder, or condition, or a reduction in the development of symptoms associated with the disease, disorder, or condition (e.g., a reduction of at least about 10% in clinically acceptable proportions for the disease or condition), or exhibition of a delay in symptoms (e.g., a delay of days, weeks, months, or years) is considered effective prevention.

The phrase "pharmaceutically acceptable carrier" as used herein refers to a pharmaceutically acceptable material, composition, or vehicle, such as a liquid or solid filler, diluent, excipient, manufacturing aid (e.g., lubricant, talc magnesium, calcium or zinc stearate, or stearic acid), or solvent encapsulating material (involving carrying or transporting a subject compound from one organ or portion of the body to another organ or portion of the body).

### siRNA and Modified Nucleotide

Provided herein is an siRNA for inhibiting the expression of an ANGPTL3 gene. Each siRNA comprises a sense strand and an antisense strand. The sense and antisense strands may each be of 17-25 nucleotides in length. The sense and antisense strands may be of the same length, or they may be of different lengths. In some embodiments, the sense and antisense strands are each independently of 17-25 nucleotides in length. In some embodiments, the sense and antisense strands are each independently of 19-23 nucleotides in length. In some embodiments, the sense strand is of 19-21 nucleotides in length and the antisense strand is of 19-23 nucleotides in length. In some embodiments, the sense strand is of about 19 nucleotides in length and the antisense strand is of about 20 nucleotides in length. In some embodiments, the sense strand is of about 19 nucleotides in length and the antisense strand is of about 21 nucleotides in length. In some embodiments, the sense strand is of about 20 nucleotides in length and the antisense strand is of about 21 nucleotides in length. In some embodiments, the sense strand is of about 20 nucleotides in length and the antisense strand is of about 22 nucleotides in length. In some embodiments, the sense strand is of 21 nucleotides in length and the antisense strand is of 22 nucleotides in length. In some embodiments, the sense strand is of 21 nucleotides in length and the antisense strand is of 23 nucleotides in length. In some embodiments, the sense and antisense strands of the siRNA are each independently of 15, 16, 17, 18, 19, 20, 21, 22, 23, 24, 25, 26, or 27 nucleotides in length. In some embodiments, the siRNA duplex has about 15, 16, 17, 18, 19, 20, 21, 22, 23, or 24 nucleotides. In some embodiments, the sense and antisense strands have a length of at least 15, 16, 17, 18, 19, 20, or 21 nucleotides complementary.

Examples of nucleotide sequences used to form siRNAs are provided in Tables 1, 2, and 3.

In some embodiments, the antisense strand of the siRNA disclosed herein differs from any one of the antisense strand sequences shown in Tables 1, 2, and 3 by 0, 1, 2, or 3 nucleotides. In some embodiments, the sense strand of the ANGPTL3 siRNA disclosed herein differs from any one of sense strand sequences shown in Table 1 by 0, 1, 2, or 3 nucleotides.

In some embodiments, the antisense strand of the siRNA comprises or consists of any one of antisense strand nucleotide sequences shown in Tables 1, 2, and 3. In some embodiments, the antisense strand of the siRNA comprises a sequence at positions 1-17, positions 2-15, positions 2-17, positions 1-18, positions 2-18, positions 1-19, positions 2-19, positions 1-20, positions 2-20, positions 1-21, positions 2-21, positions 1-22, positions 2-22, positions 1-23, positions 2-23, positions 1-24, or positions 2-24 (from the 5' end → the 3' end) of any one of antisense strand nucleotide sequences in Tables 1, 2, and 3. In certain embodiments, the antisense strand of the siRNA comprises or consists of a modified sequence of any one of the sequences in Tables 1, 2, and 3.

In some embodiments, the sense strand of the siRNA comprises or consists of any one of the sense strand nucleotide sequences shown in Tables 1, 2, and 3. In some embodiments, the sense strand of the siRNA comprises a sequence at positions 1-17, positions 2-15, positions 2-17, positions 1-18, positions 2-18, positions 1-19, positions 2-19, positions 1-20, positions 2-20, positions 1-21, positions 2-21, positions 1-22, positions 2-22, positions 1-23, positions 2-23, positions 1-24, or positions 2-24 (from the 5' end → the 3' end) of any one of the sense strand nucleotide sequences in Tables 1, 2, and 3. In certain embodiments, the sense strand of the siRNA comprises or consists of a modified sequence of any one of the sequences in Tables 1, 2, and 3.

In some embodiments, the siRNA is prepared or provided as a salt, a mixed salt, or a free acid. In some embodiments, the siRNA is prepared as a sodium salt. Such a form is well known in the art and is within the scope of the invention disclosed herein.

In some embodiments, the siRNA comprises one or more modified nucleotides. In some embodiments, the modified nucleotides include, but are not limited to: a 2'-O-methyl-modified nucleotide, a 2'-dexoy-2'-fluoro-modified nucleotide, , a 2'-deoxynucleotide, a 2'-methoxyethyl-modified nucleotide, a 2'-amino-modified nucleotide, a 2'-alkyl-modified nucleotide, a 2'-alkoxy-modified nucleotide, a 2'-F-arabinonucleotide, an abasic nucleotide, a morpholino nucleotide, and a locked nucleotide. It is not necessary to modify all positions in a given compound uniformly. Rather, one or more modifications can be added to a single siRNA or even to a single nucleotide thereof. The modification of one nucleotide is independent of the modification of another nucleotide.

In some embodiments, all or substantially all of the nucleotides of the siRNA are modified nucleotides. In some embodiments, the siRNA has 4 or less (i.e., 0, 1, 2, 3, or 4) nucleotides in both the sense and antisense strands as unmodified nucleotides. In some embodiments, the sense strand of the siRNA comprises two or less (i.e., 0, 1, or 2) nucleotides as unmodified nucleotides. In some embodiments, the antisense strand of the siRNA comprises two or less (i.e., 0, 1, or 2) nucleotides as unmodified nucleotides. In some embodiments, one or more nucleotides of the siRNA are unmodified ribonucleotides.

In some embodiments, one or more nucleotides of the siRNA are linked by a non-standard linkage or backbone (i.e., a modified internucleoside linkage or a modified backbone). The modified internucleoside linkage or backbone includes, but is not limited to, a phosphorothioate group, chiral phosphorothioate, phosphorothioate, phosphorodithioate, phosphotriester, aminoalkyl-phosphotriester, chiral phosphonate, phosphinate, phosphoramidate, thioalkyl phosphonate, thioalkyl phosphotriester, and a morpholino linkage, wherein adjacent pairs of nucleoside units are linked in the form of 3'-5' to 5'-3' or 2'-5' to 5'-2'.

In some embodiments, the sense strand of the siRNA may comprise 1, 2, 3, 4, 5, or 6 phosphorothioate linkages (phosphorothioate-modified nucleotides), and the antisense strand of the siRNA may comprise 1, 2, 3, 4, 5, or 6 phosphorothioate linkages (phosphorothioate-modified nucleotides). In some embodiments, the sense strand of the siRNA may comprise 1 or 2 phosphorothioate linkages, and the antisense strand of the siRNA may comprise 1, 2, 3, or 4 phosphorothioate linkages.

In some embodiments, the sense strand of the siRNA comprises 2 phosphorothioate internucleoside linkages. In some embodiments, the phosphorothioate internucleoside linkages are located between nucleotides at positions 1-3 from the 5' end of the sense strand. In some embodiments, the phosphorothioate internucleoside linkages are located between nucleotides at positions 1-3 from the 3' end of the sense strand. In some embodiments, one phosphorothioate internucleoside linkage is located at the 5' end of the sense strand, and the other phosphorothioate linkage is located at the 3' end of the sense strand. In some embodiments, the sense strand of the siRNA comprises 1 phosphorothioate internucleoside linkage. In some embodiments, the phosphorothioate internucleoside linkages are located between nucleotides at positions 1-2 from the 5' end of the sense strand. In some embodiments, the phosphorothioate internucleoside linkages are located between nucleotides at positions 2-3 from the 5' end of the sense strand. In some embodiments, the targeting ligand is linked to the sense strand by a phosphorothioate linkage.

In some embodiments, the antisense strand of the siRNA comprises 4 phosphorothioate internucleoside linkages. In some embodiments, the 4 phosphorothioate internucleoside linkages are located between the nucleotides at positions 1-3 from the 5' end and between the nucleotides at positions 1-3 from the 5' end of the antisense strand. In some embodiments, the antisense strand of the siRNA comprises 3 phosphorothioate internucleoside linkages. In some embodiments, 3 phosphorothioate internucleoside linkages are located between the nucleotides at positions 1-2 from the 5' end and between the nucleotides at positions 1-3 from the 3' end of the antisense strand, respectively. In some embodiments, 3 phosphorothioate internucleoside linkages are located between the nucleotides at positions 1-3 from the 5' end and between the nucleotides at positions 1-2 from the 3' end of the antisense strand, respectively. In some embodiments, the antisense strand of the siRNA comprises 2 phosphorothioate internucleoside linkages. In some embodiments, the 2 phosphorothioate internucleoside linkages are located between the nucleotides at positions 1-2 from the 5' end and between the nucleotides at positions 1-2 from the 5' end of the antisense strand.

In some embodiments, the antisense strand of the siRNA comprises a nucleotide (from the 5' end → the 3' end) sequence of any one of the antisense strand sequences in Tables 1, 2, and 3. In some embodiments, the sense strand of the siRNA comprises a nucleotide (from the 5' end → the 3' end) sequence of any one of the sense strands in Tables 1, 2, and 3. In some embodiments, the antisense strand of the siRNA comprises a nucleotide (from the 5' end → the 3' end) sequence of any one of the antisense strands in Tables 1, 2, and 3, and the sense strand comprises a nucleotide (from the 5' end → the 3' end) sequence of any one of the sense strands in Tables 1, 2, and 3.

The nucleotides herein are abbreviated as follows:
A = adenosine-3'-phosphate
C = cytidine-3'-phosphate
G = guanosine-3'-phosphate
U = uridine-3'-phosphate
mA = 2'-O-methyladenosine-3'-phosphate
mA* = 2'-O-methyladenosine-3'-phosphorothioate
mC = 2'-O-methylcytidine-3'-phosphate
mC* = 2'-O-methylcytidine-3'-phosphorothioate
mG = 2'-O-methylguanosine-3'-phosphate
mG* = 2'-O-methylguanosine-3'-phosphorothioate
mU = 2'-O-methyluridine-3'-phosphate
mU* = 2'-O-methyluridine-3'-phosphorothioate
Nf = any 2'-deoxy-2'-fluoro-modified ribonucleotide
Af = 2'-fluoroadenosine-3'-phosphate
Af* = 2'-fluoroadenosine-3'-phosphorothioate
Cf = 2'-fluorocytidine-3'-phosphate
Cf* = 2'-fluorocytidine-3'-phosphorothioate
Gf = 2'-fluoroguanosine-3'-phosphate
Gf* = 2'-fluoroguanosine-3'-phosphorothioate
Tf = 2'-fluoro-5'-methyluridine-3'-phosphate
Tf* = 2'-fluoro-5'-methyluridine-3'-phosphorothioate
Uf = 2'-fluorouridine-3'-phosphate
Uf* = 2'-fluorouridine-3'-thiophosphate
dN = any 2'-deoxyribonucleotide
dA = 2'-deoxyadenosine-3'-phosphate
dA* = 2'-deoxyadenosine-3'-phosphorothioate
dC = 2'-deoxycytidine-3'-phosphate
dC* = 2'-deoxycytidine-3'-phosphorothioate
dG = 2'-deoxyguanosine-3'-phosphate
dG* = 2'-deoxyguanosine-3'-phosphorothioate
dT = 2'-deoxythymidine-3'-phosphate
dT* = 2'-deoxythymidine-3'-phosphorothioate
dU = 2'-deoxyuridine-3'-phosphate
dU* = 2'-deoxyuridine-3'-phosphorothioate
N = any ribonucleotide

The siRNA sequences defined as having modified bases, the specific modified forms of the bases, and the siRNA sequences obtained after modifications in the electronic sequence of listing correspond to the sequences indicated by the corresponding sequence numbers of Tables 2 and 3 below.

**Table 1: nucleotide sequences of sense and antisense strands of siRNAs**

| siRNA number | Sense strand | SEQ ID NO: | Antisense strand | SEQ ID NO: |
|---|---|---|---|---|
| R-1 | AACCAACAGCAUAGUCAAAUA | 1 | UAUUUGACUAUGCUGUUGGUUUA | 35 |
| R-2 | AAAAUCAGCUCAGAAGGACUA | 2 | UAGUCCUUCUGAGCUGAUUUUCU | 36 |
| R-3 | GGAAAUCACGAAACCAACUAU | 3 | AUAGUUGGUUUCGUGAUUUCCCA | 37 |
| R-4 | AAUGCAAUCCCGGAAAACAAA | 4 | UUUGUUUUCCGGGAUUGCAUUGG | 38 |
| R-5 | GCAAAAGGACACUUCAACUGU | 5 | ACAGUUGAAGUGUCCUUUUGCUU | 39 |
| R-6 | GUCUCAAAAUGGAAGGUUAUA | 6 | UAUAACCUUCCAUUUUGAGACUU | 40 |
| R-7 | AAAUACAGAGGACUGGUAAUU | 7 | AAUUACCAGUCCUCUGUAUUUAG | 41 |
| R-8 | AAAUGGAGAUGACUACUAAGU | 8 | ACUUAGUAGUCAUCUCCAUUUGU | 42 |
| R-9 | GAAAUAAACCUCGUAACAAGU | 9 | ACUUGUUACGAGGUUUAUUUCUA | 43 |
| R-10 | AACAGCCUGACAAGCAUGUAU | 10 | AUACAUGCUUGUCAGGCUGUUUA | 44 |
| R-11 | AAAGACCCAGUCCCUAAAUUA | 11 | UAAUUUAGGGACUGGGUCUUUGU | 45 |
| R-12 | AAGACCCAGUCCCUAAAUUAU | 12 | AUAAUUUAGGGACUGGGUCUUUG | 46 |
| R-13 | GCCCAAGUUAUAUGGUGACCU | 13 | AGGUCACCAUAUAACUUGGGCAC | 47 |
| R-14 | GCUAUGUUAGACGAUGUAA | 14 | UUACAUCGUCUAACAUAGCAA | 48 |
| R-15 | AGAACACCCAGAAGUAACU | 15 | AGUUACUUCUGGGUGUUCUGG | 49 |
| R-16 | AACCAACAGCAUAGUCAAA | 16 | UUUGACUAUGCUGUUGGUUUA | 50 |
| R-17 | AAUCAGCUCAGAAGGACUA | 17 | UAGUCCUUCUGAGCUGAUUUU | 51 |
| R-18 | AGCUCAGAAGGACUAGUAU | 18 | AUACUAGUCCUUCUGAGCUGA | 52 |
| R-19 | GCUCAGAAGGACUAGUAUU | 19 | AAUACUAGUCCUUCUGAGCUG | 53 |
| R-20 | AAUGAAACGUGGGAGAACU | 20 | AGUUCUCCCACGUUUCAUUGA | 54 |
| R-21 | AUGAAACGUGGGAGAACUA | 21 | UAGUUCUCCCACGUUUCAUUG | 55 |
| R-22 | GGGCCUAGAGAAGAUAUAC | 22 | GUAUAUCUUCUCUAGGCCCAA | 56 |
| R-23 | AAAUCACGAAACCAACUAU | 23 | AUAGUUGGUUUCGUGAUUUCC | 57 |
| R-24 | ACAUCUAGUUGCGAUUACU | 24 | AGUAAUCGCAACUAGAUGUAG | 58 |
| R-25 | AAAAGGACACUUCAACUGU | 25 | ACAGUUGAAGUGUCCUUUUGC | 59 |
| R-26 | AAUACAGAGGACUGGUAAU | 26 | AUUACCAGUCCUCUGUAUUUA | 60 |
| R-27 | AUGGAGAUGACUACUAAGU | 27 | ACUUAGUAGUCAUCUCCAUUU | 61 |
| R-28 | AGAAAUAAACCUCGUAACA | 28 | UGUUACGAGGUUUAUUUCUAC | 62 |
| R-29 | GCCUGACAAGCAUGUAUAU | 29 | AUAUACAUGCUUGUCAGGCUG | 63 |
| R-30 | AGACCCAGUCCCUAAAUUA | 30 | UAAUUUAGGGACUGGGUCUUU | 64 |
| R-31 | GACCCAGUCCCUAAAUUAU | 31 | AUAAUUUAGGGACUGGGUCUU | 65 |
| R-32 | GUCAAUACUUAGCAUUAUG | 32 | CAUAAUGCUAAGUAUUGACAA | 66 |
| R-33 | AUCACAACAGAUCCCUAAAU | 33 | AUUUAGGGAUCUGUUGUGAUGU | 67 |
| NC | AAUUCUCCGAACGUGUCACGU | 34 | ACGUGACACGUUCGGAGAAUU | 68 |

| | | | | |
|---|---|---|---|---|
| Note: NC indicates negative control. | | | | |

The sense and antisense strands of the siRNA comprising or consisting of the sequences in Table 1 may be modified nucleotides or unmodified nucleotides. In some embodiments, all or substantially all of the siRNAs having sense and antisense strand sequences comprising or consisting of the sequences in Table 1 are modified nucleotides.

In some embodiments, the antisense strand of the siRNA disclosed herein differs from any one of the antisense strand sequences in Table 1 by 0, 1, 2, or 3 nucleotides. In some embodiments, the sense strand of the siRNA disclosed herein differs from any one of the sense strand sequences in Table 1 by 0, 1, 2, or 3 nucleotides.

In some embodiments, the antisense strand of the siRNA comprises or consists of the nucleotide sequences of any one of the antisense strand sequences in Table 1. In some embodiments, the sense strand of the siRNA comprises or consists of the nucleotide sequence of any one of the sense strands in Table 1. In some embodiments, the sense strand of the siRNA comprises or consists of a modified sequence of any one of the sequences in Table 1. In some embodiments, the antisense strand of the siRNA comprises or consists of a modified sequence of any one of the sequences in Table 1. In some embodiments, the siRNA is selected from siRNAs indicated by any one of siRNA numbers in Table 1.

Certain modified nucleotide sequences of antisense and sense strands of siRNAs are provided in Tables 2 and 3. In the formation of siRNAs, each of the nucleotides listed in Tables 2 and 3 below and Table 1 may be a modified nucleotide.

Examples of sense and antisense strands containing modified nucleotides are provided in Tables 2 and 3.

**Table 2: modified nucleotide sequences of sense strands and antisense strands of siRNAs**

| siRNA number | Sense strand | SEQ ID NO: | Antisense strand | SEQ ID NO: |
|---|---|---|---|---|
| SR-1-01 | fGmUmCmUmCfAfAfAfAfUfGfGfAfAf GfGmUmUfAmUfA | 69 | UAUAACCUUCCAUUUUGAGACUU | 40 |
| SR-1-02 | mGmUmCmUmCmAmAfAfAfUfGfGfA fAfGfGmUmUfAmUfA | 70 | UAUAACCUUCCAUUUUGAGACUU | 40 |
| SR-1-03 | fGmUmCmUmCmAmAmAfAfUfGfGfA fAfGfGmUmUfAmUfA | 71 | UAUAACCUUCCAUUUUGAGACUU | 40 |
| SR-1-04 | fGmUmCmUmCfAmAmAmAfUfGfGfA fAfGfGmUmUfAmUfA | 72 | UAUAACCUUCCAUUUUGAGACUU | 40 |
| SR-1-05 | fGmUmCmUmCfAfAmAmAmUfGfGfA fAfGfGmUmUfAmUfA | 73 | UAUAACCUUCCAUUUUGAGACUU | 40 |
| SR-1-06 | fGmUmCmUmCfAfAfAmAmUmGfGfA fAfGfGmUmUfAmUfA | 74 | UAUAACCUUCCAUUUUGAGACUU | 40 |
| SR-1-07 | fGmUmCmUmCfAfAfAfAmUmGmGfA fAfGfGmUmUfAmUfA | 75 | UAUAACCUUCCAUUUUGAGACUU | 40 |
| SR-1-08 | fGmUmCmUmCfAfAfAfAfUmGmGmA fAfGfGmUmUfAmUfA | 76 | UAUAACCUUCCAUUUUGAGACUU | 40 |
| SR-1-09 | fGmUmCmUmCfAfAfAfAfUfGmGmA mAfGfGmUmUfAmUfA | 77 | UAUAACCUUCCAUUUUGAGACUU | 40 |
| SR-1-10 | fGmUmCmUmCfAfAfAfAfUfGfGmAm AmGfGmUmUfAmUfA | 78 | UAUAACCUUCCAUUUUGAGACUU | 40 |
| SR-1-11 | fGmUmCmUmCfAfAfAfAfUfGfGfAmA mGmGmUmUfAmUfA | 79 | UAUAACCUUCCAUUUUGAGACUU | 40 |
| SR-1-12 | fGmUmCmUmCfAfAfAfAfUfGfGfAfA mGmGmUmUmAmUfA | 80 | UAUAACCUUCCAUUUUGAGACUU | 40 |
| SR-1-13 | fGmUmCmUmCfAfAfAfAfUfGfGfAfAf GmGmUmUmAmUmA | 81 | UAUAACCUUCCAUUUUGAGACUU | 40 |
| SR-1-14 | GUCUCAAAAUdGGAAGGUUAUA | 82 | UAUAACCUUCCAUUUUGAGACUU | 40 |
| SR-1-15 | GUCUCAAAAUGGAAGGUUAUA | 6 | mUfAmUfAmAfCmCfUmUfCmCfAmUfUm UfUmGfAmGfAmCfUmU | 121 |
| SR-1-16 | GUCUCAAAAUGGAAGGUUAUA | 6 | mUfAmUfAmAfCmCfUfUfCfCfAmUfUmU fUmGfAmGfAmCfUmU | 122 |
| SR-1-17 | GUCUCAAAAUGGAAGGUUAUA | 6 | mUmAmUfAmAfCmCfUfUfCfCfAmUfUm UfUmGfAmGfAmCfUmU | 123 |
| SR-1-18 | GUCUCAAAAUGGAAGGUUAUA | 6 | mUfAmUmAmAfCmCfUfUfCfCfAmUfUm UfUmGfAmGfAmCfUmU | 124 |
| SR-1-19 | GUCUCAAAAUGGAAGGUUAUA | 6 | mUfAmUfAmAmCmCfUfUfCfCfAmUfUm UfUmGfAmGfAmCfUmU | 125 |
| SR-1-20 | GUCUCAAAAUGGAAGGUUAUA | 6 | mUfAmUfAmAfCmCmUfUfCfCfAmUfUm UfUmGfAmGfAmCfUmU | 126 |
| SR-1-21 | GUCUCAAAAUGGAAGGUUAUA | 6 | mUfAmUfAmAfCmCfUmUfCfCfAmUfUm UfUmGfAmGfAmCfUmU | 127 |
| SR-1-22 | GUCUCAAAAUGGAAGGUUAUA | 6 | mUfAmUfAmAfCmCfUfUmCfCfAmUfUm UfUmGfAmGfAmCfUmU | 128 |
| SR-1-23 | GUCUCAAAAUGGAAGGUUAUA | 6 | mUfAmUfAmAfCmCfUfUfCmCfAmUfUm UfUmGfAmGfAmCfUmU | 129 |
| SR-1-24 | GUCUCAAAAUGGAAGGUUAUA | 6 | mUfAmUfAmAfCmCfUfUfCfCmAmUfUm UfUmGfAmGfAmCfUmU | 130 |
| SR-1-25 | GUCUCAAAAUGGAAGGUUAUA | 6 | mUfAmUfAmAfCmCfUfUfCfCfAmUmUm UfUmGfAmGfAmCfUmU | 131 |
| SR-1-26 | GUCUCAAAAUGGAAGGUUAUA | 6 | mUfAmUfAmAfCmCfUfUfCfCfAmUfUmU mUmGfAmGfAmCfUmU | 132 |
| SR-1-27 | GUCUCAAAAUGGAAGGUUAUA | 6 | mUfAmUfAmAfCmCfUfUfCfCfAmUfUmU fUmGmAmGfAmCfUmU | 133 |
| SR-1-28 | GUCUCAAAAUGGAAGGUUAUA | 6 | mUfAmUfAmAfCmCfUfUfCfCfAmUfUmU fUmGfAmGmAmCfUmU | 134 |
| SR-1-29 | GUCUCAAAAUGGAAGGUUAUA | 6 | mUfAmUfAmAfCmCfUfUfCfCfAmUfUmU fUmGfAmGfAmCmUmU | 135 |
| SR-2-01 | AfAfAfmUmCAfmCGfAfAfAfmCmCAf AfmCmUAfmU | 83 | AUAGUUGGUUUCGUGAUUUCC | 57 |
| SR-2-02 | mAmAmAmUmCAfmCGfAfAfAfmCm CAfAfmCmUAfmU | 84 | AUAGUUGGUUUCGUGAUUUCC | 57 |
| SR-2-03 | AfmAmAmUmCmAmCGfAfAfAfmCm CAfAfmCmUAfmU | 85 | AUAGUUGGUUUCGUGAUUUCC | 57 |
| SR-2-04 | AfAfmAmUmCmAmCmGAfAfAfmCm CAfAfmCmUAfmU | 86 | AUAGUUGGUUUCGUGAUUUCC | 57 |
| SR-2-05 | AfAfAfmUmCmAmCmGmAAfAfmCm CAfAfmCmUAfmU | 87 | AUAGUUGGUUUCGUGAUUUCC | 57 |
| SR-2-06 | AfAfAfmUmCAfmCmGmAmAAfmCm CAfAfmCmUAfmU | 88 | AUAGUUGGUUUCGUGAUUUCC | 57 |
| SR-2-07 | AfAfAfmUmCAfmCGfmAmAmAmCm CAfAfmCmUAfmU | 89 | AUAGUUGGUUUCGUGAUUUCC | 57 |
| SR-2-08 | AfAfAfmUmCAfmCGfAfmAmAmCmC mAAfmCmUAfmU | 90 | AUAGUUGGUUUCGUGAUUUCC | 57 |
| SR-2-09 | AfAfAfmUmCAfmCGfAfAfmAmCmC mAmAmCmUAfmU | 91 | AUAGUUGGUUUCGUGAUUUCC | 57 |
| SR-2-10 | AfAfAfmUmCAfmCGfAfAfAfmCmCm AmAmCmUmAmU | 92 | AUAGUUGGUUUCGUGAUUUCC | 57 |
| SR-2-11 | AfAfAfmUmCAfmCGfmAmAAfmCmC AfAfmCmUAfmU | 93 | AUAGUUGGUUUCGUGAUUUCC | 57 |
| SR-2-12 | AfAfAfmUmCAfmCGfAfmAmAmCmC AfAfmCmUAfmU | 94 | AUAGUUGGUUUCGUGAUUUCC | 57 |
| SR-2-13 | AfAfAfmUmCAfmCGfmAAfmAmCmC AfAfmCmUAfmU | 95 | AUAGUUGGUUUCGUGAUUUCC | 57 |
| SR-2-14 | AAAUCACGAAdACCAACUAU | 96 | AUAGUUGGUUUCGUGAUUUCC | 57 |
| SR-2-15 | AAAUCACGAAACCAACUAU | 23 | mAUfmAGfmUUfmGGfmUUfmUCfmGUf mGAfmUUfmUCfmC | 136 |
| SR-2-16 | AAAUCACGAAACCAACUAU | 23 | mAUfmAGfmUUfmGGfUfUfUfCfmGUfmG AfmUUfmUCfmC | 137 |
| SR-2-17 | AAAUCACGAAACCAACUAU | 23 | mAmUmAGfmUUfmGGfUfUfUfCfmGUfm GAfmUUfmUCfmC | 138 |
| SR-2-18 | AAAUCACGAAACCAACUAU | 23 | mAUfmAmGmUUfmGGfUfUfUfCfmGUfm GAfmUUfmUCfmC | 139 |
| SR-2-19 | AAAUCACGAAACCAACUAU | 23 | mAUfmAGfmUmUmGGfUfUfUfCfmGUfm GAfmUUfmUCfmC | 140 |
| SR-2-20 | AAAUCACGAAACCAACUAU | 23 | mAUfmAGfmUUfmGmGUfUfUfCfmGUfm GAfmUUfmUCfmC | 141 |
| SR-2-21 | AAAUCACGAAACCAACUAU | 23 | mAUfmAGfmUUfmGGfmUUfUfCfmGUfm GAfmUUfmUCfmC | 142 |
| SR-2-22 | AAAUCACGAAACCAACUAU | 23 | mAUfmAGfmUUfmGGfUfmUUfCfmGUfm GAfmUUfmUCfmC | 143 |
| SR-2-23 | AAAUCACGAAACCAACUAU | 23 | mAUfmAGfmUUfmGGfUfUfmUCfmGUfm GAfmUUfmUCfmC | 144 |
| SR-2-24 | AAAUCACGAAACCAACUAU | 23 | mAUfmAGfmUUfmGGfUfUfUfmCmGUfm GAfmUUfmUCfmC | 145 |
| SR-2-25 | AAAUCACGAAACCAACUAU | 23 | mAUfmAGfmUUfmGGfUfUfUfCfmGmUm GAfmUUfmUCfmC | 146 |
| SR-2-26 | AAAUCACGAAACCAACUAU | 23 | mAUfmAGfmUUfmGGfUfUfUfCfmGUfmG mAmUUfmUCfmC | 147 |
| SR-2-27 | AAAUCACGAAACCAACUAU | 23 | mAUfmAGfmUUfmGGfUfUfUfCfmGUfmG AfmUmUmUCfmC | 148 |
| SR-2-28 | AAAUCACGAAACCAACUAU | 23 | mAUfmAGfmUUfmGGfUfUfUfCfmGUfmG AfmUUfmUmCmC | 149 |
| SR-3-01 | fAmCfAmUmCmUfAfGmUmUfGmCfG fAmUmUfAmCmU | 97 | AGUAAUCGCAACUAGAUGUAG | 58 |
| SR-3-02 | mAmCfAmUmCmUfAfGmUmUfGmCf GfAmUmUfAmCmU | 98 | AGUAAUCGCAACUAGAUGUAG | 58 |
| SR-3-03 | mAmCmAmUmCmUfAfGmUmUfGmCf GfAmUmUfAmCmU | 99 | AGUAAUCGCAACUAGAUGUAG | 58 |
| SR-3-04 | mAmCmAmUmCmUmAfGmUmUfGm CfGfAmUmUfAmCmU | 100 | AGUAAUCGCAACUAGAUGUAG | 58 |
| SR-3-05 | fAmCmAmUmCmUmAmGmUmUfGm CfGfAmUmUfAmCmU | 101 | AGUAAUCGCAACUAGAUGUAG | 58 |
| SR-3-06 | fAmCfAmUmCmUmAmGmUmUmGm CfGfAmUmUfAmCmU | 102 | AGUAAUCGCAACUAGAUGUAG | 58 |
| SR-3-07 | fAmCfAmUmCmUfAmGmUmUmGmC mGfAmUmUfAmCmU | 103 | AGUAAUCGCAACUAGAUGUAG | 58 |
| SR-3-08 | fAmCfAmUmCmUfAfGmUmUmGmCm GmAmUmUfAmCmU | 104 | AGUAAUCGCAACUAGAUGUAG | 58 |
| SR-3-09 | fAmCfAmUmCmUfAfGmUmUfGmCm GmAmUmUmAmCmU | 105 | AGUAAUCGCAACUAGAUGUAG | 58 |
| SR-3-10 | fAmCfAmUmCmUfAfGmUmUfGmCfG mAmUmUmAmCmU | 106 | AGUAAUCGCAACUAGAUGUAG | 58 |
| SR-3-11 | fAmCfAmUmCmUfAfGmUmUfGmCfG fAmUmUmAmCmU | 107 | AGUAAUCGCAACUAGAUGUAG | 58 |
| SR-3-12 | ACAUCUAGUUdGCGAUUACU | 108 | AGUAAUCGCAACUAGAUGUAG | 58 |
| SR-3-13 | ACAUCUAGUUGCGAUUACU | 24 | mAfGmUfAmAfUmCfGmCfAmAfCmUfA mGfAmUfGmUfAmG | 150 |
| SR-3-14 | ACAUCUAGUUGCGAUUACU | 24 | mAfGmUfAmAfUmCfGfCfAfAfCmUfAmG fAmUfGmUfAmG | 151 |
| SR-3-15 | ACAUCUAGUUGCGAUUACU | 24 | mAmGmUfAmAfUmCfGfCfAfAfCmUfAm GfAmUfGmUfAmG | 152 |
| SR-3-16 | ACAUCUAGUUGCGAUUACU | 24 | mAfGmUmAmAfUmCfGfCfAfAfCmUfAm GfAmUfGmUfAmG | 153 |
| SR-3-17 | ACAUCUAGUUGCGAUUACU | 24 | mAfGmUfAmAmUmCfGfCfAfAfCmUfAm GfAmUfGmUfAmG | 154 |
| SR-3-18 | ACAUCUAGUUGCGAUUACU | 24 | mAfGmUfAmAfUmCmGfCfAfAfCmUfAm GfAmUfGmUfAmG | 155 |
| SR-3-19 | ACAUCUAGUUGCGAUUACU | 24 | mAfGmUfAmAfUmCfGmCfAfAfCmUfAm GfAmUfGmUfAmG | 156 |
| SR-3-20 | ACAUCUAGUUGCGAUUACU | 24 | mAfGmUfAmAfUmCfGfCmAfAfCmUfAm GfAmUfGmUfAmG | 157 |
| SR-3-21 | ACAUCUAGUUGCGAUUACU | 24 | mAfGmUfAmAfUmCfGfCfAmAfCmUfAm GfAmUfGmUfAmG | 158 |
| SR-3-22 | ACAUCUAGUUGCGAUUACU | 24 | mAfGmUfAmAfUmCfGfCfAfAmCmUfAm GfAmUfGmUfAmG | 159 |
| SR-3-23 | ACAUCUAGUUGCGAUUACU | 24 | mAfGmUfAmAfUmCfGfCfAfAfCmUmAm GfAmUfGmUfAmG | 160 |
| SR-3-24 | ACAUCUAGUUGCGAUUACU | 24 | mAfGmUfAmAfUmCfGfCfAfAfCmUfAmG mAmUfGmUfAmG | 161 |
| SR-3-25 | ACAUCUAGUUGCGAUUACU | 24 | mAfGmUfAmAfUmCfGfCfAfAfCmUfAmG fAmUmGmUfAmG | 162 |
| SR-3-26 | ACAUCUAGUUGCGAUUACU | 24 | mAfGmUfAmAfUmCfGfCfAfAfCmUfAmG fAmUfGmUmAmG | 163 |
| SR-4-01 | fAfGfAfAmCfAmCmCmCfAfGfAfAfG mUfAfAmCmU | 109 | AGUUACUUCUGGGUGUUCUGG | 49 |
| SR-4-02 | mAmGmAfAmCfAmCmCmCfAfGfAfA fGmUfAfAmCmU | 110 | AGUUACUUCUGGGUGUUCUGG | 49 |
| SR-4-03 | fAmGmAmAmCfAmCmCmCfAfGfAfA fGmUfAfAmCmU | 111 | AGUUACUUCUGGGUGUUCUGG | 49 |
| SR-4-04 | fAfGmAmAmCmAmCmCmCfAfGfAfA fGmUfAfAmCmU | 112 | AGUUACUUCUGGGUGUUCUGG | 49 |
| SR-4-05 | fAfGfAmAmCmAmCmCmCmAfGfAfA fGmUfAfAmCmU | 113 | AGUUACUUCUGGGUGUUCUGG | 49 |
| SR-4-06 | fAfGfAfAmCmAmCmCmCmAmGfAfA fGmUfAfAmCmU | 114 | AGUUACUUCUGGGUGUUCUGG | 49 |
| SR-4-07 | fAfGfAfAmCfAmCmCmCmAmGmAfA fGmUfAfAmCmU | 115 | AGUUACUUCUGGGUGUUCUGG | 49 |
| SR-4-08 | fAfGfAfAmCfAmCmCmCfAmGmAmA fGmUfAfAmCmU | 116 | AGUUACUUCUGGGUGUUCUGG | 49 |
| SR-4-09 | fAfGfAfAmCfAmCmCmCfAfGmAmA mGmUfAfAmCmU | 117 | AGUUACUUCUGGGUGUUCUGG | 49 |
| SR-4-10 | fAfGfAfAmCfAmCmCmCfAfGfAmAm GmUmAfAmCmU | 118 | AGUUACUUCUGGGUGUUCUGG | 49 |
| SR-4-11 | fAfGfAfAmCfAmCmCmCfAfGfAfAmG mUmAmAmCmU | 119 | AGUUACUUCUGGGUGUUCUGG | 49 |
| SR-4-12 | AGAACACCCAdGAAGUAACU | 120 | AGUUACUUCUGGGUGUUCUGG | 49 |
| SR-4-13 | AGAACACCCAGAAGUAACU | 15 | mAfGmUfUmAfCmUfUmCfUmGfGmGfU mGfUmUfCmUfGmG | 164 |
| SR-4-14 | AGAACACCCAGAAGUAACU | 15 | mAfGmUfUmAfCmUfUfCfUfGfGmGfUmG fUmUfCmUfGmG | 165 |
| SR-4-15 | AGAACACCCAGAAGUAACU | 15 | mAmGmUfUmAfCmUfUfCfUfGfGmGfUm GfUmUfCmUfGmG | 166 |
| SR-4-16 | AGAACACCCAGAAGUAACU | 15 | mAfGmUmUmAfCmUfUfCfUfGfGmGfUm GfUmUfCmUfGmG | 167 |
| SR-4-17 | AGAACACCCAGAAGUAACU | 15 | mAfGmUfUmAmCmUfUfCfUfGfGmGfUm GfUmUfCmUfGmG | 168 |
| SR-4-18 | AGAACACCCAGAAGUAACU | 15 | mAfGmUfUmAfCmUmUfCfUfGfGmGfUm GfUmUfCmUfGmG | 169 |
| SR-4-19 | AGAACACCCAGAAGUAACU | 15 | mAfGmUfUmAfCmUfUmCfUfGfGmGfUm GfUmUfCmUfGmG | 170 |
| SR-4-20 | AGAACACCCAGAAGUAACU | 15 | mAfGmUfUmAfCmUfUfCmUfGfGmGfUm GfUmUfCmUfGmG | 171 |
| SR-4-21 | AGAACACCCAGAAGUAACU | 15 | mAfGmUfUmAfCmUfUfCfUmGfGmGfUm GfUmUfCmUfGmG | 172 |
| SR-4-22 | AGAACACCCAGAAGUAACU | 15 | mAfGmUfUmAfCmUfUfCfUfGmGmGfUm GfUmUfCmUfGmG | 173 |
| SR-4-23 | AGAACACCCAGAAGUAACU | 15 | mAfGmUfUmAfCmUfUfCfUfGfGmGmUm GfUmUfCmUfGmG | 174 |
| SR-4-24 | AGAACACCCAGAAGUAACU | 15 | mAfGmUfUmAfCmUfUfCfUfGfGmGfUmG mUmUfCmUfGmG | 175 |
| SR-4-25 | AGAACACCCAGAAGUAACU | 15 | mAfGmUfUmAfCmUfUfCfUfGfGmGfUmG fUmUmCmUfGmG | 176 |
| SR-4-26 | AGAACACCCAGAAGUAACU | 15 | mAfGmUfUmAfCmUfUfCfUfGfGmGfUmG fUmUfCmUmGmG | 177 |

**Table 3: further modified nucleotide sequences of siRNAs**

| siRNA number | Sense strand | SEQ ID No. | Antisense strand | SEQ ID No. |
|---|---|---|---|---|
| XR-1-01 | mGmUmCmUmCmAAfmAAfmUGfmGmA mAmGmGmUmUmAmUmA | 178 | mUmAmUmAmACfmCUfUfCfmCAfmU UfmUUfmGmAmGmAmCmUmU | 212 |
| XR-1-02 | mGmUmCmUmCmAAfmAAfmUdGmGmA mAmGmGmUmUmAmUmA | 179 | mUmAmUmAmACfmCUfUfCfmCAfmU UfmUUfmGmAmGmAmCmUmU | 213 |
| XR-1-03 | mGmUmCmUmCmAmAAfmAUfdGmGmA mAmGmGmUmUmAmUmA | 180 | mUmAmUmAmACfmCUfUfCfmCAfmU UfmUUfmGmAmGmAmCmUmU | 214 |
| XR-1-04 | mG*mU*mCmUmCmAAfmAAfmUdGmGm AmAmGmGmUmUmAmUmA | 181 | mUmAmUmAmACfmCUfUfCfmCAfmU UfmUUfmGmAmGmAmCmUmU | 215 |
| XR-1-05 | mG*mU*mCmUmCmAmAAfmAUfdGmGm AmAmGmGmUmUmAmUmA | 182 | mUmAmUmAmACfmCUfUfCfmCAfmU UfmUUfmGmAmGmAmCmUmU | 216 |
| XR-1-07 | mGmUmCmUmCmAAfmAAfmUdGmGmA mAmGmGmUmUmAmUmA | 183 | mU*mA*mUmAmACfmCUfUfCfmCAfm UUfmUUfmGmAmGmAmC*mU*mU | 217 |
| XR-1-08 | mGmUmCmUmCmAAfmAAfmUdGmGmA mAmGmGmUmUmAmUmA | 184 | mUmAmUmAmACfmCUfUfCfCfAfmUUf mUUfmGmAmGmAmCmUmU | 218 |
| XR-1-09 | mGmUmCmUmCmAAfmAAfmUdGmGmA mAmGmGmUmUmAmUmA | 185 | mU*mA*mUmAmACfmCUfUfCfmCAfm UUfmUUfmGmAmGmAmC*mU*mU | 219 |
| XR-2-01 | mAmAmAmUmCmAmCGfmAmAdAmCm CAfmAmCmUmAmU | 186 | mAUfmAmGmUmUmGGfmUUfmUCfmG UfmGmAmUmUmUCfmC | 220 |
| XR-2-02 | mAmAmAmUmCmAmCGfmAmAAfmCmC AfmAmCmUmAmU | 187 | mAUfmAmGmUmUmGGfmUUfmUCfmG UfmGmAmUmUmUCfmC | 221 |
| XR-2-03 | mAmAmAmUmCmAmCGfmAAfdAmCmC AfmAmCmUmAmU | 188 | mAUfmAmGmUmUmGGfmUUfmUCfmG UfmGmAmUmUmUCfmC | 222 |
| XR-2-04 | mAmAmAmUmCmACfmGAfmAdAmCmC AfmAmCmUmAmU | 189 | mAUfmAmGmUmUmGGfmUUfmUCfmG UfmGmAmUmUmUCfmC | 223 |
| XR-2-05 | mAmAmAmUmCmAmCGfmAmAdAmCm CmAAfmCmUmAmU | 190 | mAUfmAmGmUmUmGGfmUUfmUCfmG UfmGmAmUmUmUCfmC | 224 |
| XR-2-06 | mAmAmAmUmCmAmCGfmAmAdAmCm CmAmACfmUmAmU | 191 | mAUfmAmGmUmUmGGfmUUfmUCfmG UfmGmAmUmUmUCfmC | 225 |
| XR-2-07 | mA*mA*mAmUmCmAmCGfmAmAdAmC mCAfmAmCmUmAmU | 192 | mAUfmAmGmUmUmGGfmUUfmUCfmG UfmGmAmUmUmUCfmC | 226 |
| XR-2-09 | mAmAmAmUmCmAmCGfmAmAdAmCm CAfmAmCmUmAmU | 193 | mAUfmAmGmUmUmGmGUfmUUfCfmG UfmGmAmUmUmUCfmC | 227 |
| XR-2-10 | mAmAmAmUmCmAmCGfmAmAdAmCm CAfmAmCmUmAmU | 194 | mAUfmAmGmUmUmGmGmUmUmCf mGUfmGmAmUmUmUCfmC | 228 |
| XR-2-11 | mAmAmAmUmCmAmCGfmAmAdAmCm CAfmAmCmUmAmU | 195 | mAUfmAmGmUmUmGGfmUUfmUCfmG UfmGmAmUmUmUCfmC | 229 |
| XR-2-12 | mAmAmAmUmCmAmCGfmAmAdAmCm CAfmAmCmUmAmU | 196 | mA*Uf*mAmGmUmUmGGfmUUfmUCf mGUfmGmAmUmUmU*Cf*mC | 230 |
| XR-2-13 | mAmAmAmUmCmAmCGfmAmAdAmCm CAfmAmCmUmAmU | 197 | mA*Uf*mAmGmUmUmGGfmUUfmUCf mGUfmGmAmUmUmUCf*mC | 231 |
| XR-2-14 | mAmAmAmUmCmAmCGfmAmAdAmCm CAfmAmCmUmAmU | 198 | mA*UfmAmGmUmUmGGfmUUfmUCfm GUfmGmAmUmUmU*Cf*mC | 232 |
| XR-2-15 | mAmAmAmUmCmAmCGfmAmAdAmCm CAfmAmCmUmAmU | 199 | mA*UfmAmGmUmUmGGfmUUfmUCfm GUfmGmAmUmUmUCf*mC | 233 |
| XR-3-01 | mAmAmAmUmCmACfmGAfmAdAmCmC AfmAmCmUmAmU | 200 | mA*Uf*mAmGmUmUmGGfmUUfmUCf mGUfmGmAmUmUmU*Cf*mC | 234 |
| XR-3-02 | mA*mAmAmUmCmACfmGAfmAdAmCm CAfmAmCmUmAmU | 201 | mA*Uf*mAmGmUmUmGGfmUUfmUCf mGUfmGmAmUmUmU*Cf*mC | 235 |
| XR-3-03 | mAmA*mAmUmCmACfmGAfmAdAmCm CAfmAmCmUmAmU | 202 | mA*Uf*mAmGmUmUmGGfmUUfmUCf mGUfmGmAmUmUmU*Cf*mC | 236 |
| XR-3-04 | mA*mA*mAmUmCmACfmGAfmAdAmCm CAfmAmCmUmAmU | 203 | mA*Uf*mAmGmUmUmGGfmUUfmUCf mGUfmGmAmUmUmU*Cf*mC | 237 |
| XR-3-05 | mAmAmAmUmCmACfmGAfmAdAmCmC AfmAmCmUmAmU | 204 | mA*Uf*mAmGmUmUmGGfmUUfmUmC mGUfmGmAmUmUmU*Cf*mC | 238 |
| XR-4-01 | mGmUmCmUmCmAAfmAAfmUdGmGmA mAmGmGmUmUmAmUmA | 205 | mU*mA*mUmAmACfmCUfUfCfmCAfm UUfmUUfmGmAmGmAmC*mU*mU | 239 |
| XR-4-02 | mG*mUmCmUmCmAAfmAAfmUdGmGm AmAmGmGmUmUmAmUmA | 206 | mU*mA*mUmAmACfmCUfUfCfmCAfm UUfmUUfmGmAmGmAmC*mU*mU | 240 |
| XR-4-03 | mGmU*mCmUmCmAAfmAAfmUdGmGm AmAmGmGmUmUmAmUmA | 207 | mU*mA*mUmAmACfmCUfUfCfmCAfm UUfmUUfmGmAmGmAmC*mU*mU | 241 |
| XR-4-04 | mG*mU*mCmUmCmAAfmAAfmUdGmGm AmAmGmGmUmUmAmUmA | 208 | mU*mA*mUmAmACfmCUfUfCfmCAfm UUfmUUfmGmAmGmAmC*mU*mU | 242 |
| XR-4-05 | mGmUmCmUmCmAAfmAAfmUdGfGmA mAmGmGmUmUmAmUmA | 209 | mU*mA*mUmAmACfmCUfUfCfmCAfm UUfmUUfmGmAmGmAmC*mU*mU | 243 |
| XR-4-06 | mGmUmCmUmCmAAfmAAfmUdGmGfA mAmGmGmUmUmAmUmA | 210 | mU*mA*mUmAmACfmCUfUfCfmCAfm UUfmUUfmGmAmGmAmC*mU*mU | 244 |
| XR-4-07 | mGmUmCmUmCmAAfmAAfmUdGmGmA mAmGmGmUmUmAmUmA | 211 | mU*mA*mUmAmACfmCUfUfCfmCAfm UUfmUUfmGmAmGmAmC*mU*mU | 245 |

In some embodiments, the antisense strand of the siRNA disclosed herein differs from any one of the antisense strand sequences in Table 2 by 0, 1, 2, or 3 nucleotides. In some embodiments, the sense strand of the siRNA disclosed herein differs from any one of the sense strand sequences in Table 2 by 0, 1, 2, or 3 nucleotides.

In some embodiments, the antisense strand of the siRNA comprises any one of the antisense strand nucleotide sequences in Table 2. In some embodiments, the sense strand of the siRNA comprises any one of the sense strand nucleotide sequences in Table 2. In some embodiments, the sense strand of the siRNA comprises or consists of a modified sequence of any one of the sequences in Table 2. In some embodiments, the antisense strand of the siRNA comprises or consists of a modified sequence of any one of the sequences in Table 2. In some embodiments, the siRNA is selected from siRNAs indicated by any one of siRNA numbers in Table 2.

In some embodiments, the antisense strand of the siRNA disclosed herein differs from any one of the antisense strand sequences in Table 3 by 0, 1, 2, or 3 nucleotides. In some embodiments, the sense strand of the siRNA disclosed herein differs from any one of the sense strand sequences in Table 3 by 0, 1, 2, or 3 nucleotides.

In some embodiments, the antisense strand of the siRNA comprises any one of the antisense strand nucleotide sequences in Table 3. In some embodiments, the antisense strand of the siRNA comprises a sequence of nucleotides at positions 1-17, positions 2-15, positions 2-17, positions 1-18, positions 2-18, positions 1-19, positions 2-19, positions 1-20, positions 2-20, positions 1-21, positions 2-21, positions 1-22, positions 2-22, positions 1-23, positions 2-23, positions 1-24, or positions 2-24 (from the 5' end → the 3' end) of any one of the sequences in Table 3. In certain embodiments, the antisense strand of the siRNA comprises or consists of a modified sequence of any one of the antisense strand sequences in Table 5.

In some embodiments, the sense strand of the siRNA comprises any one of the sense strand nucleotide sequences in Table 3. In some embodiments, the sense strand of the siRNA comprises a sequence of nucleotides at positions 1-17, positions 2-15, positions 2-17, positions 1-18, positions 2-18, positions 1-19, positions 2-19, positions 1-20, positions 2-20, positions 1-21, positions 2-21, positions 1-22, positions 2-22, positions 1-23, positions 2-23, positions 1-24, or positions 2-24 (from the 5' end → the 3' end) of any one of the sequences in Table 3. In certain embodiments, the sense strand of the siRNA comprises or consists of a modified sequence of any one of the sense strand sequences in Table 3.

In some embodiments, the antisense strand of the siRNA comprises any one of the antisense strand nucleotide sequences in Table 3, and the sense strand of the siRNA comprises any one of the sense strand nucleotide sequences in Table 3. In some embodiments, the siRNA is selected from siRNAs indicated by any one of siRNA numbers in Table 3.

In some embodiments, the siRNA comprises, consists of, or substantially consists of a duplex indicated by any one of the siRNA numbers presented herein. In some embodiments, the siRNA comprises nucleotide sequences of the sense and antisense strands of any duplex indicated by any one of the siRNA numbers presented herein. In some embodiments, the siRNA comprises nucleotide sequences of the sense and antisense strands of any duplex indicated by any one of the siRNA numbers presented herein, and a targeting group and/or linking group, wherein the targeting group and/or linking group is covalently linked (i.e., conjugated) to the sense or antisense strand. In some embodiments, the siRNA comprises modified nucleotide sequences of the sense and antisense strands indicated by any one of the siRNA numbers presented herein. In some embodiments, the siRNA comprises modified nucleotide sequences of the sense and antisense strands of any one of the siRNA numbers presented herein, and a targeting group and/or linking group, wherein the targeting group and/or linking group is covalently linked to the sense or antisense strand.

In some embodiments, the siRNA comprises an antisense strand and a sense strand having the nucleotide sequences of any one of the antisense/sense strand duplexes of Table 1, Table 2, or Table 3, and further comprises a targeting group. In some embodiments, the siRNA comprises an antisense strand and a sense strand having the nucleotide sequences of any one of the antisense/sense strand duplexes of Table 1, Table 2, or Table 3, and further comprises a targeting group for targeted identification.

### siRNA Conjugate

The present invention also provides an siRNA conjugate, which comprises the siRNA described above and a targeting group for targeting identification. In some embodiments, the siRNA conjugate comprises a targeting group for targeting identification and a linker for conjugating the two directly or indirectly. The siRNA conjugate identifies a receptor on the cell surface through a targeting group, thereby delivering an siRNA to a target.

In some embodiments, the siRNA is linked to the targeting group without a linker. In some embodiments, the targeting group itself is designed with a linker or other sites to facilitate readily present conjugation.

In some embodiments, the siRNA conjugate comprises a pharmaceutically acceptable targeting group and optionally a linker, and the selected siRNA, linker and targeting group are linked sequentially. The siRNA molecule may be non-covalently or covalently bound to the linker, e.g., may be covalently linked to the linker.

In some embodiments, the linker is linked to an end of the sense or antisense strand of the siRNA, wherein the end refers to the first 4 nucleotides from one end of the sense or antisense strand. In some embodiments, the linker is linked to the 3' end or the 5' end of the sense strand of the siRNA. In some embodiments, the linker is linked to the 3' end or the 5' end of the antisense strand of the siRNA. In some embodiments, the linker is linked to the 3' end of the sense strand of the siRNA.

In some embodiments, the pharmaceutically acceptable targeting group described herein may comprise a ligand conventionally used in the art of siRNA administration, e.g., various ligands described in WO2009082607, which is incorporated herein by reference in its entirety.

In some embodiments, examples of the linker may include (but are not limited to): reactive groups (such as primary amine and alkyne), alkyl groups, abasic nucleotides, ribitol (abasic ribose), and/or PEG groups. In some embodiments, the linker described herein may comprise a linker known in the art of siRNA administration.

The linker of the present invention is a linkage between two atoms that links one target chemical group (such as an siRNA) or fragment to another target chemical group (such as a targeting group) or fragment via one or more covalent bonds. The labile linkage contains a labile bond. The linkage may optionally include a spacer group that increases the distance between the two linked atoms. The spacer group may further increase the flexibility and/or length of the linkage. The spacer group includes, but is not limited to, alkyl (e.g., C1-12 linear or branched alkyl), alkenyl (e.g., C2-C8 linear or branched alkenyl), alkynyl (e.g., C2-C8 linear or branched alkynyl), aryl (e.g., C6-14 aryl), aralkyl (e.g., C6-14 aryl C1-12 alkyl), aralkenyl (e.g., C6-14 aryl C2-C8 alkenyl), and aralkynyl (e.g., C6-14 aryl C2-C8 alkynyl); each of them may contain one or more heteroatoms (e.g., N, O, and S), heterocycles (e.g., heterocyclyl or heteroaryl containing one or more heteroatoms selected from O, S, and N), amino acids, nucleotides, and sugars. The linker contains groups at both ends suitable for a linkage (e.g., covalent linkage) to a targeting group as well as to the siRNA. Thus, in some embodiments, the linker of the present invention has the following structure: linking group 1-spacer group-linking group 2, wherein the linking group 1 may be a group for a linkage to a targeting group, such as a carbonyl group for forming an amide group with a targeting group; the linking group 2 may be a group for forming a covalent linkage with the siRNA, such as -O-, thereby forming a linkage of -O-P(O)OH- with, for example, a phosphate group at the 3' end of the sense strand of the siRNA, while the spacer group serves to increase the flexibility and/or length of the linkage.

In some embodiments, the targeting group of the present invention binds to a cell surface receptor. For this purpose, any cell surface receptor or biomarker or part thereof is considered suitable. In some embodiments, the targeting group of the present invention specifically binds to a specific receptor of a specific tissue, thereby achieving tissue-specific targeting. In some embodiments, the targeting group of the present invention specifically targets a receptor on the surface of hepatocytes and thus specifically targets a liver tissue. In some embodiments, the targeting group of the present invention specifically targets a cell surface receptor specific to hepatocytes. In some embodiments, the targeting group of the present invention comprises (GalNAc) and specifically targets an asialoglycoprotein receptor (ASGPR) on the surface of hepatocytes.

In some embodiments, the siRNA conjugate of the present invention has excellent liver targeting specificity, and thus can efficiently deliver the conjugated siRNA to the liver, thereby effectively regulating the expression of a specific gene in hepatocytes. Therefore, the siRNA conjugate has a wide application prospect.

### Pharmaceutical Composition and Pharmaceutical Formulation

The present invention also provides a pharmaceutical composition, which comprises the siRNA or siRNA conjugate described herein, and a pharmaceutically acceptable carrier thereof. In some embodiments, one or more pharmaceutically acceptable excipients (including vehicles, carriers, diluents, and/or delivery polymers) are added to a pharmaceutical composition comprising siRNA, thereby forming a pharmaceutical formulation or pharmaceutical composition suitable for *in vivo* delivery to a subject (including a human).

The pharmaceutical composition used herein comprises a pharmacologically effective amount of at least one of the therapeutic compounds and one or more pharmaceutically acceptable excipients. The pharmaceutically acceptable excipient is a substance that is intentionally included in a drug delivery system in addition to the active pharmaceutical ingredient (API, therapeutic product, e.g. ANGPTL3 siRNA). The excipient is not, or is not intended to, exert a therapeutic effect at the intended dosage. The excipient may have the following effects: a) facilitating the processing of the drug delivery system during preparation, b) protecting, supporting, or enhancing stability, bioavailability or patient acceptability of the API; c) facilitating the product identification; and/or d) enhancing overall safety, efficacy, or any other properties in delivery of the API during storage or use.

The excipient includes (but is not limited to): an absorption enhancer, an anti-adherent, an anti-foaming agent, an antioxidant, a binder, a buffer, a carrier, a coating, a colorant, a delivery enhancer, a delivery polymer, a detergent, dextran, dextrose, a diluent, a disintegrant, an emulsifier, a swelling agent, a filler, a flavoring agent, a glidant, a wetting agent, a lubricant, an oil, a polymer, a preservative, saline, a salt, a solvent, a sugar, a surfactant, a suspending agent, a sustained release matrix, a sweetener, a thickener, a tonicity agent, a vehicle, a water repellant, and a wetting agent.

In yet another aspect, the present invention provides a pharmaceutical combination for inhibiting the expression of an ANGPTL3 gene, which comprises
the siRNA, the siRNA conjugate, or the pharmaceutical composition thereof described herein, and one or more additional therapeutic agents for inhibiting the expression of an ANGPTL3 gene. In some embodiments, the additional therapeutic agent may be a small molecule drug, an antibody, an antibody fragment, or an siRNA.

### Method and Use

The present invention provides use of the siRNA, the siRNA conjugate, the pharmaceutical composition, or the pharmaceutical combination described herein in the preparation of a medicament for treating and/or preventing an ANGPTL3-mediated disease or disorder.

The present invention provides a method for treating and/or preventing an ANGPTL3-mediated disease or disorder, which comprises administering to a subject in need thereof the siRNA, the siRNA conjugate, the pharmaceutical composition, or the pharmaceutical combination described herein.

The present invention provides the siRNA, the siRNA conjugate, the pharmaceutical composition, or the pharmaceutical combination described herein, for use in treating and/or preventing an ANGPTL3-mediated disease or disorder.

The present invention provides a method for inhibiting the expression of an ANGPTL3 gene in a subject, which comprises administering to the subject in need thereof the siRNA, the siRNA conjugate, the pharmaceutical composition, or the pharmaceutical combination described herein.

The present invention provides a method for inhibiting the expression of a target gene in a cell, which comprises contacting the cell with the conjugate or the pharmaceutical composition thereof disclosed herein for a sufficient period of time to achieve degradation of an mRNA transcript of the target gene, thereby inhibiting the expression of the target gene in the cell. In some embodiments, the expression of the target gene is inhibited by at least 30%, at least about 40%, at least about 50%, at least about 60%, or at least 70%.

In some embodiments, the disease or disorder described herein includes atherosclerosis, hypercholesterolemia, hypertriglyceridemia, acute coronary syndrome, dyslipidemia, myocardial infarction, coronary artery lesion, stroke, coronary artery disease, cardiovascular disease, diabetes, hyperlipidemia, type 2 diabetes, and kidney disease.

In some embodiments, the subject has hypercholesterolemia, dyslipidemia, or hyperlipidemia. In some embodiments, the level of serum cholesterol in the subject is reduced after administration of the compound or composition described herein.

The present invention also provides a method for treating and preventing high cholesterol. In some embodiments, these methods further comprise the step of determining the level of serum cholesterol in the subject. The level of serum cholesterol may be determined before, during, and/or after the administration.

In some embodiments of the methods of treatment and prevention provided herein, the subject is a mammal, e.g., a primate, rodent, or human.

In some embodiments of the methods of treatment and prevention provided herein, administration of the siRNA conjugate or the pharmaceutical composition of the present invention results in a reduction in the level of serum cholesterol in the subject.

In some embodiments of the methods of treatment and prevention provided herein, the siRNA, the siRNA conjugate, the pharmaceutical composition, or the pharmaceutical combination of the present invention is administered parenterally (e.g., subcutaneously, intramuscularly, or intravenously) in the form of an injection or infusion solution. In one embodiment, the siRNA, the siRNA conjugate, the pharmaceutical composition, or the pharmaceutical combination of the present invention is administered subcutaneously.

It is clear to those skilled in the art that modified nucleotide groups can be introduced into the siRNA described herein by using nucleoside monomers having corresponding modifications, and methods for preparing the nucleoside monomers having corresponding modifications and methods for introducing the modified nucleotide groups into the siRNA are also well known to those skilled in the art. All the modified nucleoside monomers are commercially available or prepared by known methods.

In some embodiments of the present invention, a phosphonyl group is used to link the siRNA to the linker. It can be understood that the manner in which the linker is linked to or conjugated with the siRNA is varied and includes, but is not limited to, direct and indirect conjugation, and the indirect conjugation may be performed through a variety of carrier groups and linkages suitable for the conjugation.

The present invention includes all combinations of the specific embodiments. Further embodiments of the present invention and the full scope of applicability will become apparent from the detailed description provided below. However, it should be understood that the detailed description and the specific examples, while indicating preferred embodiments of the present invention, are provided by way of illustration only, as various changes and modifications within the spirit and scope of the present invention will become apparent to those skilled in the art from the detailed description. All publications, patents and patent applications cited herein, including the citations, are hereby incorporated by reference in their entirety for all purposes.

The compounds of the present invention can be prepared using a variety of synthesis methods well known to those skilled in the art, including the specific embodiments listed below, embodiments formed by combinations thereof with other methods, and equivalents well known to those skilled in the art; preferred embodiments include, but are not limited to, the examples of the present invention.

### Examples

The present invention is illustrated by the following examples, which, however, are not intended to be limiting in any way. The present invention has been described in detail, and the specific embodiments are also disclosed. It will be apparent to those skilled in the art that various changes and modifications can be made to the specific embodiments of the present invention without departing from the spirit and scope of the present invention. The methods and materials used in the examples are, unless otherwise indicated, conventional in the art.

### Example 1. Synthesis of siRNAs

### 1.1 Target sequence screening

siRNAs were designed based on complete mRNA sequences of ANGPTL3, all of which were derived from the NCBI Gene database (https://www.ncbi.nlm.nih.gov/gene/). All siRNAs were designed to ensure complete sequence identity to the sequences of human (Gene ID: 27329) and cynomolgus monkey (Gene ID: 102136264).

The complete sequences were scanned to obtain all potential siRNA sequences of 19-21 nucleotides in length, which were aligned to the sequences of the cynomolgus monkey to ensure a match to the sequences of the cynomolgus monkey. All human/cynomolgus monkey binding sequences were aligned by BLAST for the human whole transcriptome mRNA sequences, and all siRNAs with possible off-target effects were removed. The activity of all siRNAs was evaluated according to the rational siRNA design principle, and molecules with lower theoretical activity were removed. Thirty-three pairs of siRNAs were preliminarily designed and synthesized.

### 1.2 Synthesis of siRNAs

siRNAs were designed in different regions for ANGPTL3, and obtained by synthesizing and annealing by Genscript Biotech Corporation after adding 2 additional nucleotides complementary to mRNA in the antisense strand. The sequences of the siRNAs are shown in Table 1.

### Example 2. In Vitro Activity Assay Method for siRNAs

### 2.1 Fluorogenic quantitative PCR

### 2.1.1 Cell culture and transfection

HepG2 cells or Huh-7 cells (ATCC) were cultured in a DMEM medium (Gibco) with 10% fetal bovine serum (FBS, Gibco) and diabody (Gibco) at 37 °C with 5% CO₂, and were digested with trypsin and resuspended after the cells had grown to nearly completely cover the flask. After resuspension, the cells were adjusted for density and placed into a 96-well plate at 2×10⁴ cells/well, and an siRNA transfection complex was added in suspension. The siRNA transfection complex was obtained by mixing 0.3 µL/well Lipofectamine RNAi Max (Thermo) in MaxOpti-MEM (Gibco) with siRNA at a ratio of 1:1. After the cells were cultured for a certain period of time, the whole mRNA was obtained by extraction using the Dynabeads^{™} mRNA isolation kit (Thermo) according to the instructions. In the final step of mRNA elution, the TIANGEN first-strand synthesis kit was used. The system contained 5.2 µL of 5× buffer and 1.3 µL of dNTPs (deoxyribonucleoside triphosphates), and ddH₂O was added to make a final volume of 19.5 µL. The mixture was incubated at 80 °C for 5 min, and then quickly placed on a magnetic rack for magnetic bead separation to obtain 15 µL of supernatant. The heating was performed by a Thermo Veriti 96 well Thermal Cycler PCR instrument.

The primary cynomolgus monkey hepatocytes were obtained from Miaotong (Shanghai) Biotechnology Co., Ltd., thawed at 4 °C using a matched thaw medium, washed, resuspended in a maintenance medium, and placed into a 96-well plate coated with a matched coating medium at an adjusted density of 3×10⁴ cells/well. The above siRNA transfection complex was added after the cells were completely adhered to the wall. After the cells were cultured for a certain period of time, the whole mRNA was obtained by extraction using the Dynabeads^{™} mRNA isolation kit (Thermo) according to the instructions. In the final step of mRNA elution, the TIANGEN first-strand synthesis kit was used. The system contained 5.2 µL of 5× buffer and 1.3 µL of dNTPs, and ddH₂O was added to make a final volume of 19.5 µL. The mixture was incubated at 80 °C for 5 min, and then quickly placed on a magnetic rack for magnetic bead separation to obtain 15 µL of supernatant. The heating was performed by a Thermo Veriti 96 well Thermal Cycler PCR instrument.

### 2.1.2 cDNA reverse transcription

cDNA synthesis was performed using the TIANGEN first-strand synthesis kit. 2 µL of oligo dT (poly-deoxythymidine), 1 µL of reverse transcriptase, and 0.5 µL of RNase inhibitor were added to 15 µL of the supernatant transferred in Example 2.1.1 above, and H₂O was added to make a final volume of 20 µL. cDNA synthesis was performed using a Thermo Veriti 96 well Thermal Cycler PCR instrument at 42 °C for 60 min and 85 °C for 5 min, and then stored at 4 °C for a continuous period of time.

### 2.1.3 Fluorogenic quantitative PCR

ANGPTL3 and GAPDH (glyceraldehyde-3-phosphate dehydrogenase) were detected by using the SYBR green method (TIANGEN) or TaqMan probe method (Thermo), and the relative mRNA level of ANGPTL3 was measured by the ΔΔCt method. The instrument used was Applied Biosystem Stepone Plus or QuantStudio5 (Applied Biosystem). The reaction conditions were as follows: (1) pre-denaturation at 95 °C for 10 min; (2) denaturation at 95 °C for 15 s, and annealing and extension at 60 °C for 30 s. The step (2) lasted 40 cycles. The results obtained were normalized using a negative control to obtain the relative mRNA level and knockdown efficiency. If IC₅₀ is required to be calculated, it is obtained by four-parameter fitting using Graphpad Prism.

### 2.2 Reporter gene method

HEK293-psiCheck-ANGPTL3 cells contain the full-length sequence of ANGPTL3 and the tandem expression system of a luciferase gene. The cells were cultured in a DMEM medium (Gibco) with 10% FBS (Gibco), 2 µg/mL puromycin, and diabody (Gibco) at 37 °C with 5% CO₂, and were digested with trypsin and resuspended after the cells had grown to nearly completely cover the flask. After resuspension, the cells were adjusted for density and placed into a 96-well plate at 2×10⁴ cells/well, and an siRNA transfection complex was added after the cells were completely adhered to the wall. The siRNA transfection complex was obtained by mixing MaxOpti-MEM (Gibco) containing 0.3 µL/well Lipofectamine RNAi Max (Thermo) with siRNA at a ratio of 1:1. After cultured for a certain period of time, the cells were lysed and a Renilla substrate (Promega) was added for fluorescence activity assay.

### 2.3 Stem-loop PCR

The stem-loop method has been widely used to detect the absolute concentrations of siRNA and miRNA (Curr Protoc Mol Biol. 2011 Jul; Chapter 15: Unit 15.10.). Corresponding stem-loop primers were designed for different siRNAs, and the primers were used for replacing oligo dT for reverse transcription. After that, the siRNA concentration was determined by conversion from a standard curve by fluorogenic quantitative PCR. The reverse transcription conditions were as follows: (1) 16 °C for 30 s; (2) 30 °C for 30 s, 42 °C for 30 s, and 50 °C for 1 s, lasting 60 cycles; (3) 95 °C for 5 min. The fluorogenic quantitative PCR reaction conditions were as follows: (1) 95 °C for 5 min; (2) 95 °C for 5 s, 60 °C for 10 s, and 72 °C for 1 s. The step (2) lasted 40 cycles.

### Example 3 siRNA sequence screening

### 3.1 Screening for unmodified siRNA sequences

To evaluate the *in vitro* activity of siRNA against ANGPTL3, the siRNAs designed in Example 1 were functionally evaluated *in vitro* by the fluorogenic quantitative PCR and reporter gene method in Example 2.

The siRNAs were separately assayed for activity in HepG2 and Huh-7 cell lines by fluorogenic quantitative PCR and in a HEK293-psiCheck-ANGPTL3 (RGA) cell line by the reporter gene method. The results are shown in Table 4. Table 4 shows the expression level of ANGPTL3 mRNA of siRNAs relative to NC (negative control group).

**Table 4: In vitro activity assay of siRNAs**

| Number | HepG2 cell | | | | Huh-7 cell | | | | RGA | |
|---|---|---|---|---|---|---|---|---|---|---|
| | 10nM | | 0.1nM | | 10nM | | 0.1nM | | 100nM | 10nM |
| | Mean value | Standard deviation | Mean value | Standard deviation | Mean value | Standard deviation | Mean value | Standard deviation | | |
| R-1 | 0.45 | 0.13 | 1.03 | 0.09 | 0.20 | 0.01 | 1.00 | 0.01 | 0.11 | 0.29 |
| R-2 | 0.14 | 0.06 | 1.10 | 0.33 | 0.10 | 0.00 | 1.04 | 0.04 | 0.25 | 0.47 |
| R-3 | 0.02 | 0.02 | 0.78 | 0.02 | 0.04 | 0.00 | 0.91 | 0.05 | 0.04 | 0.19 |
| R-4 | 0.57 | 0.76 | 1.38 | 0.01 | 0.10 | 0.00 | 1.70 | 0.12 | 0.29 | 0.27 |
| R-5 | 0.00 | N.A. | 1.25 | 0.08 | 0.10 | 0.01 | 1.23 | 0.01 | 0.08 | 0.30 |
| R-6 | 0.16 | 0.03 | 0.92 | 0.37 | 0.07 | 0.00 | 0.83 | 0.00 | 0.10 | 0.23 |
| R-7 | 0.01 | N.A. | 1.22 | 0.41 | 0.29 | 0.01 | 0.82 | 0.00 | 0.10 | 0.43 |
| R-8 | 0.38 | 0.29 | 0.85 | 0.08 | 0.23 | 0.00 | 0.70 | 0.02 | 0.19 | 0.38 |
| R-9 | 0.37 | 0.08 | 1.01 | 0.15 | 0.40 | 0.01 | 1.10 | 0.05 | 0.25 | 0.27 |
| R-10 | 0.96 | 0.36 | 1.32 | 0.28 | 0.56 | 0.03 | 1.68 | 0.09 | 0.19 | 0.33 |
| R-11 | 3.81 | 0.65 | 2.71 | 1.68 | 2.65 | 0.06 | 1.90 | 0.06 | 0.13 | 0.27 |
| R-12 | 0.50 | 0.11 | 5.18 | 0.28 | 1.48 | 0.10 | 2.75 | 0.08 | 0.21 | 0.30 |
| R-13 | 1.01 | 0.14 | 4.25 | 1.30 | 2.90 | 0.23 | 1.68 | 0.08 | 0.09 | 0.26 |
| R-14 | 0.35 | 0.09 | 6.06 | 3.01 | 0.62 | 0.00 | 1.77 | 0.04 | 0.13 | 0.37 |
| R-15 | 0.23 | 0.04 | 4.11 | 0.41 | 0.12 | 0.01 | 1.70 | 0.03 | 0.17 | 0.31 |
| R-16 | 0.20 | 0.01 | 4.82 | 1.24 | 0.56 | 0.03 | 1.53 | 0.01 | 0.11 | 0.25 |
| R-17 | 0.06 | 0.00 | 2.47 | 0.05 | 0.27 | 0.02 | 1.56 | 0.01 | 0.14 | 0.33 |
| R-18 | 0.06 | N.A. | 1.90 | 1.53 | 0.17 | 0.01 | 1.04 | 0.05 | 0.08 | 0.35 |
| R-19 | 0.11 | 0.10 | 0.82 | 0.02 | 0.14 | 0.01 | 0.97 | N.A. | 0.11 | 0.27 |
| R-20 | 0.27 | 0.16 | 0.57 | 0.01 | 0.27 | 0.01 | 2.04 | N.A. | 0.15 | 0.34 |
| R-21 | 1.42 | 0.52 | 0.72 | 0.18 | 0.37 | 0.02 | 1.79 | N.A. | 0.04 | 0.16 |
| R-22 | 0.28 | 0.04 | 0.73 | 0.04 | 0.28 | 0.00 | 1.49 | N.A. | 0.09 | 0.21 |
| R-23 | 0.03 | N.A. | 1.18 | 0.12 | 0.04 | 0.00 | 0.70 | 0.05 | 0.06 | 0.21 |
| R-24 | 0.07 | 0.08 | 1.07 | 0.04 | 0.03 | 0.00 | 1.42 | 0.01 | 0.05 | 0.12 |
| R-25 | 0.08 | 0.03 | 0.59 | 0.52 | 0.22 | 0.00 | 1.25 | 0.08 | 0.09 | 0.24 |
| R-26 | 0.72 | 0.17 | 1.13 | 0.51 | 1.04 | 0.03 | 1.36 | 0.13 | 0.12 | 0.30 |
| R-27 | 0.56 | 0.15 | 0.86 | 0.09 | 0.71 | 0.02 | 1.31 | 0.10 | 0.19 | 0.40 |
| R-28 | 0.35 | 0.17 | 1.84 | 0.18 | 0.51 | 0.47 | 1.60 | 0.12 | 0.18 | 0.36 |
| R-29 | 1.61 | 0.67 | 1.47 | 0.19 | 1.24 | 0.02 | 1.19 | 0.07 | 0.27 | 0.36 |
| R-30 | 1.69 | 0.56 | 1.11 | 0.39 | 0.45 | 0.00 | 1.04 | 0.18 | 0.20 | 0.36 |
| R-31 | 0.51 | 0.02 | 0.82 | 0.38 | 0.67 | 0.00 | 1.08 | 0.14 | 0.15 | 0.26 |
| R-32 | 0.28 | 0.07 | 0.61 | 0.13 | 1.36 | 0.06 | 1.75 | 0.27 | 0.20 | 0.33 |
| R-3 3 | 1.61 | 0.27 | 1.18 | 0.23 | 1.84 | 0.00 | 1.40 | 0.43 | 0.10 | 0.18 |

| | | | | | | | | | | |
|---|---|---|---|---|---|---|---|---|---|---|
| Note: N.A. indicates that the experimental data is missing. | | | | | | | | | | |

The results showed that the siRNAs of the present invention were able to effectively reduce the level of ANGPTL3 mRNA in HepG2 cells, Huh-7 cells, or RGA cells. Among them, R-6, R-15, R-23, and R-24 were able to better reduce the activity of ANGPTL3 mRNA.

### 3.2 Screening for modified siRNA sequences

After modification of the siRNAs obtained in Example 3.1 (synthesized by Genscript Biotech Corporation), Huh7 and HepG2 cells were assayed using the fluorogenic quantitative PCR method of Example 2, and HEK293-psiCheck-ANGPTL3 was functionally assayed *in vitro* using the reporter gene method of Example 2. All results were normalized using a negative control (NC in Table 1). The siRNA sequences are shown in Tables 2 and 3, and the relative expression levels of ANGPTL3 mRNA of the siRNA sequences are shown in Table 5 and Tables 6-1 and 6-2.

**Table 5: In vitro activity assay of modified siRNAs**

| Number | Huh7 cell | | | | HepG2 cell | | | |
|---|---|---|---|---|---|---|---|---|
| | 10nM | | 0.1nM | | 10nM | | 0.1nM | |
| | Mean value | Standard deviation | Mean value | Standard deviation | Mean value | Standard deviation | Mean value | Standard deviation |
| SR-1-01 | 0.09 | 0.00 | 0.59 | 0.02 | 0.08 | 0.02 | 0.99 | 0.18 |
| SR-1-02 | 0.11 | 0.02 | 0.53 | 0.01 | 0.08 | 0.03 | 1.30 | 0.23 |
| SR-1-03 | 0.11 | 0.01 | 1.03 | 0.10 | 0.09 | 0.00 | 1.29 | 0.13 |
| SR-1-04 | 0.13 | 0.01 | 0.61 | 0.02 | 0.06 | 0.02 | 1.24 | 0.01 |
| SR-1-05 | 0.13 | 0.02 | 0.57 | 0.01 | 0.04 | 0.00 | 1.00 | 0.17 |
| SR-1-06 | 0.18 | 0.01 | 0.66 | 0.00 | 0.11 | 0.00 | 1.11 | 0.00 |
| SR-1-07 | 0.21 | 0.02 | 0.77 | 0.01 | 0.06 | 0.02 | 1.19 | 0.08 |
| SR-1-08 | 0.22 | 0.01 | 0.67 | 0.04 | 0.13 | 0.06 | 0.73 | 0.05 |
| SR-1-09 | 0.11 | 0.00 | 0.58 | 0.04 | 0.10 | 0.01 | 3.44 | 0.32 |
| SR-1-10 | 0.10 | 0.00 | 0.69 | 0.01 | 0.12 | 0.01 | 0.93 | 0.09 |
| SR-1-11 | 0.08 | 0.00 | 0.66 | 0.02 | 0.12 | 0.02 | 1.21 | 0.05 |
| SR-1-12 | 0.10 | 0.00 | 0.76 | 0.01 | 0.23 | 0.08 | 1.24 | 0.03 |
| SR-1-13 | 0.10 | 0.00 | 0.73 | 0.04 | 0.13 | 0.01 | 1.00 | 0.02 |
| SR-1-14 | 0.16 | 0.00 | 0.77 | 0.02 | 0.16 | 0.03 | 1.10 | 0.04 |
| SR-1-15 | 0.14 | 0.01 | 0.59 | 0.00 | 0.02 | 0.01 | 0.78 | 0.06 |
| SR-1-16 | 0.16 | 0.01 | 0.67 | 0.00 | 0.04 | 0.00 | 0.85 | 0.02 |
| SR-1-17 | 0.16 | 0.01 | 0.64 | 0.01 | 0.03 | 0.01 | 0.97 | 0.01 |
| SR-1-18 | 0.11 | 0.01 | 0.65 | 0.02 | 0.04 | 0.01 | 0.92 | 0.03 |
| SR-1-19 | 0.21 | 0.00 | 0.72 | 0.00 | 0.05 | 0.01 | 0.74 | 0.01 |
| SR-1-20 | 0.21 | 0.01 | 0.75 | 0.00 | 0.06 | 0.00 | 0.96 | 0.12 |
| SR-1-21 | 0.24 | 0.02 | 0.87 | 0.06 | 0.05 | 0.01 | 0.76 | 0.04 |
| SR-1-22 | 0.20 | 0.00 | 0.67 | 0.03 | 0.06 | 0.00 | 0.74 | 0.02 |
| SR-1-23 | 0.08 | 0.00 | 0.70 | 0.00 | 0.03 | 0.01 | 0.92 | 0.12 |
| SR-1-24 | 0.22 | 0.00 | 0.71 | 0.02 | 0.08 | 0.01 | 0.91 | 0.29 |
| SR-1-25 | 0.24 | 0.00 | 0.76 | 0.00 | 0.08 | 0.00 | 1.03 | 0.00 |
| SR-1-26 | 0.18 | 0.00 | 0.73 | 0.01 | 0.07 | 0.02 | 1.04 | 0.02 |
| SR-1-27 | 0.11 | 0.00 | 0.78 | 0.03 | 0.03 | 0.00 | 0.86 | 0.01 |
| SR-1-28 | 0.16 | 0.00 | 0.95 | 0.02 | 0.05 | 0.00 | 0.96 | 0.13 |
| SR-1-29 | 0.20 | 0.00 | 0.88 | 0.04 | 0.04 | 0.01 | 0.72 | 0.04 |
| SR-4-01 | 0.55 | 0.00 | 1.43 | 0.07 | 0.16 | 0.03 | 1.00 | 0.10 |
| SR-4-02 | 0.76 | 0.00 | 1.60 | 0.05 | 0.21 | 0.01 | 1.00 | 0.09 |
| SR-4-03 | 0.45 | 0.03 | 1.01 | 0.02 | 0.14 | 0.03 | 1.10 | 0.14 |
| SR-4-04 | 0.31 | 0.02 | 1.30 | 0.04 | 0.09 | 0.02 | 0.74 | 0.05 |
| SR-4-05 | 0.33 | 0.01 | 1.07 | 0.07 | 0.08 | 0.02 | 0.65 | 0.09 |
| SR-4-06 | 0.55 | 0.00 | 1.12 | 0.08 | 0.10 | 0.02 | 0.86 | 0.14 |
| SR-4-07 | 0.50 | 0.00 | 1.17 | 0.07 | 0.12 | 0.01 | 0.78 | 0.19 |
| SR-4-08 | 0.66 | 0.01 | 1.15 | 0.11 | 0.17 | 0.01 | 0.92 | 0.64 |
| SR-4-09 | 0.54 | 0.01 | 1.03 | 0.05 | 0.13 | 0.05 | 1.51 | 0.27 |
| SR-4-10 | 0.55 | 0.00 | 0.83 | 0.01 | 0.11 | 0.01 | 1.16 | 0.19 |
| SR-4-11 | 0.58 | 0.01 | 1.26 | 0.08 | 0.16 | 0.05 | 1.02 | 0.45 |
| SR-4-12 | 0.08 | 0.00 | 1.13 | 0.03 | 0.07 | 0.01 | 0.79 | 0.16 |
| SR-4-13 | 0.04 | 0.00 | 1.04 | 0.04 | 0.05 | N.A. | 1.89 | 0.53 |
| SR-4-14 | 0.06 | 0.00 | 1.02 | 0.02 | 0.03 | 0.01 | 1.32 | 0.08 |
| SR-4-15 | 0.25 | 0.01 | 0.67 | 0.01 | 0.06 | 0.01 | 1.82 | 0.46 |
| SR-4-16 | 0.04 | 0.00 | 0.81 | 0.02 | 0.03 | 0.01 | 1.95 | 0.02 |
| SR-4-17 | 0.07 | 0.00 | 0.86 | 0.02 | 0.04 | 0.01 | 1.03 | 0.39 |
| SR-4-18 | 0.06 | 0.00 | 0.85 | 0.02 | 0.04 | N.A. | 1.44 | 0.03 |
| SR-4-19 | 0.09 | 0.01 | 0.98 | 0.06 | 0.02 | 0.00 | 1.23 | 0.02 |
| SR-4-20 | 0.06 | 0.00 | 1.16 | 0.01 | 0.04 | 0.00 | 0.90 | 0.16 |
| SR-4-21 | 0.05 | 0.00 | 1.25 | 0.01 | 0.03 | 0.00 | 1.39 | 0.23 |
| SR-4-22 | 0.07 | 0.00 | 0.69 | 0.01 | 0.04 | 0.01 | 1.68 | 0.09 |
| SR-4-23 | 0.55 | 0.00 | 0.60 | 0.01 | 0.10 | 0.00 | 0.94 | 0.37 |
| SR-4-24 | 0.12 | 0.00 | 0.82 | 0.03 | 0.07 | 0.01 | 1.06 | 0.19 |
| SR-4-25 | 0.06 | 0.00 | 0.80 | 0.06 | 0.02 | 0.00 | 0.79 | 0.08 |
| SR-4-26 | 0.05 | 0.00 | 0.67 | 0.00 | 0.04 | 0.00 | 1.31 | 0.30 |
| SR-3-01 | 0.05 | 0.00 | 0.67 | 0.00 | 0.06 | 0.01 | 1.05 | 0.10 |
| SR-3-02 | 0.07 | 0.00 | 0.73 | 0.02 | 0.15 | 0.01 | 1.59 | 0.00 |
| SR-3-03 | 0.06 | 0.00 | 0.69 | 0.02 | 0.10 | 0.00 | 0.92 | 0.17 |
| SR-3-04 | 0.08 | 0.00 | 0.92 | 0.04 | 0.17 | 0.00 | 1.89 | 0.21 |
| SR-3-05 | 0.05 | 0.00 | 1.00 | 0.03 | 0.35 | 0.00 | 1.15 | 0.11 |
| SR-3-06 | 0.05 | 0.00 | 0.67 | 0.03 | 0.27 | 0.09 | 1.60 | 0.02 |
| SR-3-07 | 0.09 | 0.00 | 0.75 | 0.01 | 0.22 | 0.05 | 1.33 | 0.01 |
| SR-3-08 | 0.05 | 0.01 | 0.85 | 0.01 | 0.11 | 0.05 | 1.28 | 0.08 |
| SR-3-09 | 0.06 | 0.00 | 0.82 | 0.02 | 0.06 | 0.01 | 0.91 | 0.03 |
| SR-3-10 | 0.04 | 0.00 | 0.80 | 0.02 | 0.08 | 0.00 | 1.45 | 0.17 |
| SR-3-11 | 0.05 | 0.00 | 1.02 | 0.13 | 0.06 | 0.02 | 0.95 | 0.14 |
| SR-3-12 | 0.06 | 0.01 | 1.01 | 0.00 | 0.12 | 0.05 | 1.07 | 0.04 |
| SR-3-13 | 0.04 | 0.00 | 0.99 | 0.02 | 0.09 | 0.01 | 1.75 | 0.09 |
| SR-3-14 | 0.06 | 0.00 | 0.93 | 0.00 | 0.13 | 0.02 | 1.87 | 0.31 |
| SR-3-15 | 0.23 | 0.00 | 0.94 | 0.02 | 0.25 | 0.02 | 1.69 | 0.01 |
| SR-3-16 | 0.04 | 0.00 | 1.06 | 0.02 | 0.12 | 0.01 | 1.33 | 0.07 |
| SR-3-17 | 0.05 | 0.00 | 1.01 | 0.01 | 0.13 | 0.01 | 1.72 | 0.03 |
| SR-3-18 | 0.04 | 0.00 | 1.22 | 0.01 | 0.13 | 0.01 | 1.27 | 0.44 |
| SR-3-19 | 0.05 | 0.01 | 0.92 | 0.04 | 0.18 | 0.01 | 1.46 | 0.17 |
| SR-3-20 | 0.06 | 0.00 | 0.87 | 0.00 | 0.18 | 0.02 | 2.00 | 0.42 |
| SR-3-21 | 0.04 | 0.00 | 1.23 | 0.02 | N.A. | N.A. | 1.20 | 0.20 |
| SR-3-22 | 0.05 | 0.00 | 1.00 | 0.01 | 0.19 | 0.03 | 1.18 | 0.17 |
| SR-3-23 | 0.28 | 0.00 | 1.07 | 0.04 | 0.30 | 0.00 | 1.42 | 0.22 |
| SR-3-24 | 0.11 | 0.01 | 1.12 | 0.02 | 0.32 | 0.03 | 1.34 | 0.06 |
| SR-3-25 | 0.07 | 0.00 | 1.00 | 0.01 | 0.18 | 0.05 | 2.09 | 0.45 |
| SR-3-26 | 0.05 | 0.00 | 1.42 | 0.02 | 0.25 | 0.02 | 1.15 | 0.25 |
| SR-2-01 | 0.18 | 0.01 | 0.92 | 0.03 | 0.27 | 0.05 | 1.36 | 0.17 |
| SR-2-02 | 0.15 | 0.00 | 1.06 | 0.03 | 0.18 | N.A. | 1.29 | 0.07 |
| SR-2-03 | 0.12 | 0.00 | 1.07 | 0.01 | 0.20 | 0.02 | 1.32 | 0.13 |
| SR-2-04 | 0.12 | 0.00 | 1.03 | 0.00 | 0.27 | N.A. | 1.14 | 0.06 |
| SR-2-05 | 0.12 | 0.00 | 1.04 | 0.02 | 0.23 | 0.04 | 1.51 | 0.04 |
| SR-2-06 | 0.14 | 0.00 | 0.85 | 0.03 | 0.19 | N.A. | 1.26 | 0.22 |
| SR-2-07 | 0.17 | 0.00 | 0.82 | 0.01 | 0.32 | N.A. | 1.11 | 0.02 |
| SR-2-08 | 0.18 | 0.00 | 0.85 | 0.03 | 0.21 | 0.01 | 1.05 | 0.01 |
| SR-2-09 | 0.32 | 0.00 | 0.90 | 0.00 | 0.12 | N.A. | 1.27 | 0.14 |
| SR-2-10 | 0.61 | 0.00 | 1.04 | 0.02 | 0.46 | 0.04 | 1.25 | 0.10 |
| SR-2-11 | 0.17 | 0.00 | 0.96 | 0.02 | 0.25 | 0.02 | 1.24 | 0.07 |
| SR-2-12 | 0.31 | 0.01 | 1.27 | 0.02 | 0.19 | N.A. | 1.19 | 0.03 |
| SR-2-13 | 0.20 | 0.01 | 0.95 | 0.02 | 0.18 | 0.00 | 1.18 | 0.05 |
| SR-2-14 | 0.14 | 0.01 | 0.94 | 0.03 | 0.17 | 0.00 | 1.05 | 0.07 |
| SR-2-15 | 0.40 | 0.28 | 0.64 | 0.13 | 0.23 | N.A. | 0.87 | 0.10 |
| SR-2-16 | 0.68 | 0.45 | 0.79 | 0.13 | 0.34 | 0.30 | 0.79 | 0.03 |
| SR-2-17 | 0.95 | 0.64 | 0.77 | 0.10 | 0.87 | 0.66 | 0.75 | 0.01 |
| SR-2-18 | 0.50 | 0.33 | 0.79 | 0.10 | 0.30 | 0.22 | 0.67 | 0.04 |
| SR-2-19 | 0.52 | 0.35 | 0.85 | 0.14 | 0.26 | 0.21 | 0.70 | 0.13 |
| SR-2-20 | 0.49 | 0.34 | 0.91 | 0.11 | 0.40 | 0.31 | 0.74 | 0.19 |
| SR-2-21 | 0.28 | 0.20 | 0.76 | 0.09 | 0.58 | N.A. | 0.66 | 0.06 |
| SR-2-22 | 0.34 | 0.25 | 0.79 | 0.08 | 0.19 | N.A. | 3.59 | 0.69 |
| SR-2-23 | 0.37 | 0.26 | 0.77 | 0.28 | 0.34 | 0.18 | 0.77 | 0.07 |
| SR-2-24 | 0.60 | 0.43 | 0.75 | 0.25 | 0.31 | N.A. | 1.12 | 0.00 |
| SR-2-25 | 0.86 | 0.57 | 0.85 | 0.27 | 0.57 | 0.33 | 0.62 | 0.00 |
| SR-2-26 | 0.50 | 0.33 | 1.14 | 0.34 | 0.41 | 0.30 | 0.87 | 0.08 |
| SR-2-27 | 0.31 | 0.21 | 1.07 | 0.38 | 0.20 | 0.13 | 0.71 | 0.07 |
| SR-2-28 | 1.27 | 0.78 | 1.25 | 0.44 | 1.67 | 1.06 | 0.72 | 0.06 |

| | | | | | | | | |
|---|---|---|---|---|---|---|---|---|
| Note: N.A. indicates that the experimental data is missing. | | | | | | | | |

**Table 6-1: In vitro activity assay of modified siRNAs**

| Number | HEK293-psiCheck-ANGPTL3 cell | | | |
|---|---|---|---|---|
| | 100nM | | 10nM | |
| | Mean value | Standard deviation | Mean value | Standard deviation |
| XR-2-01 | 0.28 | 0.00 | 0.53 | 0.08 |
| XR-2-02 | 0.49 | 0.00 | 0.69 | 0.00 |
| XR-2-03 | 0.16 | 0.01 | 0.79 | 0.06 |
| XR-2-04 | 0.10 | 0.01 | 0.52 | 0.02 |
| XR-2-05 | 0.18 | 0.01 | 0.65 | 0.04 |
| XR-2-06 | 0.34 | 0.01 | 0.76 | 0.08 |
| XR-2-07 | 0.31 | 0.01 | 0.80 | 0.11 |
| XR-2-08 | 0.28 | 0.01 | 0.50 | 0.06 |
| XR-2-09 | 0.22 | 0.01 | 0.46 | 0.02 |
| XR-2-10 | 0.35 | 0.02 | 0.52 | 0.01 |
| XR-2-11 | 0.30 | 0.02 | 0.57 | 0.08 |
| XR-2-12 | 0.14 | 0.00 | 0.50 | 0.01 |
| XR-2-13 | 0.15 | 0.01 | 0.42 | 0.01 |
| XR-2-14 | 0.16 | 0.01 | 0.45 | 0.01 |
| XR-2-15 | 0.13 | 0.00 | 0.57 | 0.04 |
| XR-1-01 | 0.49 | 0.01 | 0.86 | 0.00 |
| XR-1-02 | 0.28 | 0.00 | 0.89 | 0.00 |
| XR-1-03 | 0.55 | 0.02 | 0.83 | 0.06 |
| XR-1-04 | 0.46 | 0.03 | 0.87 | 0.03 |
| XR-1-05 | 0.86 | 0.02 | 0.87 | 0.05 |
| XR-1-06 | 0.35 | 0.04 | 0.93 | 0.05 |
| XR-1-07 | 0.40 | 0.00 | 0.93 | 0.01 |
| XR-1-08 | 0.30 | 0.00 | 0.89 | 0.03 |
| XR-1-09 | 0.33 | 0.02 | 0.86 | 0.00 |

**Table 6-2: In vitro activity assay of modified siRNAs**

| Number | HEK293-psiCheck-ANGPTL3 cell | | | | | |
|---|---|---|---|---|---|---|
| | 100nM | | 10nM | | 1nM | |
| | Mean value | Standard deviation | Mean value | Standard deviation | Mean value | Standard deviation |
| XR-3-01 | 0.04 | 0.00 | 0.21 | 0.02 | 0.83 | 0.08 |
| XR-3-02 | 0.06 | 0.01 | 0.23 | 0.00 | 0.87 | 0.00 |
| XR-3-03 | 0.08 | 0.02 | 0.29 | 0.03 | 0.84 | 0.02 |
| XR-3-04 | 0.07 | 0.02 | 0.29 | 0.00 | 0.82 | 0.03 |
| XR-3-05 | 0.06 | 0.02 | 0.44 | 0.06 | 0.83 | 0.06 |
| XR-4-01 | 0.23 | 0.02 | 0.77 | 0.05 | 0.78 | 0.00 |
| XR-4-02 | 0.26 | 0.09 | 0.67 | 0.04 | 0.79 | 0.03 |
| XR-4-03 | 0.22 | 0.07 | 0.64 | 0.10 | 0.80 | 0.09 |
| XR-4-04 | 0.19 | 0.00 | 0.60 | 0.11 | 0.82 | 0.01 |
| XR-4-05 | 0.20 | 0.07 | 0.49 | 0.07 | 0.81 | 0.00 |
| XR-4-06 | 0.28 | 0.01 | 0.67 | 0.05 | 0.85 | 0.07 |
| XR-4-07 | 0.33 | 0.01 | 0.67 | 0.01 | 1.08 | 0.00 |

The result showed that the siRNAs of the present invention were able to effectively reduce the level of ANGPTL3 mRNA in HepG2 cells, Huh-7 cells, or HEK293-psiCheck-ANGPTL3 cells.

### Example 4. Synthesis of siRNA conjugates

Synthesis of siRNA conjugates 1 and 2:

siRNA conjugates 1 and 2 were prepared according to the following reaction routes.

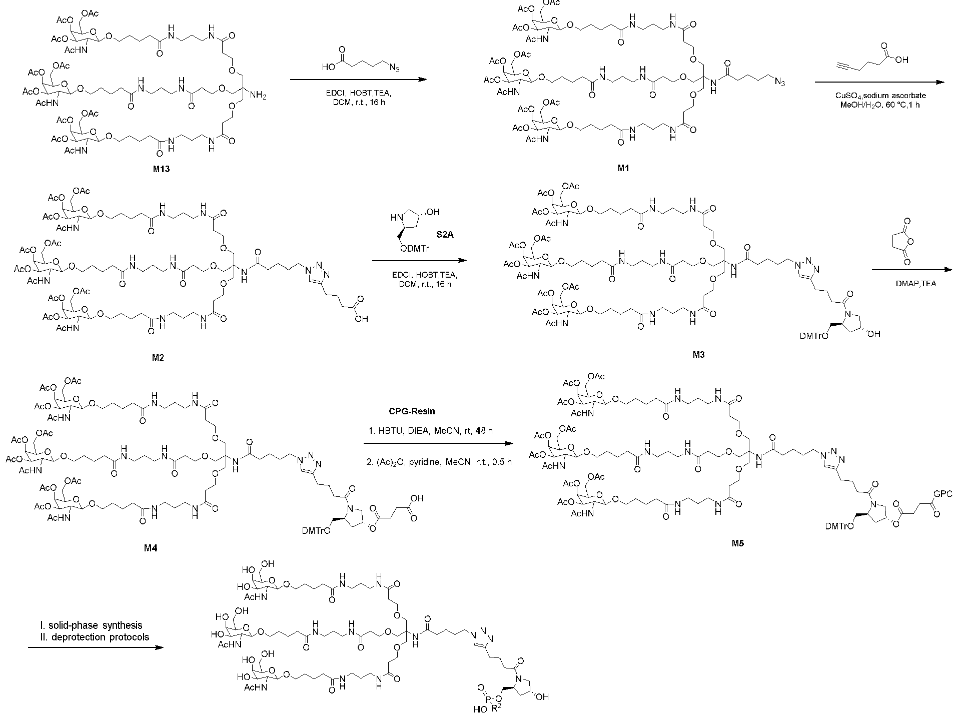

### Step 1: preparation of intermediate M1

5-Azidopentanoic acid (263.32 mg, 1.84 mmol, 1.5 *eq.)* was dissolved in 20 mL of dichloromethane, and then EDCI (352.64 mg, 1.84 mmol, 1.5 *eq.)* and HOBT (248.56 mg, 1.84 mmol, 1.5 *eq.)* were added. The mixture was stirred at 25 °C for 15 min under nitrogen atmosphere. Compound M13 (2.2 g, 1.23 mmol, 1 *eq.)* was then added to the reaction system. The resulting mixture was stirred at 25 °C for 16 h under nitrogen atmosphere. The starting material M13 (see T. P. Prakash, M. J. Graham, P. P. Seth, etc. Nucleic Acids Research, 2014, 42, 8796-8807. The same applies below.) was completely consumed as monitored by TLC (dichloromethane:methanol = 8: 1). 10 mL of water was added to the reaction mixture to quench the reaction. The reaction liquid was extracted with dichloromethane (10 mL × 3). The organic phases were combined and washed with an aqueous NaCl solution (15 mL). The resulting organic phase was dried over anhydrous sodium sulfate and concentrated by rotary evaporation to remove the solvent to obtain a crude product. The crude product was separated by silica gel column chromatography (methanol/dichloromethane = 0: 100-15: 100) to obtain M1 (1.1 g, yield: 46.7%) as a white solid.

### Step 2: preparation of intermediate M2

M1 (800 mg, 416.88 µmol, 1 *eq*.), 5-acetylic acid (46.74 mg, 416.88 µmol, 1 *eq*.), copper sulfate (79.84 mg, 500.25 µmol, 1.2 *eq*.) were added to a suspension (methanol:water = 1:1, 2 mL) of sodium ascorbate (206.47 mg, 1.04 mmol, 2.5 *eq*.) at 25 °C. The resulting mixture was heated to 60 °C and then stirred at this temperature for 1 h. The starting material was completely consumed and a new product was generated, as monitored by LC-MS. The reaction mixture was concentrated by rotary evaporation to remove the solvent, and the resulting residue was separated by silica gel column chromatography (methanol/dichloromethane = 0:100-15:100) to obtain M2 (550 mg, yield: 64.9%) as a yellow solid.

### Step 3: preparation of intermediate M3

M2 (350 mg, 172.32 µmol, 1 *eq*.) was dissolved in dichloromethane (10 mL) to give a clear solution. EDCI (49.55 mg, 258.47 µmol, 1.5 *eq.*) and HOBt (34.92 mg, 258.47 µmol, 1.5 *eq.*) were then added to the reaction system. The resulting mixture was stirred at 25 °C for 15 min under nitrogen atmosphere. Intermediate S2A (86.75 mg, 206.78 µmol, 1.2 *eq.)* (see N. Hébert, P. W. Davis, E. L. D. Baets, O. L. Acevedo, Tetrahedron Letter, 1994, 35, 9509-9512. The same applies below) was then added. The above mixture was stirred at 25 °C for 16 h. The starting material M2 was completely consumed as monitored by TLC (dichloromethane:methanol = 8:1). Water (24 mL) was added to the reaction mixture to quench the reaction. The reaction liquid was extracted with dichloromethane (24 mL × 3). The organic phases were combined and washed with an aqueous NaCl solution (30 mL). The resulting organic phase was dried over anhydrous sodium sulfate and concentrated by rotary evaporation to remove the solvent to obtain a crude product. The crude product was separated by silica gel column chromatography (methanol/dichloromethane (0.1% triethylamine) = 0: 100-15: 100) to give M3 (350 mg, yield: 83.5%) as a yellow solid.

### Step 4: preparation of intermediate M4

M3 (350 mg, 143.88 mmol, 1 *eq.*), succinic anhydride (115.18 mg, 1.15 mmol, 8 *eq*.), DMAP (35.15 mg, 287.75 µmol, 2 *eq*.), and TEA (349.41 mg, 3.45 mmol, 479.96 µL, 24 *eq.*) were dissolved in DCM (10 mL). The resulting mixture was stirred at 20 °C for 16 h under nitrogen atmosphere. The starting material was completely consumed and a new product was generated, as monitored by LC-MS. The reaction mixture was concentrated by rotary evaporation to remove the solvent to obtain a crude product. The above crude product was separated by preparative HPLC (carbon-18 chromatographic column, acetonitrile/0.01% ammonia water) to obtain M4 (155 mg, yield: 44.1%) as a white solid. HPLC purity: 99.48%, LCMS (ESI), Cal. for C₁₂₀H₁₇₄N₁₄O₄₅: 2531.2, Found [M+H]⁺: 2532.6.

### Step 5: preparation of intermediate M5

(I) M4 (92 mg, 36.32 µmol, 1 *eq*.) and HBTU (27.55 mg, 72.65 µmol, 2 *eq*.) were dissolved in 8 mL of acetonitrile, and then *N*,*N-*diisopropylethylamine (18.78 mg, 145.30 µmol, 25.31 µL, 4 *eq*.) was added. The mixture was shaken for 3-4 min, and then a CPG-amino resin (1.09 g, 50 µmol/g) was added. The resulting mixture was shaken on a shaker at room temperature for 48 h. Then, the mixture was filtered, and the filter cake was washed twice with acetonitrile (25 mL). The resulting solid was dried at 45 °C for two hours to obtain a white solid (1.08 g). (II) Acetic anhydride (3.64 mg, 35.64 µmol, 0.0931 *eq*.) and pyridine (7.77 mg, 98.18 µmol, 7.90 µL, 0.256 *eq.*) were dissolved in 10 mL of acetonitrile. The resulting mixture was shaken uniformly, and then the above white solid (1.08 g, 382.95 µmol, 1 *eq.*) was added. The resulting mixture was shaken on a shaker at room temperature for 0.5 h. Then, the mixture was filtered, and the filter cake was washed twice with acetonitrile (10 mL). The resulting solid was dried at 45 °C for two hours to give a solid phase-loaded product M5 (1.08 g) as a white powder. The loading amount was 23.3 µmol/g.

### Step 6: preparation of siRNA conjugates 1 and 2

siRNA XR-3-01 (SEQ ID NO: 200/234) or siRNA XR-3-02 (SEQ ID NO: 201/235) was used as R², and siRNA conjugates 1 and 2 were obtained after solid phase synthesis and deprotection (see M. J. Damha, K. K. Ogilvie, Methods Mol. Biol. 1993, 20, 81-114; the same applies below) of the solid phase-loaded product M5.

### Synthesis of siRNA conjugates 3 and 4

siRNA conjugates **3 and 4** were prepared according to the following reaction routes.

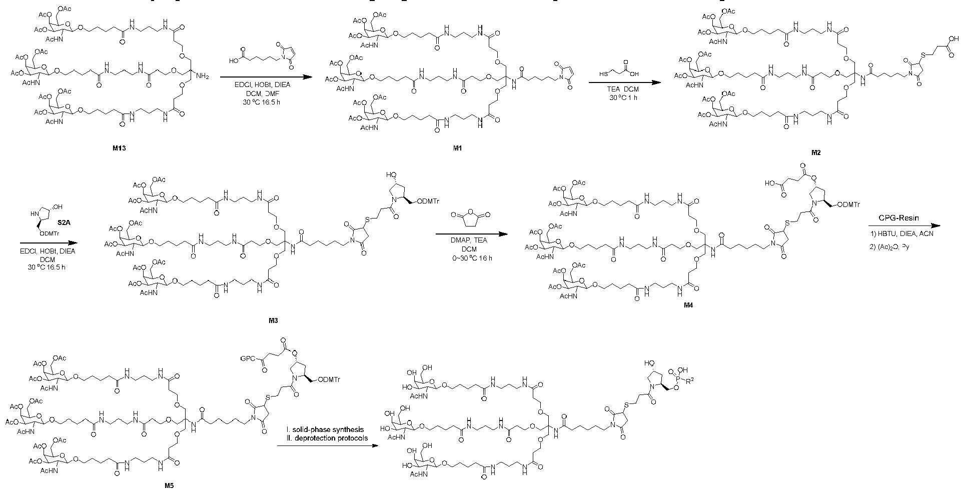

### Step 1: preparation of intermediate M1

HOBT (120.51 mg, 891.91 µmol, 2 *eq.*) and EDCI (170.98 mg, 891.91 µmol, 2 *eq.*) were dissolved in dichloromethane DCM (10 mL), and then *N*,*N-*diisopropylethylamine (288.18 mg, 2.23 mmol, 388.38 µL, 5 *eq.*) was added, followed by SM1 (145 mg, 686.51 µmol, 1.54 *eq.*)*.* After stirring for 0.5 h, M13 (800 mg, 445.96 µmol, 1 *eq.*) was added to the above mixture. The resulting mixture was stirred at 25 °C for 16 h under nitrogen atmosphere. A new product was generated as monitored by LC-MS. 15 mL of water was added to the reaction mixture to quench the reaction. The reaction liquid was extracted with dichloromethane (30 mL × 2). The organic phases were combined and washed with an aqueous NaCl solution (30 mL). The resulting organic phase was dried over anhydrous sodium sulfate and concentrated by rotary evaporation to remove the solvent to obtain a crude product. The crude product was separated by silica gel column chromatography (dichloromethane/methanol = 99:1, 95:5, 90:10, and 80:20) to obtain M1 (613 mg) as a light yellow solid.

### Step 2: preparation of intermediate M2

M1 (400 mg, 161.04 µmol, 1 *eq.*) and 3-mercaptopropionic acid (17.09 mg, 161.04 µmol, 14.03 µL, 1 *eq.*) were dissolved in dichloromethane (10 mL), and then triethylamine (48.89 mg, 483.12 µmol, 67.15 µL, 3 *eq.*) was added. The resulting mixture was stirred at 25 °C for 0.5 h. A target product was generated as monitored by LC-MS. The reaction mixture was used directly in the next step.

### Step 3: preparation of intermediate M3

EDCI (92.61 mg, 483.11 µmol, 3 *eq.*)*,* HOBT (65.28 mg, 483.11 µmol, 3 *eq.*)*,* and *N*,*N-*diisopropylethylamine (124.88 mg, 966.22 µmol, 168.30 µL, 6 *eq.*) were added to a solution ofM2 (337.09 mg, 161.04 µmol, 1 *eq.*) in dichloromethane (5 mL). The mixture was stirred at 30 °C for 0.5 h under nitrogen atmosphere. Then, a solution of S2A (135.11 mg, 322.07 µmol, 2 *eq.*) in dichloromethane (3 mL) was added to the above mixture, and the resulting mixture was stirred at 30 °C for 16 h. A new product was generated as monitored by HPLC-MS. The reaction liquid was concentrated by rotary evaporation to remove the solvent to obtain a crude product. The crude product was separated by silica gel column chromatography (dichloromethane/methanol = 99:1, 95:5, 93:7, and 90:10) to obtain M3 (420 mg).

### Step 4: preparation of intermediate M4

DMAP (28.79 mg, 235.70 µmol, 2 *eq.*)*,* triethylamine (536.62 mg, 5.30 mmol, 737.12 µL, 45 *eq.*)*,* and succinic anhydride (176.90 mg, 1.77 mmol, 15 *eq.*) were added to a solution of M3 (420.00 mg, 117.85 µmol, 1 *eq.*) in dichloromethane (20 mL) in an ice bath. The resulting mixture was stirred at 25-30 °C for 16 h under nitrogen atmosphere. A new product was generated as monitored by HPLC-MS. The reaction liquid was concentrated by rotary evaporation to remove the solvent to obtain a crude product. The above crude product was separated by preparative HPLC (carbon-18 chromatographic column, acetonitrile/0.01% ammonia water) twice to obtain M4 (40 mg, yield: 44.1%) as a white solid. HPLC purity: 98.83%, LCMS (ESI), Cal. for C₁₂₂H₁₇₆N₁₂O₄₇S: 2594.85, Found [M+H]⁺: 2595.7.

### Step 5: preparation of intermediate M5

(I) M4 (80 mg, 30.83 µmol, 1 *eq.*) and HBTU (23.38 mg, 61.66 µmol, 2 *eq.*) were dissolved in 10 mL of acetonitrile, and then *N*,*N-*diisopropylethylamine (15.94 mg, 123.32 µmol, 21.48 µL, *4 eq.*) was added. The mixture was shaken for 3-4 min, and then a CPG-amino resin (925.00 mg, 50 µmol/g) was added. The resulting mixture was shaken on a shaker at room temperature for 48 h. Then, the mixture was filtered, and the filter cake was washed twice with acetonitrile (25 mL). The resulting solid was dried at 35 °C for two hours to obtain a white solid (950 mg). (II) Acetic anhydride (4.85 mg, 47.5 µmol, 4.49 µL, 1.24e-1 *eq.*) and pyridine (11.27 mg, 142.5 µmol, 11.47 µL, 3.72e-1 *eq.*) were dissolved in 10 mL of acetonitrile. The resulting mixture was shaken uniformly, and then the above white solid was added. The resulting mixture was shaken on a shaker at room temperature for 0.5 h. Then, the mixture was filtered, and the filter cake was washed twice with acetonitrile (10 mL). The resulting solid was dried at 40 °C for two hours to give a solid phase-loaded product M5 (925 mg) as a white powder. The loading amount was 18.9 µmol/g.

### Step 6: preparation of siRNA conjugates 3 and 4

siRNA XR-3-01 (SEQ ID NO: 200/234) or siRNA XR-3-02 (SEQ ID NO: 201/235) was used as R², and siRNA conjugates 3 and 4 were obtained after solid phase synthesis and deprotection of the solid phase-loaded product M5.

### Synthesis of siRNA conjugates 5 and 6

siRNA conjugates 5 and 6 were prepared according to the following reaction routes.

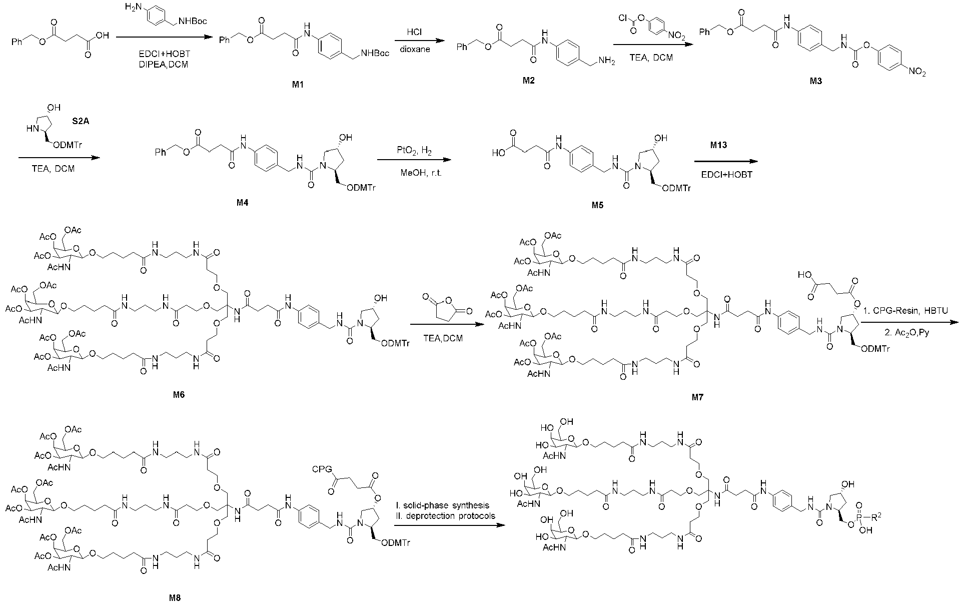

### Step 1: preparation of intermediate M1

Monobenzyl succinate (1.44 g, 6.93 mmol, 1.1 *eq.*)*,* EDCI (1.81 g, 9.45 mmol, 1.5 *eq.*)*,* and HOBT (1.28 g, 9.45 mmol, 1.5 *eq.*) were dissolved in 30 mL of dichloromethane, and then 4-(N-Boc-aminomethyl)aniline (1.4 g, 6.30 mmol, 1 *eq.*) was added. The resulting mixture was stirred at 25 °C for 2 h. The starting material was completely consumed and a new product was generated, as monitored by LC-MS. 15 mL of water was added to the reaction mixture to quench the reaction. The reaction liquid was extracted with dichloromethane (20 mL × 2). The organic phases were combined and washed with an aqueous NaCl solution (15 mL). The resulting organic phase was dried over anhydrous sodium sulfate and concentrated by rotary evaporation to remove the solvent to obtain a residue. The residue was separated by silica gel column chromatography (ethyl acetate/petroleum ether = 0:100-20:100) to obtain M1 (2.9 g, yield: 81.5%) as a yellow solid.

### Step 2: preparation of intermediate M2

M1 was dissolved (1.0 g, 2.42 mmol, 1 *eq.*) in 10 mL of 1,4-dioxane. Then, a 4 N solution of HCl in dioxane (40.00 mmol, 10 mL, 16.5 *eq.*) was added. The resulting mixture was stirred at 25 °C for 1 h. The starting material was completely consumed and a new product was generated, as monitored by LC-MS. The reaction mixture was concentrated by rotary evaporation to remove the solvent to obtain a hydrochloride salt of M2 (840 mg) as a yellow solid. The yield was more than 95%.

### Step 3: preparation of intermediate M3

The hydrochloride salt of M2 (840 mg, 2.41 mmol, 1 *eq.*) and triethylamine (731.03 mg, 7.22 mmol, 1.00 mL, 3 *eq.*) were dissolved in 40 mL of dichloromethane, and then *p*-nitrophenyl chloroformate (970.77 mg, 4.82 mmol, 2 *eq.*) was added. The resulting mixture was stirred at 25 °C for 1 h. The starting material was completely consumed and a new product was generated, as monitored by LC-MS. 30 mL of water was added to the reaction mixture to quench the reaction. The reaction liquid was extracted with dichloromethane (30 mL × 2). The organic phases were combined and washed with an aqueous sodium chloride solution (30 mL). The resulting organic phase was dried over anhydrous sodium sulfate and concentrated by rotary evaporation to remove the solvent to obtain a residue. The residue was recrystallized from dichloromethane (20 mL) to give M3 (800 mg, yield: 69.6%) as a white solid.

### Step 4: preparation of intermediate M4

M3 (600 mg, 1.26 mmol, 1 *eq.*) and S2A (527.17 mg, 1.26 mmol, 1 *eq.*) were dissolved in 25 mL of a mixed solution of dichloromethane and tetrahydrofuran (dichloromethane/tetrahydrofuran = 4:1, v/v), and then triethylamine (508.64 mg, 5.03 mmol, 698.68 µL, 4 *eq.*) was added. The resulting mixture was stirred at 25 °C for 2 h. The starting material was completely consumed and a new product was generated, as monitored by LC-MS. 20 mL of water was added to the reaction mixture to quench the reaction. The reaction liquid was extracted with dichloromethane (20 mL × 2). The organic phases were combined and washed with an aqueous NaCl solution (30 mL). The resulting organic phase was dried over anhydrous sodium sulfate and concentrated by rotary evaporation to remove the solvent to obtain a residue. The residue was separated by silica gel column chromatography (methanol/dichloromethane = 0:100-4:100) to obtain M4 (800 mg, yield: 84.0%) as a yellow solid.

### Step 5: preparation of intermediate M5

M4 (600.00 mg, 791.69 µmol, 1 *eq.*) was dissolved in 20 mL of methanol, and then PtO₂ (257.14 mg, 1.13 mmol, 1.43 *eq.*) was added. The resulting mixture was stirred at 25-30 °C for 3 h under hydrogen atmosphere. The starting material was completely consumed and a new product was generated, as monitored by LC-MS. After the reaction mixture was filtered, 15 mL of water was added. The reaction liquid was extracted with dichloromethane (20 mL × 2). The organic phases were combined and washed with an aqueous NaCl solution (30 mL). The resulting organic phase was dried over anhydrous sodium sulfate and concentrated by rotary evaporation to remove the solvent to obtain a residue. The residue was separated by silica gel column chromatography (methanol:dichloromethane = 0:100-20:100, containing 0.1% triethylamine) to obtain M5 (500 mg, yield: 94.6%) as a yellow solid.

### Step 6: preparation of intermediate M6

M13 (268.65 mg, 149.76 µmol, 1 *eq.*) was dissolved in 10 mL of dichloromethane, and then EDCI (57.42 mg, 299.52 µmol, 2.0 *eq.*) and HOBT (40.47 mg, 299.52 µmol, 2.0 *eq.*) were added. The resulting mixture was stirred at 25 °C for 15 min under nitrogen atmosphere. M5 (100 mg, 149.76 µmol, 1 *eq.*) was then added to the reaction system. The resulting mixture was stirred at 25 °C for 16 h. The starting material was completely consumed and a new product was generated, as monitored by LC-MS. 15 mL of water was added to the reaction liquid. The reaction liquid was extracted with dichloromethane (10 mL × 2). The organic phases were combined and washed with an aqueous NaCl solution (10 mL). The resulting organic phase was dried over anhydrous sodium sulfate and concentrated by rotary evaporation to remove the solvent to obtain a residue. The residue was separated by silica gel column chromatography (methanol:dichloromethane = 0:100-5:100, containing 0.1% triethylamine) to obtain M6 (300 mg, yield: 68.0%) as a white solid.

### Step 7: preparation of intermediate M7

M6 (300 mg, 122.77 µmol, 1 *eq.*)*,* succinic anhydride (122.86 mg, 1.23 mmol, 10 *eq.*)*,* DMAP (30.00 mg, 245.54 µmol, 2 *eq.*)*,* and TEA (372.69 mg, 3.68 mmol, 511.93 µL, 30 *eq.*) were dissolved in 20 mL of dichloromethane. The resulting mixture was stirred at 20 °C for 16 h under nitrogen atmosphere. The starting material was completely consumed and a new product was generated, as monitored by LC-MS. The reaction mixture was concentrated by rotary evaporation to remove the solvent to obtain an oil. The above oil was separated by preparative HPLC (carbon-18 chromatographic column, acetonitrile/0.01% ammonia water) to obtain M7 (53 mg, yield: 37.9%) as a white solid. LCMS (ESI), Cal. for C₁₂₁H₁₇₁N₁₃O₄₆: 2542.2, Found [M+H]⁺: 2543.2.

### Step 8: preparation of intermediate M8

(I) M7 (53 mg, 20.84 µmol, 1 *eq.*) and HBTU (15.80 mg, 41.67 µmol, 2 *eq.*) were dissolved in 10 mL of acetonitrile, and then *N*,*N-*diisopropylethylamine (10.77 mg, 83.34 µmol, 14.52 µL, 4 *eq.*) was added. The mixture was shaken for 3-4 min, and then a CPG-amino resin (625 mg, 50 µmol/g) was added. The resulting mixture was shaken on a shaker at room temperature for 48 h. Then, the mixture was filtered, and the filter cake was washed twice with acetonitrile (10 mL). The resulting solid was dried at 45 °C for two hours to obtain a white solid (620 mg). (II) Acetic anhydride (3.06 mg, 30 µmol, 1.37e-1 *eq.*) and pyridine (4.44 mg, 56.15 µmol, 4.52 µL, 2.56e-1 *eq.*) were dissolved in 10 mL of acetonitrile. The resulting mixture was shaken uniformly, and then the above white solid was added. The resulting mixture was shaken on a shaker at room temperature for 0.5 h. Then, the mixture was filtered, and the filter cake was washed twice with acetonitrile (10 mL). The resulting solid was dried at 45 °C for 2 h to obtain a solid phase-loaded product M8 (480 mg) as a white powder. The loading amount was 13.3 µmol/g.

### Step 9: preparation of siRNA conjugates 5 and 6

siRNA XR-3-01 (SEQ ID NO: 200/234) or siRNA XR-3-02 (SEQ ID NO: 201/235) was used as R², and siRNA conjugates 5 and 6 were obtained after solid phase synthesis and deprotection of the solid phase-loaded product M8.

### Synthesis of siRNA conjugates 7 and 8

siRNA conjugates **7 and 8** were prepared according to the following reaction routes.

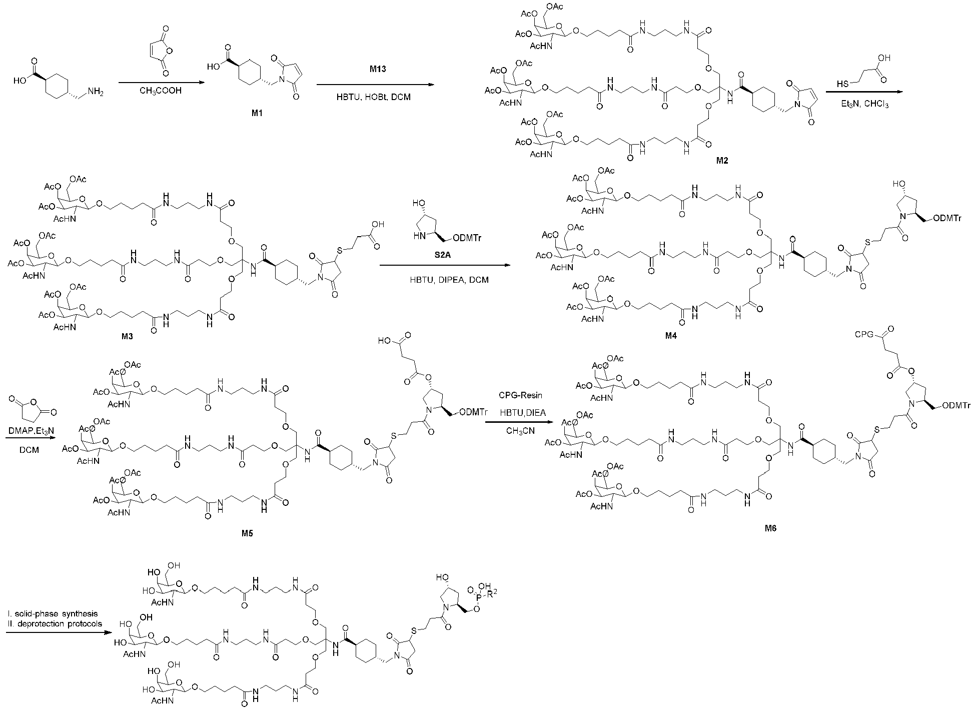

### Step 1: preparation of intermediate M1

Maleic anhydride (1 g, 10.20 mmol, 1 *eq.*) was dissolved in 20 mL of acetic acid, and tranexamic acid (1.60 g, 10.20 mmol, 1 *eq.*) was added. The resulting mixture was stirred at 160 °C for 6 h under nitrogen atmosphere. The maleic anhydride was almost completely consumed as monitored by TLC. The reaction liquid was concentrated by rotary evaporation at 60 °C, and the resulting crude product was separated by silica gel column chromatography (methanol/dichloromethane, 0-4%) to obtain M1 (1.1 g, yield: 45.5%) as a white solid. ¹H NMR (500 MHz, DMSO-*d₆*) δ 7.03 (s, 2H), 3.27 (d, *J* = 7.0 Hz, 2H), 2.13 (d, *J* = 11.0 Hz, 1H), 1.90 (d, *J* = 13.0 Hz, 2H), 1.65 (d, *J* = 12.5 Hz, 2H), 1.56 (s, 1H), 1.25 (q, *J* = 12.5 Hz, 2H), 0.97 (q, *J* = 12.5 Hz, 2H).

### Step 2: preparation of intermediate M2

M1 (79.35 mg, 334.47 µmol, 1 *eq.*) was dispersed in dichloromethane (10 mL), and then HBTU (253.69 mg, 668.93 µmol, 2 *eq.*)*, N*,*N-*diisopropylethylamine (86.45 mg, 668.93 µmol, 116.51 µL, 2 *eq.*)*,* HOBT (225.97 mg, 1.67 mmol, 5 *eq.*)*,* and M13 (600 mg, 334.47 µmol, 1 *eq.*) were added. The resulting mixture was stirred at 25 °C for 16 h under nitrogen atmosphere. A new product was generated as monitored by LCMS. The reaction liquid was concentrated by rotary evaporation to remove the solvent to obtain a residue. The residue was separated by silica gel column chromatography (methanol/dichloromethane, 0-20%) to obtain M2 (300 mg, yield: 50.0%) as a light yellow solid.

### Step 3: preparation of intermediate M3

*M2* (300 mg, 149.02 µmol, 1 *eq.*) and 3-mercaptopropionic acid (15.82 mg, 149.02 µmol, 1 *eq.*) were dissolved in chloroform (10 mL), and then triethylamine (15.08 mg, 149.02 µmol, 1 *eq.*) was added. The resulting mixture was stirred at 25 °C for 2 h. A target product was generated as monitored by LC-MS. The reaction mixture was used directly in the next step.

### Step 4: preparation of intermediate M4

EDCI (54.27 mg, 283.11 µmol, 2 eq.), HOBT (38.25 mg, 283.11 µmol, 2 *eq.*)*, N,N-*diisopropylethylamine (73.18 mg, 566.23 µmol, 98.62 µL, 4 *eq.*) and S2A (59.38 mg, 141.56 µmol, 1 *eq.*) were added to a solution of M3 (300 mg, 141.56 µmol, 1 *eq.*) in dichloromethane (5 mL). The resulting mixture was stirred at 25 °C for 16 h. A new product was generated as monitored by HPLC-MS. The reaction liquid was concentrated by rotary evaporation to remove the solvent to obtain a crude product. The crude product was separated by silica gel column chromatography (methanol/dichloromethane, 0-20%) to obtain M4 (140 mg, yield: 39.2%).

### Step 5: preparation of intermediate M5

M4 (140 mg, 55.54 µmol, 1 *eq.*)*,* triethylamine (252.90 mg, 2.50 mmol, 45 *eq.*)*,* and DMAP (13.57 mg, 111.08 µmol, 2 *eq.*) were mixed in 5 mL of dichloromethane. The above mixture was cooled in an ice bath, and then succinic anhydride (83.37 mg, 833.08 µmol, 15 *eq.*) was added. The resulting mixture was gradually heated to 25 °C and stirred at this temperature for 16 h under nitrogen atmosphere. A new product was generated as monitored by LC-MS. The mixture was concentrated by rotary evaporation to remove the solvent to obtain a crude product. The crude product was separated by preparative HPLC (carbon-18 chromatographic column, acetonitrile/0.01% ammonia water) to obtain M5 (32 mg, yield: 22.0%) as a white solid. HPLC purity: > 99.9%, LCMS (ESI), Cal. for C₁₂₄H₁₇₈N₁₂O₄₇S: 2620.89, Found [M-H]⁻ 2619.5.

### Step 6: preparation of intermediate M6

(I) M5 (32 mg, 12.21 µmol, 1 *eq.*) and HBTU (9.26 mg, 24.42 µmol, 2 *eq.*) were dissolved in 5 mL of acetonitrile, and then *N*,*N*-diisopropylethylamine (6.31 mg, 48.84 µmol, 8.51 µL, 4 *eq.*) was added. The mixture was shaken for 3-4 min, and then a CPG-amino resin (366.3 mg, 50 µmol/g) was added. The resulting mixture was shaken on a shaker at room temperature for 48 h. Then, the mixture was filtered, and the filter cake was washed twice with acetonitrile (10 mL). The resulting solid was dried at 45 °C for two hours to obtain a white solid (360 mg). (II) The above white solid and pyridine (391.64 mg, 4.95 mmol, 398.42 µL, 40 *eq.*) were mixed in 5 mL of acetonitrile, and then acetic anhydride (252.73 mg, 2.48 mmol, 20 *eq.*) was added in an ice bath. The resulting mixture was shaken on a shaker at room temperature for 0.5 h. Then, the mixture was filtered, and the filter cake was washed twice with acetonitrile (10 mL). The resulting solid was dried at 45 °C for 2 h to obtain a solid phase-loaded product M6 (345 mg) as a white powder. The loading amount was 27.0 µmol/g.

### Step 7: preparation of siRNA conjugates 7 and 8

siRNA XR-3-01 (SEQ ID NO: 200/234) or siRNA XR-3-02 (SEQ ID NO: 201/235) was used as R², and siRNA conjugates 7 and 8 were obtained after solid phase synthesis and deprotection of the solid phase-loaded product M6.

### Synthesis of siRNA conjugates 9 and 10

siRNA conjugates **9 and 10** were prepared according to the following reaction routes.

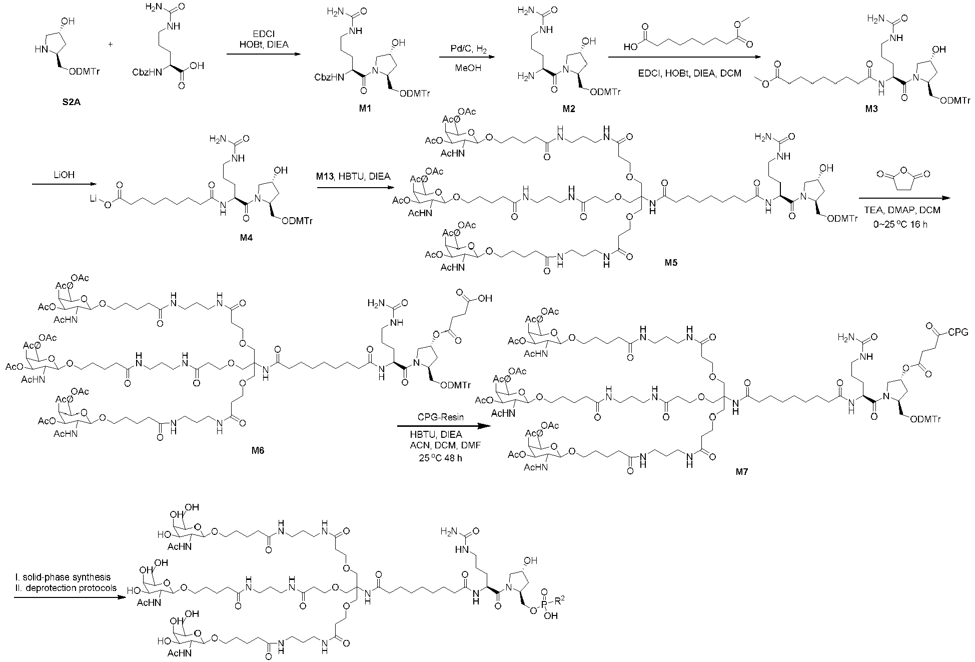

### Step 1: preparation of intermediate M1

(s)-2-(((Benzyloxy)carbonyl)amino)-5-ureidopentanoic acid (0.8 g, 2.59 mmol, 1 *eq.*)*,* EDCI (991.60 mg, 5.17 mmol, 2 *eq.*)*, N,N*-diisopropylethylamine (1.34 g, 10.35 mmol, 1.80 mL, 4 *eq.*)*,* and HOBt (349.47 mg, 2.59 mmol, 1 *eq.*) were mixed in 20 mL of dichloromethane, and then S2A (1.09 g, 2.60 mmol, 1 *eq.*) was added. The resulting mixture was stirred at 25 °C for 16 h under nitrogen atmosphere. A new product was generated as monitored by LC-MS. The reaction liquid was diluted with 20 mL of dichloromethane, and then 20 mL of water was added. The mixture was left to stand for liquid separation to obtain an organic phase. The organic phase was dried over anhydrous sodium sulfate and concentrated by rotary evaporation to remove the solvent to obtain a crude product. The crude product was separated by silica gel column chromatography to obtain M1 (1.2 g, yield: 65.0%).

### Step 2: preparation of intermediate M2

M1 (1.2 g, 1.69 mmol, 1 *eq.*) was dissolved in 10 mL of methanol, and then Pd/C (102.52 mg) was added. The resulting mixture was stirred at 25 °C for 16 h under H₂ atmosphere. A target product was generated as monitored by LC-MS. The reaction mixture was filtered to obtain a solution. The solution was concentrated by rotary evaporation to remove the solvent to obtain M2 (860 mg), which was used directly in the next step.

### Step 3: preparation of intermediate M3

Monomethyl azelate (301.61 mg, 1.49 mmol, 1 *eq.*)*,* HBTU (1.13 g, 2.98 mmol, 2 *eq.*)*,* and *N*,*N-*diisopropylethylamine (770.94 mg, 5.97 mmol, 1.04 mL, 4 *eq.*) were mixed in 10 mL of dichloromethane, and then M2 (860 mg, 1.49 mmol, 1 *eq.*) was added. The resulting mixture was stirred at 25 °C for 16 h under nitrogen atmosphere. A target product was generated as monitored by LC-MS. The reaction liquid was diluted with 20 mL of dichloromethane, and then 20 mL of water was added. The mixture was left to stand for liquid separation to obtain an organic phase. The organic phase was dried over anhydrous sodium sulfate and concentrated by rotary evaporation to remove the solvent to obtain a crude product. The crude product was separated by silica gel column chromatography (methanol/dichloromethane = 0-6%) to obtain M3 (680 mg, yield: 59.9%).

### Step 4: preparation of intermediate M4

M3 (600 mg, 788.53 µmol, 1 *eq*.) was dissolved in 10 mL of methanol and 10 mL of water, and then lithium hydroxide (188.85 mg, 7.89 mmol, 10 *eq.*) was added. The resulting mixture was stirred at 80 °C for 3 h. A target product was generated as monitored by LC-MS. The reaction liquid was concentrated by rotary evaporation to remove the solvent to obtain M4 (590 mg), which was used directly in the next step.

### Step 5: preparation of intermediate M5

M4 (201.43 mg, 267.57 µmol, 1.5 *eq.*) was dissolved in 5 mL of DMF, and then HOBT (1 *eq.*)*,* EDCI (2 *eq.*)*,* and DIPEA (2 *eq.*) were added. After stirring for 0.5 h, M13 (320 mg, 178.38 µmol, 1 *eq.*) was added. The resulting mixture was stirred at 25 °C for 16 h under nitrogen atmosphere. A target product was generated as monitored by LC-MS. 15 mL of water was added to the reaction system, and the resulting mixture was extracted with dichloromethane (15 mL × 3). The organic phases were combined and concentrated by rotary evaporation to remove the solvent to obtain a crude product. The crude product was separated by silica gel column chromatography (dichloromethane (containing 0.1% triethylamine)/methanol = 90:10, 80:20, and 70:30) to obtain M5 (230 mg).

### Step 6: preparation of intermediate M6

M5 (230 mg, 91.17 µmol, 1 *eq.*) was dissolved in 10 mL of dichloromethane, and then DMAP (22.28 mg, 182.34 µmol, 2 *eq.*) and triethylamine (415.14 mg, 4.10 mmol, 570.25 µL, 45 *eq.*) were added. The mixture was cooled in an ice bath, and succinic anhydride (136.85 mg, 1.37 mmol, 15 *eq.*) was added to the reaction system. The resulting mixture was gradually heated to 25 °C and stirred for 16 h under this condition. A target product was generated as monitored by LC-MS. The reaction mixture was concentrated, and the resulting crude product was separated by preparative HPLC (carbon-18 chromatographic column, acetonitrile/0.01% ammonia water) to obtain M6 (33.8 mg). HPLC purity: 99.83%. LCMS (ESI): Cal. for C₁₂₄H₁₈₄N₁₄O₄₇: 2622.89, Found [M-H]⁻: 2621.7.

### Step 7: preparation of intermediate M7

(I) M6 (33.8 mg, 12.89 µmol, 1 *eq.*) and HBTU (9.77 mg, 25.77 µmol, 2 *eq.*) were dissolved in 5 mL of acetonitrile, and then *N*,*N-*diisopropylethylamine (6.66 mg, 51.55 µmol, 8.98 µL, 4 *eq.*) was added. The mixture was shaken for 3-4 min, and then a CPG-amino resin (386.7 mg, 50 µmol/g) was added. The resulting mixture was shaken on a shaker at room temperature for 48 h. The reaction mixture was filtered, and the filter cake was washed twice with acetonitrile (25 mL). The resulting solid was dried at 35 °C for 2 h to obtain a white solid (382.6 mg). (II) The above white solid and pyridine (4.54 mg, 57.39 µmol, 4.62 µL, 4.37e-1 *eq.*) were mixed in 10 mL of acetonitrile, and then acetic anhydride (1.95 mg, 19.13 µmol, 1.81 µL, 1.46e-1 *eq.*) was added in an ice bath. The resulting mixture was shaken on a shaker at room temperature for 0.5 h. The mixture was filtered, and the filter cake was washed twice with acetonitrile (10 mL). The resulting solid was dried at 40 °C for 2 h to obtain M7 (261.6 mg) as a white solid. The loading amount was 20.5 µmol/g.

### Step 8: preparation of siRNA conjugates 9 and 10

siRNA XR-3-01 (SEQ ID NO: 200/234) or siRNA XR-3-02 (SEQ ID NO: 201/235) was used as R², and siRNA conjugates 9 and 10 were obtained after solid phase synthesis and deprotection of the solid phase-loaded product M7.

### Example 5: In vivo Efficacy of ANGPTL3 siRNAs in Mice

To evaluate the effect of siRNAs on the *in vivo* activity of ANGPTL3, an *in vivo* activity assay was performed on male ANGPTL3 humanized mice (purchased from Model Organisms) which were randomly divided into 6 groups of 6 mice each, as follows:
G1 NC (9 mg/kg) negative control group;
G2 Ref-A (3 mg/kg) positive control group;
G3 Ref-A (9 mg/kg) positive control group;
G4 2-851 (3 mg/kg) treatment group;
G5 2-851 (9 mg/kg) treatment group; and
G6 2-L96 (3 mg/kg) treatment group.

Ref-A is GalNac-conjugated AD-52981, which is synthesized according to US20160186180A1; 2-851 is siRNA conjugate 10 (obtained by siRNA XR-3-02 (SEQ ID NO: 201/235) and solid phase synthesis and deprotection of a solid phase-loaded product M7) in Example 4; 2-L96 is obtained by siRNA XR-3-02 (SEQ ID NO: 201/235) and solid phase synthesis and deprotection of a solid phase-loaded product L96; NC is obtained by siRNA (SEQ ID NO: 246/247) and solid phase synthesis and deprotection of a solid phase-loaded product L96.

The sense strand and the antisense strand of NC are respectively:
mGmCmAmAmCmU/i2FA/mA/i2FC/mCdUmAmA/i2FA/mCmAmAmUmA (SEQ ID NO: 246)
mU*/i2FA/*mUmUmGmUmU/i2FU/mA/i2FG/mG/i2FU/mU/i2FA/mGmUmUmGmC*/i2F C/*mC (SEQ ID NO: 247)

The structural formula of L96 is shown as follows:

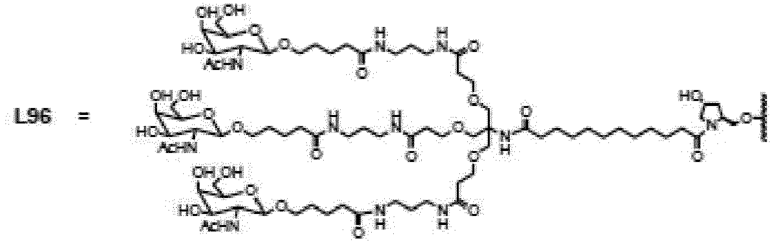

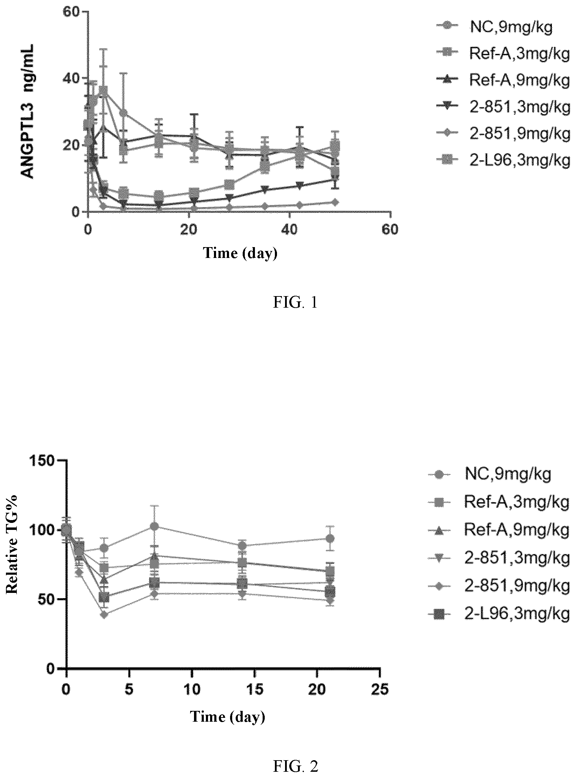

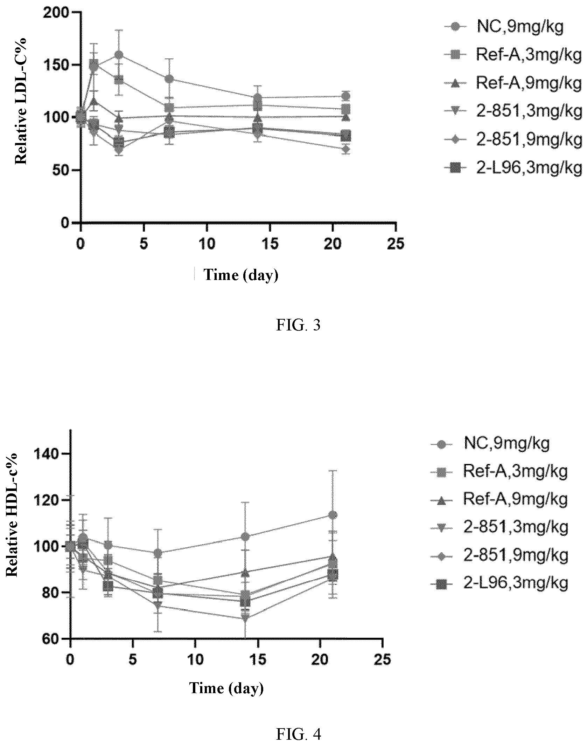

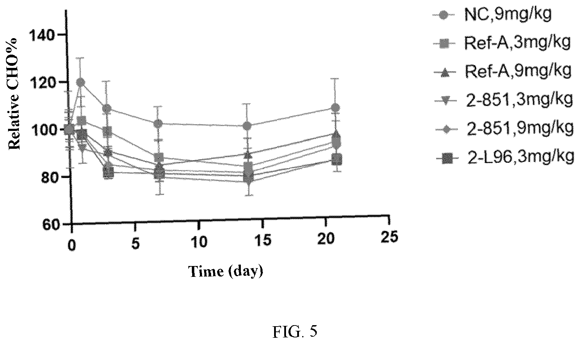

Pre-administration serum samples were obtained after a 4-hour fast on day -1. The above siRNA conjugates were each diluted with normal saline and then injected subcutaneously on day 1 according to the experimental design. NC was used as negative control. Plasma was collected after a 4-hour fast on days 1, 3, 7, 14, 21, 28, 35, 42, and 49. The level of an ANGPTL3 protein in plasma was determined by the ELISA method according to the experimental protocol provided by the supplier (R&D Systems). Triglyceride, total cholesterol, high-density lipoprotein cholesterol (HDL-c), and low-density lipoprotein cholesterol (LDL-c) in serum were determined using an automatic biochemical analyzer. This process was performed by Shanghai Beyotime Biological Tech. Co., Ltd. The results are shown in FIGs. 1-5.

The levels of triglyceride (TG), total cholesterol (CHO), HDL-c, and LDL-c were normalized for each animal. The normalization method was as follows: The levels of triglyceride, LDL, HDL, and total cholesterol in each animal were divided by the pre-treatment expression levels (in this case, expression levels on day -1) in the animal, respectively, at one time point, so as to determine the ratio "normalized to pre-treatment". The expression at a specific time point was then normalized to the NC group by dividing the ratio "normalized to pre-treatment" for an individual animal by the mean ratio "normalized to pre-treatment" of all mice in the NC group.

The result showed that the siRNAs were able to reduce the levels of ANGPTL3 protein and TG over a long period of time, with a significantly dose effect and a control rate superior to that of the positive control; meanwhile, they also showed a certain control effect on HDL-c and CHO.

### Example 6: In vivo Efficacy of ANGPTL3 siRNAs in Rhesus Monkey with Familial Hyperlipemia

To evaluate the *in vivo* activity of siRNAs against ANGPTL3, an *in vivo* activity assay was performed on male rhesus monkeys with familial hyperlipemia. Pre-administration serum samples were obtained after a 4-hour fast on days-7 and -14. The siRNA conjugates were each diluted with normal saline and then injected subcutaneously on day 1 according to the experimental design. Blank normal saline was used as a negative control. Plasma was collected after a 4-hour fast on days 3, 7, 14, 28, 42, 56, 70, and 84. The level of an ANGPTL3 protein in plasma was determined by the ELISA method according to the experimental procedures provided by the supplier (R&D Systems). Triglyceride, total cholesterol, high-density lipoprotein cholesterol (HDL-c), and low-density lipoprotein cholesterol (LDL-c), Apo-A1, and Apo-B in serum were determined using an automatic biochemical analyzer.

The levels of ANGPTL3 protein, triglyceride, total cholesterol, HDL-c, and LDL-c were normalized for each animal. For normalization, the levels of ANGPTL3 protein, triglyceride, HDL-c, LDL-c, total cholesterol, Apo-A1, and Apo-B in each animal were divided by the pre-treatment expression levels (mean values of samples of the group before the experiment), respectively, at one time point, so as to determine the expression ratio "normalized to pre-treatment". The expression at a specific time point was then normalized to the normal saline group by dividing the ratio "normalized to pre-treatment" for an individual animal by the mean ratio "normalized to pre-treatment" of all animals in the normal saline group.

The results showed that the siRNAs of the present invention were able to reduce the levels of ANGPTL3 protein, total cholesterol, and LDL-c over a long period of time.

## Claims

1. An siRNA for inhibiting the expression of an ANGPTL3 gene, comprising a sense strand and an antisense strand, wherein each nucleotide in the siRNA is independently a modified or unmodified nucleotide, the antisense strand comprises at least 17 contiguous nucleotides differing from those of any one of antisense strand sequences shown in Table 1, Table 2, or Table 3 by 0, 1, 2, or 3 nucleotides, and the sense strand has at least 15, 16, 17, 18, 19, 20, or 21 nucleotides complementary to those of the antisense strand.

2. The siRNA according to claim 1, wherein the sense strand comprises at least 17 contiguous nucleotides differing from those of any one of sense strand sequences shown in Table 1, Table 2, or Table 3 by 0, 1, 2, or 3 nucleotides.

3. The siRNA according to claim 2, wherein the antisense strand comprises any one of antisense strand nucleotide sequences shown in Table 1, and the sense strand comprises any one of sense strand nucleotide sequences shown in Table 1; preferably, the siRNA is selected from an siRNA indicated by any one of siRNA numbers in Table 1.

4. The siRNA according to claim 3, wherein the sense strand and the antisense strand comprise or consist of nucleotide sequences (5' → 3') selected from:
R-6
sense strand: GUCUCAAAAUGGAAGGUUAUA (SEQ ID NO: 6)
antisense strand: UAUAACCUUCCAUUUUGAGACUU (SEQ ID NO: 40)
R-15
sense strand: AGAACACCCAGAAGUAACU (SEQ ID NO: 15)
antisense strand: AGUUACUUCUGGGUGUUCUGG (SEQ ID NO: 49)
R-23
sense strand: AAAUCACGAAACCAACUAU (SEQ ID NO: 23)
antisense strand: AUAGUUGGUUUCGUGAUUUCC (SEQ ID NO: 57)
R-24
sense strand: ACAUCUAGUUGCGAUUACU (SEQ ID NO: 24)
antisense strand: AGUAAUCGCAACUAGAUGUAG (SEQ ID NO: 58)
wherein C, G, U, and A indicate cytidine-3'-phosphate, guanosine-3'-phosphate, uridine-3'-phosphate, and adenosine-3'-phosphate, respectively.

5. The siRNA according to any one of claims 1-4, wherein at least one nucleotide in the sense strand and the antisense strand is a modified nucleotide.

6. The siRNA according to claim 5, wherein the modified nucleotide is selected from a 2'-O-methyl-modified nucleotide, a 2'-deoxy-2'-fluoro-modified nucleotide, a 2'-deoxynucleotide, a 2'-methoxyethyl-modified nucleotide, a 2'-amino-modified nucleotide, a 2'-alkyl-modified nucleotide, a 2'-alkoxy-modified nucleotide, a 2'-F-arabinonucleotide, a phosphorothioate-modified nucleotide, an abasic nucleotide, a morpholino nucleotide, and a locked nucleotide.

7. The siRNA according to claim 6, wherein the modified nucleotide is selected from: a 2'-O-methyl-modified nucleotide, a 2'-deoxy-2'-fluoro-modified nucleotide, a 2'-deoxynucleotide, and a phosphorothioate-modified nucleotide.

8. The siRNA according to claim 7, wherein the modified nucleotide is selected from nucleotides as follows:
(1) in the direction from the 5' end to the 3' end, nucleotides at positions 7 and 9 of the sense strand are 2'-deoxy-2'-fluoro-modified nucleotides, and nucleotides at the rest of positions are 2'-O-methyl-modified nucleotides; or nucleotides at positions 7, 9, and 14 of the sense strand are 2'-deoxy-2'-fluoro-modified nucleotides, and nucleotides at the rest of positions are 2'-O-methyl-modified nucleotides; or nucleotides at positions 8 and 14 of the sense strand are 2'-deoxy-2'-fluoro-modified nucleotides, and nucleotides at the rest of positions are 2'-O-methyl-modified nucleotides; and/or
(2) in the direction from the 5' end to the 3' end, nucleotides at positions 6, 8, 9, 10, 12, 14, and 16 of the antisense strand are 2'-deoxy-2'-fluoro-modified nucleotides, and nucleotides at the rest of positions are 2'-O-methyl-modified nucleotides; or nucleotides at positions 2, 8, 10, 12, 14, and 20 of the antisense strand are 2'-deoxy-2'-fluoro-modified nucleotides, and nucleotides at the rest of positions are 2'-O-methyl-modified nucleotides; and/or
(3) in the direction from the 5' end to the 3' end, a nucleotide at position 11 of the sense strand is a 2'-deoxynucleotide; and/or
(4) in the direction from the 5' end to the 3' end, the 5' end of the sense strand comprises 1 or 2 phosphorothioate-modified nucleotides; and/or the 5' end and the 3' end of the antisense strand each independently comprise 1 or 2 phosphorothioate-modified nucleotides.

9. The siRNA according to claim 8, wherein the modified nucleotide is selected from nucleotides as follows:
in the direction from the 5' end to the 3' end, nucleotides at positions 7 and 9 of the sense strand are 2'-deoxy-2'-fluoro-modified nucleotides, a nucleotide at position 11 of the sense strand is a 2'-deoxynucleotide, and nucleotides at the rest of positions are 2'-O-methyl-modified nucleotides; and nucleotides at positions 6, 8, 9, 10, 12, 14, and 16 of the antisense strand are 2'-deoxy-2'-fluoro-modified nucleotides, and nucleotides at the rest of positions are 2'-O-methyl-modified nucleotides; or
in the direction from the 5' end to the 3' end, nucleotides at positions 7, 9, and 14 of the sense strand are 2'-deoxy-2'-fluoro-modified nucleotides, a nucleotide at position 11 of the sense strand is a 2'-deoxynucleotide, and nucleotides at the rest of positions are 2'-O-methyl-modified nucleotides; and nucleotides at positions 2, 8, 10, 12, 14, and 20 of the antisense strand are 2'-deoxy-2'-fluoro-modified nucleotides, and nucleotides at the rest of positions are 2'-O-methyl-modified nucleotides; or
in the direction from the 5' end to the 3' end, nucleotides at positions 8 and 14 of the sense strand are 2'-deoxy-2'-fluoro-modified nucleotides, a nucleotide at position 11 is a 2'-deoxynucleotide, and nucleotides at the rest of positions are 2'-O-methyl-modified nucleotides; and nucleotides at positions 2, 8, 10, 12, 14, and 20 of the antisense strand are 2'-deoxy-2'-fluoro-modified nucleotides, and nucleotides at the rest of positions are 2'-O-methyl-modified nucleotides.

10. The siRNA according to claim 9, wherein the 5' end of the sense strand comprises 1 or 2 phosphorothioate-modified nucleotides; and/or the 5' end and the 3' end of the antisense strand each independently comprise 1 or 2 phosphorothioate-modified nucleotides.

11. The siRNA according to any one of claims 1-7, wherein the antisense strand comprises any one of antisense strand nucleotide sequences shown in Table 2, and the sense strand comprises any one of sense strand nucleotide sequences shown in Table 2; or the antisense strand comprises any one of antisense strand nucleotide sequences shown in Table 3, and the sense strand comprises any one of sense strand nucleotide sequences shown in Table 3; preferably, the siRNA is selected from an siRNA indicated by any one of siRNA numbers in Table 2.

12. The siRNA according to claim 11, wherein the sense strand and the antisense strand comprise or consist of nucleotide sequences (5' → 3') selected from:
XR-1-01
sense strand: mGmUmCmUmCmAAfmAAfmUGfmGmAmAmGmGmUmUmAmUmA (SEQ ID NO: 178); antisense strand:
mUmAmUmAmACfmCUfUfCfmCAfmUUfmUUfmGmAmGmAmCmUmU (SEQ ID NO: 212)
XR-1-02
sense strand: mGmUmCmUmCmAAfmAAfmUdGmGmAmAmGmGmUmUmAmUmA (SEQ ID NO: 179); antisense strand:
mUmAmUmAmACfmCUfUfCfmCAfmUUfmUUfmGmAmGmAmCmUmU (SEQ ID NO: 213)
XR-1-03
sense strand: mGmUmCmUmCmAmAAfmAUfdGmGmAmAmGmGmUmUmAmUmA (SEQ ID NO: 180); antisense strand:
mUmAmUmAmACfmCUfUfCfmCAfmUUfmUUfmGmAmGmAmCmUmU (SEQ ID NO: 214)
XR-1-04
sense strand: mG*mU*mCmUmCmAAfmAAfmUdGmGmAmAmGmGmUmUmAmUmA (SEQ ID NO: 181); antisense strand:
mUmAmUmAmACfmCUfUfCfmCAfmUUfmUUfmGmAmGmAmCmUmU (SEQ ID NO: 215)
XR-1-05
sense strand: mG*mU*mCmUmCmAmAAfmAUfdGmGmAmAmGmGmUmUmAmUmA (SEQ ID NO: 182); antisense strand:
mUmAmUmAmACfmCUfUfCfmCAfmUUfmUUfmGmAmGmAmCmUmU (SEQ ID NO: 216)
XR-1-07
sense strand: mGmUmCmUmCmAAfmAAfmUdGmGmAmAmGmGmUmUmAmUmA (SEQ ID NO: 183); antisense strand:
mU*mA*mUmAmACfmCUfUfCfmCAfmUUfmUUfmGmAmGmAmC*mU*mU (SEQ ID NO: 217)
XR-1-08
sense strand: mGmUmCmUmCmAAfmAAfmUdGmGmAmAmGmGmUmUmAmUmA (SEQ ID NO: 184); antisense strand:
mUmAmUmAmACfmCUfUfCfCfAfmUUfmUUfmGmAmGmAmCmUmU (SEQ ID NO: 218)
XR-1-09
sense strand: mGmUmCmUmCmAAfmAAfmUdGmGmAmAmGmGmUmUmAmUmA (SEQ ID NO: 185); antisense strand:
mU*mA*mUmAmACfmCUfUfCfmCAfmUUfmUUfmGmAmGmAmC*mU*mU (SEQ ID NO: 219)
XR-2-01
sense strand: mAmAmAmUmCmAmCGfmAmAdAmCmCAfmAmCmUmAmU (SEQ ID NO: 186); antisense strand:
mAUfmAmGmUmUmGGfmUUfmUCfmGUfmGmAmUmUmUCfmC (SEQ ID NO: 220)
XR-2-02
sense strand: mAmAmAmUmCmAmCGfmAmAAfmCmCAfmAmCmUmAmU (SEQ ID NO: 187); antisense strand:
mAUfmAmGmUmUmGGfmUUfmUCfmGUfmGmAmUmUmUCfmC (SEQ ID NO: 221)
XR-2-03
sense strand: mAmAmAmUmCmAmCGfmAAfdAmCmCAfmAmCmUmAmU (SEQ ID NO: 188); antisense strand:
mAUfmAmGmUmUmGGfmUUfmUCfmGUfmGmAmUmUmUCfmC (SEQ ID NO: 222)
XR-2-04
sense strand: mAmAmAmUmCmACfmGAfmAdAmCmCAfmAmCmUmAmU (SEQ ID NO: 189); antisense strand:
mAUfmAmGmUmUmGGfmUUfmUCfmGUfmGmAmUmUmUCfmC (SEQ ID NO: 223)
XR-2-05
sense strand: mAmAmAmUmCmAmCGfmAmAdAmCmCmAAfmCmUmAmU (SEQ ID NO: 190); antisense strand:
mAUfmAmGmUmUmGGfmUUfmUCfmGUfmGmAmUmUmUCfmC (SEQ ID NO: 224)
XR-2-06
sense strand: mAmAmAmUmCmAmCGfmAmAdAmCmCmAmACfmUmAmU (SEQ ID NO: 191); antisense strand:
mAUfmAmGmUmUmGGfmUUfmUCfmGUfmGmAmUmUmUCfmC (SEQ ID NO: 225)
XR-2-07
sense strand: mA*mA*mAmUmCmAmCGfmAmAdAmCmCAfmAmCmUmAmU (SEQ ID NO: 192); antisense strand:
mAUfmAmGmUmUmGGfmUUfmUCfmGUfmGmAmUmUmUCfmC (SEQ ID NO: 226)
XR-2-09
sense strand: mAmAmAmUmCmAmCGfmAmAdAmCmCAfmAmCmUmAmU (SEQ ID NO: 193); antisense strand:
mAUfmAmGmUmUmGmGUfmUUfCfmGUfmGmAmUmUmUCfmC (SEQ ID NO: 227)
XR-2-10
sense strand: mAmAmAmUmCmAmCGfmAmAdAmCmCAfmAmCmUmAmU (SEQ ID NO: 194); antisense strand:
mAUfmAmGmUmUmGmGmUmUmUCfmGUfmGmAmUmUmUCfmC (SEQ ID NO: 228)
XR-2-11
sense strand: mAmAmAmUmCmAmCGfmAmAdAmCmCAfmAmCmUmAmU (SEQ ID NO: 195); antisense strand:
mAUfmAmGmUmUmGGfmUUfmUCfmGUfmGmAmUmUmUCfmC (SEQ ID NO: 229)
XR-2-12
sense strand: mAmAmAmUmCmAmCGfmAmAdAmCmCAfmAmCmUmAmU (SEQ ID NO: 196); antisense strand:
mA*Uf*mAmGmUmUmGGfmUUfmUCfmGUfmGmAmUmUmU*Cf*mC (SEQ ID NO: 230)
XR-2-13
sense strand: mAmAmAmUmCmAmCGfmAmAdAmCmCAfmAmCmUmAmU (SEQ ID NO: 197); antisense strand:
mA*Uf*mAmGmUmUmGGfmUUfmUCfmGUfmGmAmUmUmUCf*mC (SEQ ID NO: 231)
XR-2-14
sense strand: mAmAmAmUmCmAmCGfmAmAdAmCmCAfmAmCmUmAmU (SEQ ID NO: 198); antisense strand:
mA*UfmAmGmUmUmGGfmUUfmUCfmGUfmGmAmUmUmU*Cf*mC (SEQ ID NO: 232)
XR-2-15
sense strand: mAmAmAmUmCmAmCGfmAmAdAmCmCAfmAmCmUmAmU (SEQ ID NO: 199); antisense strand:
mA*UfmAmGmUmUmGGfmUUfmUCfmGUfmGmAmUmUmUCf*mC (SEQ ID NO: 233)
XR-3-01
sense strand: mAmAmAmUmCmACfmGAfmAdAmCmCAfmAmCmUmAmU (SEQ ID NO: 200); antisense strand:
mA*Uf*mAmGmUmUmGGfmUUfmUCfmGUfmGmAmUmUmU*Cf*mC (SEQ ID NO: 234)
XR-3-02
sense strand: mA*mAmAmUmCmACfmGAfmAdAmCmCAfmAmCmUmAmU (SEQ ID NO: 201); antisense strand:
mA*Uf*mAmGmUmUmGGfmUUfmUCfmGUfmGmAmUmUmU*Cf*mC (SEQ ID NO: 235)
XR-3-03
sense strand: mAmA*mAmUmCmACfmGAfmAdAmCmCAfmAmCmUmAmU (SEQ ID NO: 202); antisense strand:
mA*Uf*mAmGmUmUmGGfmUUfmUCfmGUfmGmAmUmUmU*Cf*mC (SEQ ID NO: 236)
XR-3-04
sense strand: mA*mA*mAmUmCmACfmGAfmAdAmCmCAfmAmCmUmAmU (SEQ ID NO: 203); antisense strand:
mA*Uf*mAmGmUmUmGGfmUUfmUCfmGUfmGmAmUmUmU*Cf*mC (SEQ ID NO: 237)
XR-3-05
sense strand: mAmAmAmUmCmACfmGAfmAdAmCmCAfmAmCmUmAmU (SEQ ID NO: 204); antisense strand:
mA*Uf*mAmGmUmUmGGfmUUfmUmCmGUfmGmAmUmUmU*Cf*mC (SEQ ID NO: 238)
XR-4-01
sense strand: mGmUmCmUmCmAAfmAAfmUdGmGmAmAmGmGmUmUmAmUmA (SEQ ID NO: 205); antisense strand:
mU*mA*mUmAmACfmCUfUfCfmCAfmUUfmUUfmGmAmGmAmC*mU*mU (SEQ ID NO: 239)
XR-4-02
sense strand: mG*mUmCmUmCmAAfmAAfmUdGmGmAmAmGmGmUmUmAmUmA (SEQ ID NO: 206);
antisense strand:
mU*mA*mUmAmACfmCUfUfCfmCAfmUUfmUUfmGmAmGmAmC*mU*mU (SEQ ID NO: 240)
XR-4-03
sense strand: mGmU*mCmUmCmAAfmAAfmUdGmGmAmAmGmGmUmUmAmUmA (SEQ ID NO: 207);
antisense strand:
mU*mA*mUmAmACfmCUfUfCfmCAfmUUfmUUfmGmAmGmAmC*mU*mU (SEQ ID NO: 241)
XR-4-04
sense strand: mG*mU*mCmUmCmAAfmAAfmUdGmGmAmAmGmGmUmUmAmUmA (SEQ ID NO: 208);
antisense strand:
mU*mA*mUmAmACfmCUfUfCfmCAfmUUfmUUfmGmAmGmAmC*mU*mU (SEQ ID NO: 242)
XR-4-05
sense strand: mGmUmCmUmCmAAfmAAfmUdGfGmAmAmGmGmUmUmAmUmA (SEQ ID NO: 209); antisense strand:
mU*mA*mUmAmACfmCUfUfCfmCAfmUUfmUUfmGmAmGmAmC*mU*mU (SEQ ID NO: 243)
XR-4-06
sense strand: mGmUmCmUmCmAAfmAAfmUdGmGfAmAmGmGmUmUmAmUmA (SEQ ID NO: 210); antisense strand:
mU*mA*mUmAmACfmCUfUfCfmCAfmUUfmUUfmGmAmGmAmC*mU*mU (SEQ ID NO:
244)
XR-4-07
sense strand: mGmUmCmUmCmAAfmAAfmUdGmGmAmAmGmGmUmUmAmUmA (SEQ ID NO: 211); antisense strand:
mU*mA*mUmAmACfmCUfUfCfmCAfmUUfmUUfmGmAmGmAmC*mU*mU (SEQ ID NO: 245),
wherein C, G, U, and A indicate cytidine-3'-phosphate, guanosine-3'-phosphate, uridine-3'-phosphate, and adenosine-3'-phosphate, respectively; m indicates that one adjacent nucleotide on the right side of the letter m is a 2'-O-methyl-modified nucleotide; f indicates that one adjacent nucleotide on the left side of the letter f is a 2'-deoxy-2'-fluoro-modified nucleotide; * indicates that one adjacent nucleotide on the left side of * is a phosphorothioate-modified nucleotide; f* indicates that one adjacent nucleotide on the left side of f* is a phosphorothioate- and 2'-deoxy-2'-fluoro-modified nucleotide; d indicates that one adjacent nucleotide on the right side of the letter d is a 2'-deoxyribonucleotide.

13. The siRNA according to any one of claims 1-12, wherein the sense strand and the antisense strand are each independently of 17-25 nucleotides in length; preferably, the sense strand and the antisense strand are each of independently 19-23 nucleotides in length.

14. An siRNA conjugate comprising the siRNA according to any one of claims 1-13 and a targeting group.

15. The siRNA conjugate according to claim 14, wherein the targeting group is a ligand with affinity for an asialoglycoprotein receptor.

16. The siRNA conjugate according to claim 14 or 15, wherein the targeting group comprises a group derived from a lipophil, wherein the lipophil is selected from cholesterol, cholic acid, amantanoacetic acid, 1-pyrenebutanoic acid, dihydrotestosterone, 1,3-*bis*-O(hexadecyl)glycerol, geranyloxyhexyl, hexadecyl glycerol, borneol, menthol, 1,3-propanediol, heptadecyl, palmitic acid, myristic acid, O-3-(oleoyl)lithocholic acid, O-3-(oleoyl)cholic acid, dimethoxytribenzyl, and phenoxazine.

17. The siRNA conjugate according to claim 14 or 15, wherein the targeting group comprises a group derived from a carbohydrate, wherein the carbohydrate is selected from allose, altrose, arabinose, cladinose, erythrose, erythrulose, fructose, D-fucitol, L-fucitol, fucosamine, fucose, fuculose, galactosamine, D-galactosaminol, *N*-acetyl-galactosamine (GalNAc), galactose, glucosamine, *N*-acetyl-glucosamine, glucosaminitol, glucose, glucose-6-phosphate, gulonoglyceraldehyde, L-glycero-D-mannose-heptose, glycerol, glycerone, gulose, idose, lyxose, mannosamine, mannose, mannose-6-phosphate, psicose, quinovose, quinovosamine, rhamnitol, rhamnosamine, rhamnose, ribose, ribulose, sedoheptulose, sorbose, tagatose, talose, tartaric acid, threose, xylose, and xylulose.

18. The siRNA conjugate according to claim 17, wherein the targeting group comprises a group derived from *N*-acetyl-galactosamine (GalNAc).

19. The siRNA conjugate according to any one of claims 14-18, wherein the targeting group is 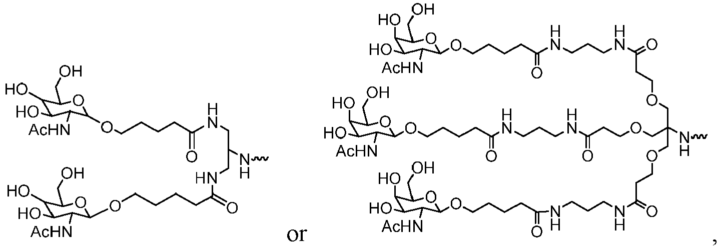 wherein the wavy line indicates a position at which the targeting group is linked to the rest of the siRNA conjugate.

20. The siRNA conjugate according to any one of claims 14-19, further comprising a linker, wherein the siRNA, the linker, and the targeting group are sequentially covalently or non-covalently linked.

21. The siRNA conjugate according to claim 20, wherein the linker comprises: reactive groups (such as primary amine and alkyne), alkyl groups, abasic nucleotides, ribitol (abasic ribose), and/or PEG groups; preferably, one end of the linker is a carbonyl group, through which the linker is covalently linked to the targeting group, and the other end is an -O- group for covalent linkage to the siRNA through a phosphoester bond (-O-P(O)OH-).

22. The siRNA conjugate according to claim 20 or 21, wherein the linker is selected from: 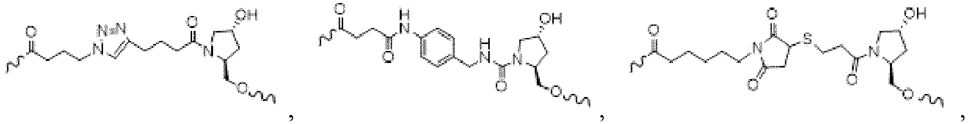 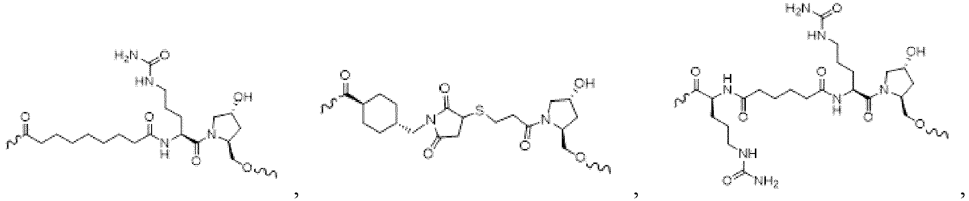 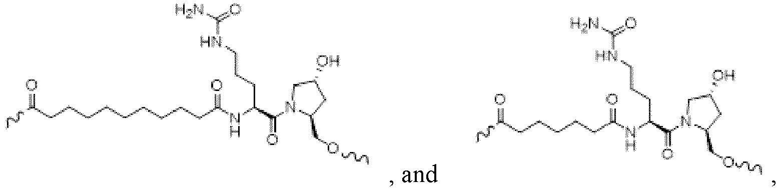 wherein the wavy line indicates a position at which the linker is linked to the rest of the siRNA conjugate, wherein the targeting group is linked to the carbonyl group of the linker; the siRNA is linked to the other end of the linker through a phosphoester bond.

23. The siRNA conjugate according to any one of claims 14-22, having a structure shown in the formula below: 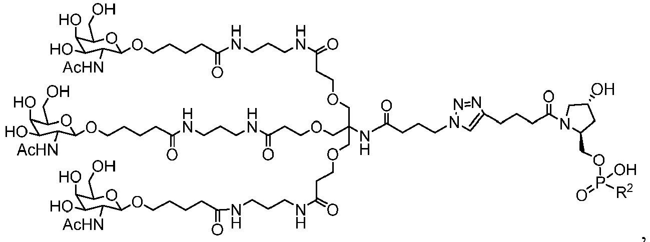 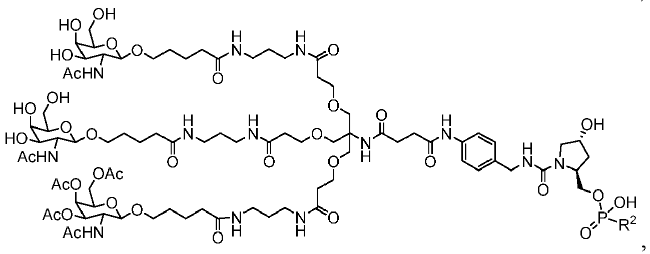 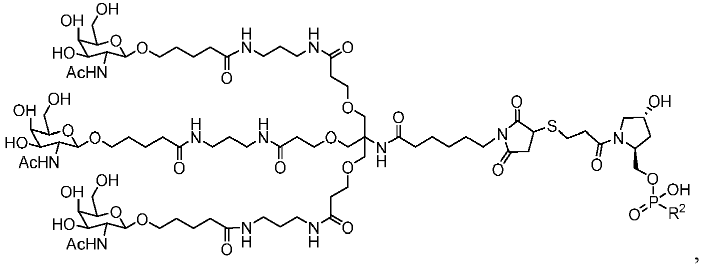 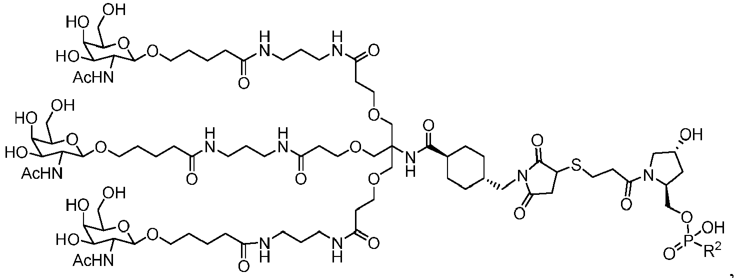 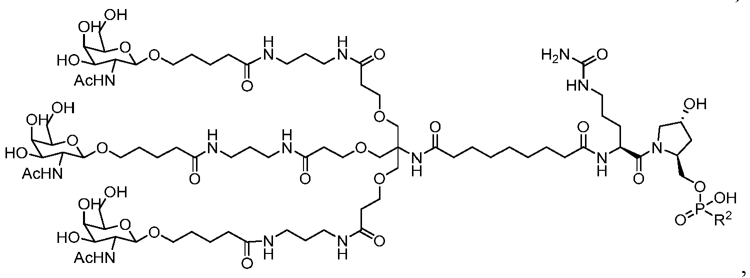 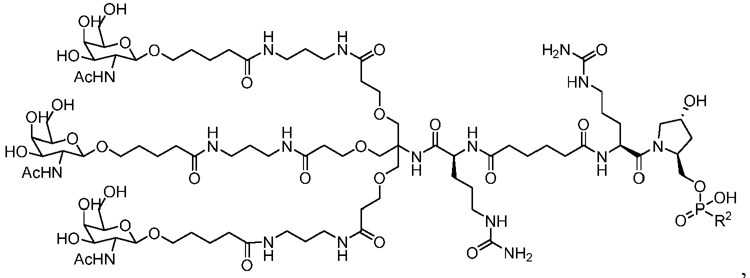 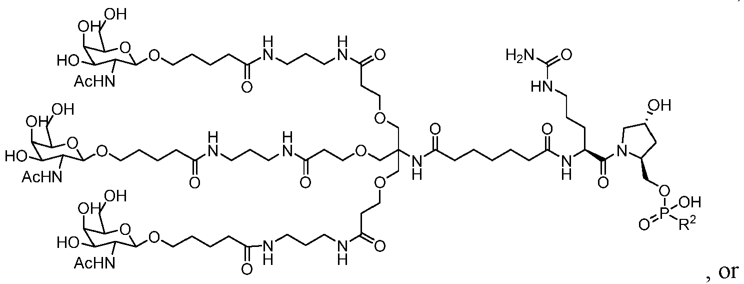 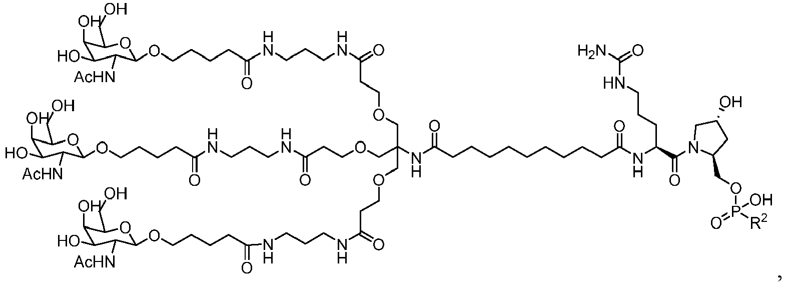
an isomer thereof or a pharmaceutically acceptable salt thereof,
wherein R² is the siRNA as defined in any one of claims 1-13.

24. The siRNA conjugate according to any one of claims 14-23, wherein the R² is covalently linked, at the 3' end of a sense strand thereof, to the targeting group or the linker via a phosphonyl group.

25. A pharmaceutical composition comprising the siRNA according to any one of claims 1-13, the siRNA conjugate according to any one of claims 14-24, an isomer thereof or a pharmaceutically acceptable salt thereof, and a pharmaceutically acceptable carrier.

26. Use of the siRNA according to any of claims 1-13, the siRNA conjugate according to any of claims 14-24, an isomer thereof or a pharmaceutically acceptable salt thereof, and the pharmaceutical composition according to claim 25, in the preparation of a medicament for treating and/or preventing an ANGPTL3-mediated disease or disorder.

27. The use according to claim 26, wherein the disease or disorder comprises atherosclerosis, hypercholesterolemia, hypertriglyceridemia, acute coronary syndrome, dyslipidemia, myocardial infarction, coronary artery lesion, stroke, coronary artery disease, cardiovascular disease, diabetes, hyperlipidemia, type 2 diabetes, and kidney disease.
